# EUROPEAN PATENT SPECIFICATION

(11) **EP 4 291 664 B1**
(45) Date of publication and mention of the grant of the patent: **27.05.2026**
(21) Application number: 22753488.0
(22) Date of filing: 14.02.2022
(51) Int. Cl.: C12N 15/85, C12N 15/09, C12N 9/22, C12N 15/113, C12N 9/10, C12N 15/10

(54) **SITE-SPECIFIC GENOME MODIFICATION TECHNOLOGY**
TECHNOLOGIE ZUR ORTSSPEZIFISCHEN GENOMMODIFIZIERUNG
TECHNOLOGIE DE MODIFICATION DU GÉNOME SPÉCIFIQUE D'UN SITE

(30) Priority: 15.02.2021 US 202163149419 P
(43) Date of publication of application: 20.12.2023
(73) Proprietor: North Carolina State University, Raleigh, North Carolina 27606 (US)
(72) Inventor: BEISEL, Chase Lawrence, Raleigh, North Carolina 27606 (US); COLLINS, Scott Patrick, Raleigh, North Carolina 27606 (US)
(74) Representative: Glawe, Delfs, Moll
(86) International application number: PCT/US2022/016313
(87) International publication number: WO 2022/174144

(56) References cited:
- WO-A1-2015/021426
- WO-A1-2015/089406
- WO-A1-2020/123512
- US-A1- 2017 058 298
- US-A1- 2020 063 127
- SUTCU HASER H ET AL: "Role of PARP-catalyzed ADP-ribosylation in the Crosstalk Between DNA Strand Breaks and Epigenetic Regulation", JOURNAL OF MOLECULAR BIOLOGY, ACADEMIC PRESS, UNITED KINGDOM, vol. 432, no. 6, 20 December 2019 (2019-12-20), pages 1769 - 1791, XP086100720, ISSN: 0022-2836, [retrieved on 20191220], DOI: 10.1016/J.JMB.2019.12.019
- JANKEVICIUS ET AL.: "Toxin-Antitoxin System DarTG Catalyzes Reversible ADP-Ribosylation of DNA", MOL CELL, vol. 64, no. 6, 15 December 2016 (2016-12-15), pages 1109 - 1116, XP029851111, DOI: 10.1016/j.molcel.2016.11.014

## Description

### CROSS REFERENCE TO RELATED APPLICATION

This application claims priority to and the benefit of U.S. Provisional Patent Application No. 63/149,419 filed February 15, 2021.

### SEQUENCE LISTING

The text of the computer readable sequence listing filed herewith, titled "39212-601_SEQUENCE_LISTING_ST25", created February 14, 2022, having a file size of 144,908 bytes, has been filed.

### GOVERNMENT FUNDING

This invention was made with government support under grant number GM119561 awarded by the National Institutes of Health. The government has certain rights in the invention.

### FIELD

The present disclosure provides compositions, methods, and systems related to template-mediated genome modification. In particular, the present disclosure provides novel genome modification technology involving site-specific chemical modification of a nucleotide to introduce a replication-blocking lesion. The compositions, methods, and systems described herein facilitate efficient site-specific genome modification of a DNA target, while minimizing the unintended edits and cellular toxicity associated with current genome editing approaches.

### BACKGROUND

CRISPR-based genome editing tools have found widespread application, relying on their easily programmable targeting and robust activity. Early use of these CRISPR-based tools has focused on the ability of Cas nucleases to cleave DNA. In the process of repairing the cleaved DNA, a genomic edit is introduced through homologous recombination with a supplied DNA repair template. DNA cleavage is, however, among the most toxic cellular events; DNA cleavage sets off cellular alarm systems which lead to mutations, DNA re-arrangements, or loss of cellular viability. Subsequent CRISPR-Cas genome editing tools have sought alternative approaches through target modification of individual bases or integration of a short template encoded within the guide RNA. Still, these methods are restricted in the range of edits that can be generated and can produce undesired edits. Therefore, there is a need for efficient genome editing and modification platforms that overcome the limitations of current systems. Fusion proteins comprising a DNA modifying domain and an inactive Cas protein, wherein the DNA modifying domain may be derived from, e.g., a nuclease, methyltransferase, glycosidase, polymerase, ligase, topoisomerase, kinase, an oxidoreductase or histone deacetylase or a deaminase, nuclease, transcriptional activators or repressors, histone modifying proteins, integrases or recombinases, are known from the prior art, see e.g. WO 2015/021426 A1 and WO 2015/089406 A1. However, said prior art is silent about complexes with DNA-modifying domains being an ADP-ribosyltransferase, as defined in the appended claim set.

### SUMMARY

The invention is set out in the appended claims. The embodiments of the description which do not fall within the scope of said claims are provided for illustrative purposes only and do not form part of the present invention. Embodiments of the present disclosure include a composition for targeted genome modification. In accordance with these embodiments, the composition includes a gap editor complex comprising a DNA-recognition domain and a DNA-modifying domain, wherein the DNA-recognition domain binds a DNA target sequence in the genome, and wherein the DNA-modifying domain induces formation of a replication blocking moiety on at least one nucleotide in the genome.

In some embodiments, the composition further comprises a donor nucleic acid template. In some embodiments, the donor nucleic acid template comprises a polynucleotide from an endogenous homologous sequence corresponding to the DNA target sequence. In some embodiments, the donor nucleic acid template comprise an exogenous single-stranded DNA (ssDNA) molecule or double-stranded DNA (dsDNA) molecule. In some embodiments, the donor nucleic acid template is an RNA molecule. In some embodiments, the presence of the donor nucleic acid template facilitates homology-directed gap repair and/or recombination, wherein the donor nucleic acid template or a fragment thereof is recombined into the genome of the DNA target sequence.

In some embodiments, the DNA-recognition domain comprises at least one Cas protein or fragment thereof lacking deoxyribonuclease activity. In some embodiments, the DNA-recognition domain comprises a complex of Cas proteins lacking deoxyribonuclease activity. In some embodiments, the DNA-recognition domain comprises a Cas protein or fragment thereof having nickase activity. In some embodiments, the Cas protein or Cas protein complex comprises a Type I Cascade, a Type II Cas9, a Type IV effector module, a Type V Cas12, a Cas9-related IscB, a Cas9-related TnpB, and combinations thereof.

In some embodiments, the DNA-recognition domain and the DNA-modifying domain are functionally coupled. In some embodiments, functionally coupled comprises polypeptide fusions, peptide tags, peptide linkers, RNA tags, and any combinations thereof.

In some embodiments, the DNA-modifying domain blocks DNA replication by adding the replication blocking moiety to: (i) at least one nucleotide in the DNA strand complementary to the DNA target sequence; (ii) at least one nucleotide in the DNA strand containing the DNA target sequence; or (iii) both at least one nucleotide in the DNA strand complementary to the DNA target sequence and at least one nucleotide in the DNA strand containing the DNA target sequence.

In some embodiments, the DNA-recognition domain induces a single-stranded break in the DNA target strand, and the DNA-modifying domain adds the replication blocking moiety to at least one nucleotide in the DNA strand complementary to the DNA target sequence.

In some embodiments, the DNA-modifying domain has been engineered to have reduced DNA binding, increased specificity to single-stranded DNA, and/or decreased enzymatic activity.

In some embodiments, the DNA-modifying domain catalyzes addition of ADP ribose to a thymine or guanine nucleotide. In some embodiments, the DNA-modifying domain comprises a DarT enzyme or a functional fragment, derivative, or variant thereof. In some embodiments, the DNA-modifying domain comprises a catalytic domain having at least 70% amino acid sequence identity with any of SEQ ID NOs: 18-21. In some embodiments, the DarT enzyme comprises one or more of the following amino acid substitutions: G49D, K56A, M86L, R92A, and/or R193A.

In some embodiments, the DNA-modifying domain comprises a Scabin enzyme or a functional fragment, derivative, or variant thereof. In some embodiments, the DNA-modifying domain comprises a catalytic domain having at least 70% amino acid sequence identity with any of SEQ ID NOs: 22-24. In some embodiments, the Scabin enzyme comprises an amino acid substitution that is K130A.

In some embodiments, the DNA-modifying domain catalyzes methylcarbamoylation of an adenine nucleotide. In some embodiments, the DNA-modifying domain comprises a Mom enzyme or a functional fragment, derivative, or variant thereof. In some embodiments, the DNA-modifying domain comprises a catalytic domain having at least 70% amino acid sequence identity with SEQ ID NO: 25-27. In some embodiments, the Mom enzyme comprises an amino acid substitution that is D149A.

In some embodiments, the DNA-modifying domain catalyzes addition a replication blocking moiety selected from the group consisting of: glucose, threonyl carbamoyl adenosine, acetate, glyceryl, L-ascorbic acid, uridine, adenosine mono-phosphate, a lipid, an amino acid, agmatine, L-threonylcarbamoyladenylate, L-threonylcarbamoyl, methylthiolate, sulfur, a methyl group, S-adenosyl-L-methione or a subgroup of S-adenosyl-L-methione, and dimethylallyl diphosphate or a subgroup thereof.

In some embodiments, the DNA-modifying enzyme domain comprises an enzyme or functional fragment, derivative, or variant thereof, selected from the group consisting of: Pierisin, Scabin, Cell cycle and apoptosis regulator 1 (CARP-1), SCO5461 protein (ScARP), adenine modification enzyme, acetyltransferase, amino acid transferase, nucleotidyl transferase, uridyltransferase, acyltransferase, ADP-ribosyltransferase, methylthiotransferase, N-acetyl transferase 10, tRNA(Met) cytidine acetyltransferase (TmcA), tRNA cytidine acetyltransferase, GCN5-related N-acetyltransferase, lysidine synthase, m⁷G methyltransferase, N6 carbamoylmethyltransferase (Mom), N6-adenosine threonylcarbamoyltransferase, threonyl carbomyl transferase or threonyl carbomyl transferase complex, TsaB-TsaE-TsaD (TsaBDE) complex, tRNA N6-adenosine threonylcarbamoyltransferase (Qri7, Tcs4), methyltransferase, ATrm5a, tRNA:m¹G/imG2 methyltransferase, tRNA (adenosine(37)-N6)-dimethylallyltransferase, tRNA dimethylallyltransferase (MiaA), and isopentenyltransferase.

In some embodiments, the composition comprises at least one guide RNA molecule. In some embodiments, the at least one guide RNA comprises gRNA, sgRNA, crRNA, or any combinations thereof. In some embodiments, the at least one guide RNA comprises a handle sequence and a targeting sequence. In some embodiments, the at least one guide RNA is complementary to the DNA target sequence.

In some embodiments, the composition further comprises at least one gap editor accessory factor. In some embodiments, the at least one gap editor accessory factor comprises a protein that augments at least one step in a genome modification process. In some embodiments, the at least one gap editor accessory factor is recruited to the gap editor complex via interaction with the DNA-modifying domain, the DNA-recognition domain, and/or the at least one guide RNA. In some embodiments, the recruitment of the at least one gap editor accessory factor to the gap editor complex comprises a peptide tag, a peptide linker, an RNA tag, and any combinations thereof. In some embodiments, the at least one gap editor accessory factor comprises Rap, DarG, Orf, ExoI, Exonuclease III, PrimPol , RecJ, RecQ1, Rad51, Rad52, CtIP, Rad18, and any combinations thereof.

Embodiments of the present disclosure also includes a kit for targeted genome modification. In accordance with these embodiments, the kit includes a gap editor complex comprising a DNA-recognition domain and a DNA-modifying domain, wherein the DNA-recognition domain binds a DNA target sequence in the genome, and wherein the DNA-modifying domain induces formation of a replication blocking moiety on at least one nucleotide in the genome.

In some embodiments, the kit further comprises a donor nucleic acid template. In some embodiments, the presence of the donor nucleic acid template facilitates homology-directed gap repair and/or recombination.

In some embodiments, the kit further comprises a guide RNA molecule.

In some embodiments of the kit, the DNA-recognition domain comprises at least one Cas protein or fragment thereof lacking deoxyribonuclease activity. In some embodiments, the DNA-recognition domain comprises at least one Cas protein or fragment thereof having nickase activity. In some embodiments, the Cas protein or Cas protein complex comprises a Type I Cascade, a Type II Cas9, a Type IV effector module, a Type V Cas12, a Cas9-related IscB, a Cas9-related TnpB, and combinations thereof.

In some embodiments of the kit, the DNA-recognition domain and the DNA-modifying domain are functionally coupled. In some embodiments, the DNA-recognition domain induces a single-stranded break in the DNA target strand, and wherein the DNA-modifying domain adds the replication blocking moiety to at least one nucleotide in the DNA strand complementary to the DNA target sequence.

In some embodiments of the kit, the DNA-modifying domain catalyzes addition of ADP ribose to a thymine or guanine nucleotide. In some embodiments, the DNA-modifying domain comprises a DarT enzyme or a functional fragment, derivative, or variant thereof. In some embodiments, the DNA-modifying domain comprises a Scabin enzyme or a functional fragment, derivative, or variant thereof. In some embodiments, the DarT enzyme has been engineered to have reduced DNA binding, increased specificity to single-stranded DNA, and/or decreased enzymatic activity.

In some embodiments of the kit, the DNA-modifying domain catalyzes methylcarbamoylation of an adenine nucleotide. In some embodiments, the DNA-modifying domain comprises a Mom enzyme or a functional fragment, derivative, or variant thereof. In some embodiments, the Mom enzyme has been engineered to have reduced DNA binding, increased specificity to single-stranded DNA, and/or decreased enzymatic activity.

In some embodiments of the kit, the DNA-modifying domain catalyzes addition a replication blocking moiety selected from the group consisting of: glucose, threonyl carbamoyl adenosine, acetate, glyceryl, L-ascorbic acid, uridine, adenosine mono-phosphate, a lipid, an amino acid, agmatine, L-threonylcarbamoyladenylate, L-threonylcarbamoyl, methylthiolate, sulfur, a methyl group, S-adenosyl-L-methione or a subgroup of S-adenosyl-L-methione, and dimethylallyl diphosphate or a subgroup thereof.

In some embodiments of the kit, the DNA-modifying enzyme domain comprises an enzyme or functional fragment, derivative, or variant thereof, selected from the group consisting of: Pierisin, Scabin, Cell cycle and apoptosis regulator 1 (CARP-1), SCO5461 protein (ScARP), adenine modification enzyme, acetyltransferase, amino acid transferase, nucleotidyl transferase, uridyltransferase, acyltransferase, ADP-ribosyltransferase, methylthiotransferase, N-acetyl transferase 10, tRNA(Met) cytidine acetyltransferase (TmcA), tRNA cytidine acetyltransferase, GCN5-related N-acetyltransferase, lysidine synthase, m⁷G methyltransferase, N6 carbamoylmethyltransferase (Mom), N6-adenosine threonylcarbamoyltransferase, threonyl carbomyl transferase or threonyl carbomyl transferase complex, TsaB-TsaE-TsaD (TsaBDE) complex, tRNA N6-adenosine threonylcarbamoyltransferase (Qri7, Tcs4), methyltransferase, ATrm5a, tRNA:m¹G/imG2 methyltransferase, tRNA (adenosine(37)-N6)-dimethylallyltransferase, tRNA dimethylallyltransferase (MiaA), and isopentenyltransferase.

In some embodiments of the kit, the at least one guide RNA comprises gRNA, sgRNA, crRNA, or any combinations thereof. In some embodiments, the at least one guide RNA comprises a handle sequence and a targeting sequence. In some embodiments, the targeting sequence in the at least one guide RNA is complementary to the DNA target sequence.

In some embodiments, the kit further comprises at least one gap editor accessory factor.

Embodiments of the present disclosure also include a method for targeted genome modification. In accordance with these embodiments, the method includes introducing any of the compositions of the present disclosure into a cell, and assessing the cell for presence of a desired genome alteration.

In some embodiments, a gap editor complex and/or a at least one guide RNA molecule are introduced into the cell as a polypeptide(s), mRNA(s), and/or DNA expression construct(s). In some embodiments, the gap editor complex and/or the guide RNA are introduced into the cell as part of a gene drive system.

In some embodiments, the cell is a prokaryotic cell or a eukaryotic cell. In some embodiments, the cell is a mammalian cell. In some embodiments, the cell is a plant cell.

In some embodiments, the method leads to a reduced degree of indel formation, chromosomal rearrangements, and/or DNA duplications.

In some embodiments, cell viability is enhanced and/or cell toxicity is reduced.

Other aspects and embodiments of the disclosure will be apparent in light of the following detailed description.

### BRIEF DESCRIPTION OF THE DRAWINGS

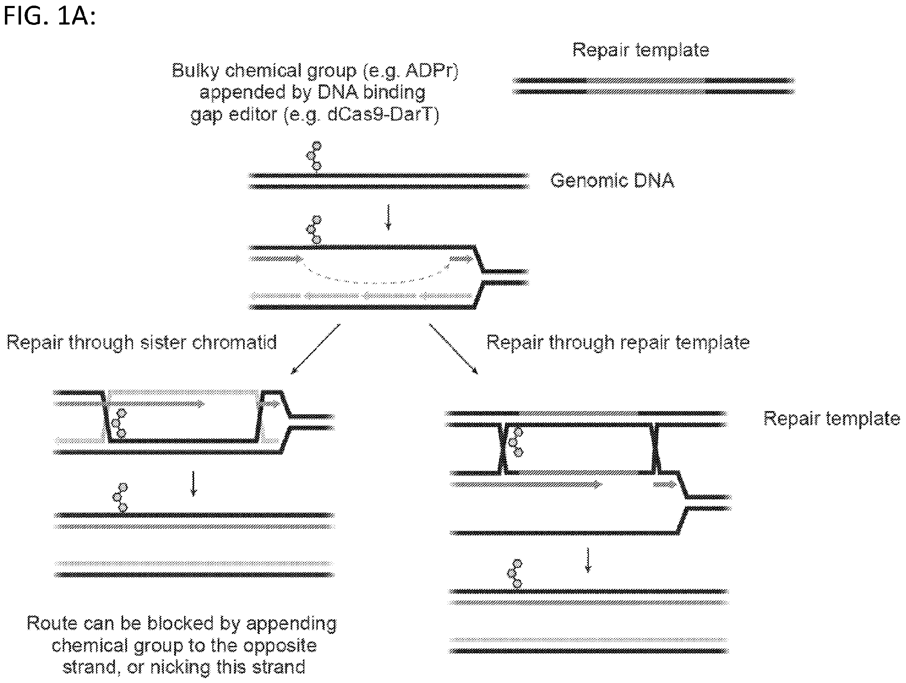

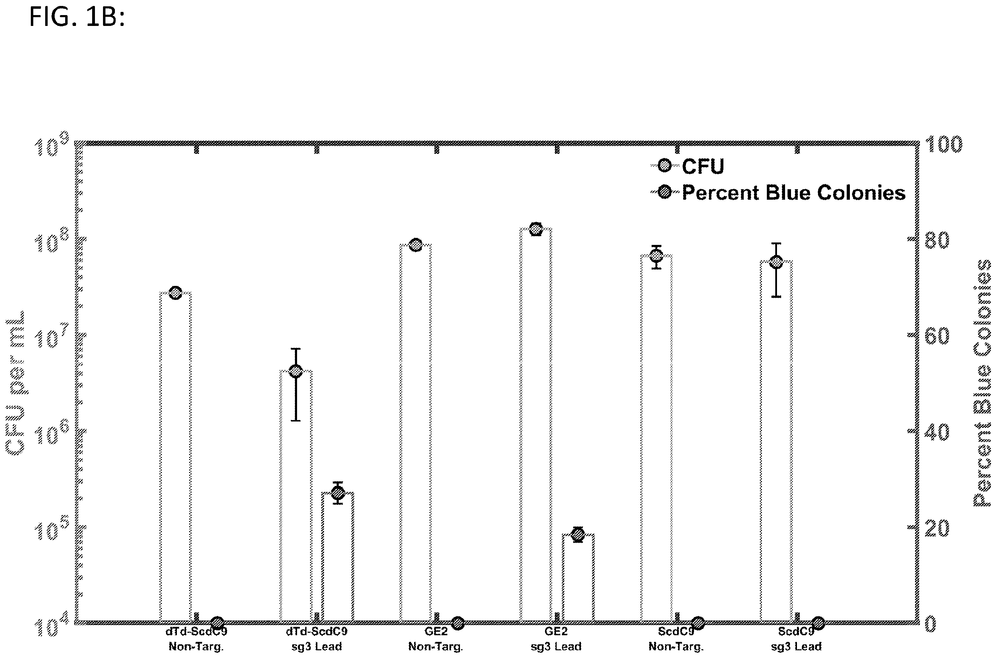

FIGS. 1A-1B: FIG. 1A provides a representative illustration of the general mechanism of gap editing. A bulky chemical group appended to one strand of DNA by a gap editor blocks DNA replication, resulting in a single-stranded DNA gap. That gap is then repaired through homologous recombination that can integrate a homologous repair template. The opposite strand can also be nicked or chemically modified to block recombination with sister chromatid and enhance editing. FIG. 1B includes representative results of experiments demonstrating efficient *lacZ* gene repair with significantly reduced cytotoxic effects using gap editor complexes comprising a DNA-modifying enzyme (DarT) engineered to have reduced DNA binding.
FIG. 2 includes representative results of experiments demonstrating efficient *lacZ* gene repair with significantly reduced cytotoxic effects using gap editor complexes comprising a DNA-recognition domain (DarT_G49D_K56A-ScnCas9 or GE2n) engineered to have nickase activity.
FIG. 3 includes representative results of experiments demonstrating the attenuation of *lacZ* gene repair by gap editor complexes when a gap editor accessory factor is used (DarG) to counteract the function of the DNA-modifying domain (DarT) of the gap editor complex.
FIG. 4 includes representative results of experiments demonstrating successful genome modification through increased frequency of kanamycin gene repair using gap editor complexes comprising a DNA-modifying domain (Scabin) in combination with a Cas9 DNA-recognition domain (Scabin-K130A-ScdCas9).
FIG. 5 includes representative results of experiments demonstrating successful genome modification through increased frequency of kanamycin gene repair using gap editor complexes comprising a DNA-modifying domain (Mom) in combination with a Cas9 DNA-recognition domain (Mom-D149A-ScdCas9).
FIG. 6 includes representative results of experiments demonstrating that successful genome modification (e.g., though increased frequency of kanamycin gene repair) using gap editor complexes relies on a DNA-modifying domain (DarT) in combination with a Cas9 DNA-recognition domain (DarT-G49D-ScdCas9) and active RNA-directed targeting. (ScdCas9 alone did not lead to kanamycin gene repair.)
FIG. 7 includes representative results of experiments using a gap editor complex with a DarT DNA-modifying domain comprising a specific mutation (R193A) that significantly reduces toxicity (DarT-G49D-R193A-ScdCas9).
FIG. 8 includes representative results of experiments using a gap editor complex with a DarT DNA-modifying domain comprising mutations (G49D, R193A, M86L, and R92A) that significantly reduces background editing while maintaining on-target editing, as demonstrated through reduced and maintained frequency of kanamycin gene repair, respectively.
FIG. 9 includes representative results of experiments demonstrating successful genome modification through increased frequency of kanamycin gene repair using gap editor complexes comprising a DNA-modifying domain (DarT) with mutations (G49D and/or R193A) that significantly reduce toxicity in combination with a Cas9 DNA-recognition domain having nickase activity (ScdCas9). Adding the R193A mutation to the G49D mutation further reduced toxicity without compromising modification. Site-specific genome modification was nearly 100% effective.
FIG. 10 includes representative results of experiments demonstrating that gene knockout of *fcy1* confers resistance to 5-Fluorocytosine (5-FC). Targeting the *fcy1* gene in Saccharomyces Cerevisiae with a Cas9 nickase (ScnCas9) or the fusion of an engineered DarT gene to a Cas9 nickase and providing a repair template resulted in genome modification at *fcy1.* For all mutations, the fusion of DarT provides a >10-fold increase in the rate of genome editing, demonstrating the utility of the introduction of replication blocking moieties in a eukaryotic cell.

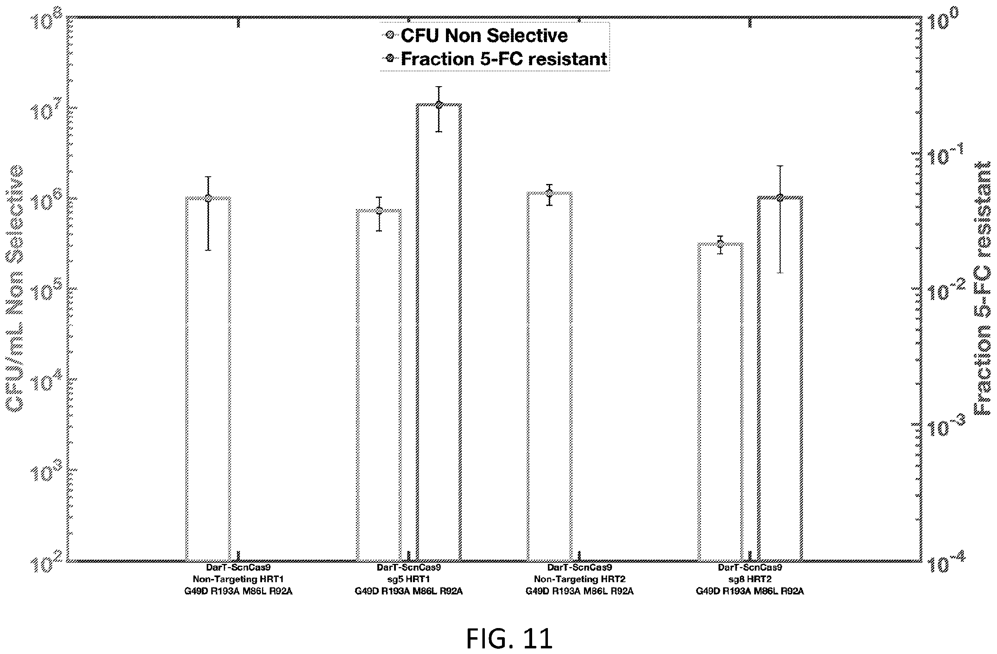

FIG. 11 includes representative results of experiments demonstrating that gene knockout of *fcy1* confers resistance to 5-Fluorocytosine (5-FC). Targeting the *fcy1* gene in Saccharomyces Cerevisiae with a Cas9 nickase (ScnCas9) or the fusion of an engineered DarT gene to a Cas9 nickase and providing a repair template resulted in genome modification at *fcy1.* The repair template encodes 6 mutations introducing two or three stop codons in *fcy1*, which results in a loss of *fcy1* function after genome modification, and resistance to 5-FC. The use of an engineered DarT variant including the G49D, R193A, M86L and R92A mutations improves cell viability up to approximately 50-fold over DarT with the G49D and R193A mutations alone. This gap editor complex effectuates efficient and low toxicity genome modification using two separate single guide RNAs and repair templates targeting *fcy1* in yeast.
FIG. 12 includes representative chromatographs providing confirmation of *fcy1* genome modification and gene knockout by sanger sequencing. Two or three stop codons were introduced by targeting a gap editor complex to the *fcy1* gene and providing a DNA repair template. The edited nucleotides are highlighted in red. Genomic edits for two separate targets within *fcy1* are shown.
FIG. 13 includes representative results of experiments demonstrating that gene knockout of *lacZ* results in a white colony color in the presence of the lactose analog IPTG and the colorimetric indicator X-gal. Targeting the *lacZ* gene in *E. coli* with a nuclease-inactive Cas12a protein (dLbCas12a) fused to an engineered DarT gene and providing a repair template resulted in genome modification at *lacZ.* No genome modification was observed without targeting of the gap editor complex to the *lacZ* gene.
FIG. 14 includes representative chromatographs demonstrating successful introduction of one or more stop codons into the *lacZ* gene, eliminating beta-galactosidase expression and thereby resulting in a white colored colony when plated in the presence of the inducer IPTG and the colorimetric indicator X-gal using DarT(G49D/R193A)-dLbCas12a associated with different crRNAs.
FIG. 15 includes representative results of experiments demonstrating that introduction of the D516G mutation into the *rpoB* gene confers resistance to the antibiotic rifampicin, and thus serves as a readout of genome modification. Targeting the *rpoB* gene in *E. coli* with an engineered DarT variant fused to a Cas9 nickase (ScnCas9) and co-expression of an RNA repair template and a reverse transcriptase resulted in site-specific RNA templated genome modification.
FIG. 16 includes representative results of experiments demonstrating that introduction of the D516G mutation into the *rpoB* gene confers resistance to the antibiotic rifampicin, and thus serves as a readout of genome modification. Targeting the *rpoB* gene in E. coli with an engineered DarT variant fused to a Cas9 nickase (ScnCas9) and providing a linear single-stranded DNA repair template resulted in genome modification at *rpoB.* Targeting of the gap editor complex to *rpoB* results in a 100 to 6,000-fold increase in genome modification rates, demonstrating the effect of the gap editors.
FIG. 17 includes representative chromatograms of the RNA-templated mutations in the *rpoB* gene introduced by the targeting of a gap editor complex to the *rpoB* gene, expression of the RNA repair template, and expression of the reverse transcriptase Ec86. Mutations include the AC>GT mutation required for D516G mediated rifampicin resistance.
FIG. 18 includes an image of a consensus sequence for a DarT catalytic domain (SEQ ID NO: 18) of the DNA-modifying domains of the gap editor complexes of the present disclosure.
FIG. 19 includes an image of a consensus sequence for a DarT catalytic domain (SEQ ID NO: 19) of the DNA-modifying domains of the gap editor complexes of the present disclosure.
FIG. 20 includes an image of a consensus sequence for a DarT catalytic domain (SEQ ID NO: 20) of the DNA-modifying domains of the gap editor complexes of the present disclosure.
FIG. 21 includes an image of a consensus sequence for a DarT catalytic domain (SEQ ID NO: 21) of the DNA-modifying domains of the gap editor complexes of the present disclosure.
FIG. 22 includes an image of a consensus sequence for a Scabin catalytic domain (SEQ ID NO: 22) of the DNA-modifying domains of the gap editor complexes of the present disclosure.
FIG. 23 includes an image of a consensus sequence for a Scabin catalytic domain (SEQ ID NO: 23) of the DNA-modifying domains of the gap editor complexes of the present disclosure.
FIG. 24 includes an image of a consensus sequence for a Scabin catalytic domain (SEQ ID NO: 24) of the DNA-modifying domains of the gap editor complexes of the present disclosure.
FIG. 25 includes an image of a consensus sequence for a Mom catalytic domain (SEQ ID NO: 25) of the DNA-modifying domains of the gap editor complexes of the present disclosure.
FIG. 26 includes an image of a consensus sequence for a Mom catalytic domain (SEQ ID NO: 26) of the DNA-modifying domains of the gap editor complexes of the present disclosure.
FIG. 27 includes an image of a consensus sequence for a Mom catalytic domain (SEQ ID NO: 27) of the DNA-modifying domains of the gap editor complexes of the present disclosure.

### DETAILED DESCRIPTION

The invention is set out in the appended claims. The embodiments of the description which do not fall within the scope of said claims are provided for illustrative purposes only and do not form part of the present invention. Nucleotide modifications can take the form of functional modifications, such as DNA methylation at certain positions, or damaging modification (DNA lesions), such as cross-linking, oxidation, and nitrosylation. These DNA lesions need to be repaired to maintain information fidelity and DNA functionality. Commonly occurring lesions are directly repaired through base excision, mismatch, and nucleotide excision repair processes. However, if these lesions are not repaired before DNA replication, then they can become locked into the genome as mutated DNA or stifle cellular division altogether. To avoid this, replication-dependent repair processes have evolved. One such process, translesion synthesis, can directly bypass some DNA lesions; however, this can introduce DNA mutations across some DNA lesions. Alternatively, replicating the DNA near the lesion can be skipped altogether by re-priming synthesis downstream of the lesion. This re-priming can occur via a lagging strand primase, or in higher eukaryotes by the leading strand primase-polymerase, PRIMPOL. This re-priming action enables replication to continue but leaves an unreplicated region complementary to the DNA lesion and surrounding DNA. The cell still needs to determine the appropriate sequence complementary to the DNA lesion, and to do this, cells employ a mechanism called homology-dependent gap repair (a subset of homologous recombination).

Homology-dependent gap repair (HDGR) is a highly accurate repair process in which a sister chromatid is used as a template to copy DNA complementary to the lesion-containing strand. As a subset of homologous recombination, experiments were conducted, as described further herein, to investigate whether this pathway could be co-opted to instead use an ectopic repair template instead of (or in addition to) the sister chromatid, generating synthetic genomic edits. Previous results demonstrated that site-specific introduction of abasic DNA could trigger HDGR and be completed using a plasmid-borne DNA template for repair, generating accurately edited genomic DNA. However, in some cases, this approach can be somewhat dependent on the stability of the abasic site. For example, an abasic site can be stabilized through inhibition of a cell's AP endonuclease activity but AP endonuclease inhibition can negatively affect cell viability and genomic stability and may not be feasible for some applications. Therefore, as described further herein, an alternative class of DNA lesions was identified that are not as susceptible to base excision or similar repair processes. Embodiments of the present disclosure include a class of lesions involving the addition of chemical groups to DNA that block DNA replication (replication blocking moiety) and facilitate HDGR.

For example, experiments were conducted to investigate whether the addition of adenosine-diphosphate ribose (ADPr) might be a promising DNA lesion candidate and act as a replication blocking moiety. ADPr transferases, which catalyze ADPr addition to nucleotides, are cytotoxic. Therefore, methods were developed to limit ADPr activity to the R-loop exposed after CRISPR-Cas binding to the genome, in an effort to trigger HDGR without loss of cell viability. Extracted dsDNA binding ADPr-transferases were shown to be lethal when electroporated into eukaryotic cells. Separately, dsDNA binding DNA modifying enzymes have been fused to DNA binding proteins to localize their activity, but they retain high rates of off-target modification, which necessitates additional mitigating steps to control activity. Single-stranded DNA binding enzymes can have their activity localized to the DNA R-loop exposed after target binding by a Cas effector to the DNA.

Previous work has described a class of single-stranded binding ADPr-transferase enzymes, including DarT and the DarT mutant DarT_G49D, which acts as a bacterial toxin. DarT expression is lethal in *E. coli,* and seems to be primarily repaired through recombination, and more weakly, through nucleotide excision repair. Therefore, experiments were conducted to investigate whether DarT could be used to trigger site-specific HDGR templated not by the genome, but by a recombinant DNA sequence. Experiments sought to understand whether DarT could be sufficiently controlled to localize ADPr modification to the Cas target site, avoiding cytotoxicity and allowing for efficient genome modification.

Section headings as used in this section and the entire disclosure herein are merely for organizational purposes and are not intended to be limiting.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present disclosure. The phrase "in one embodiment" as used herein does not necessarily refer to the same embodiment, though it may. Furthermore, the phrase "in another embodiment" as used herein does not necessarily refer to a different embodiment, although it may. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

"Correlated to" as used herein refers to compared to.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "gene" refers to a nucleic acid (e.g., DNA) sequence that comprises coding sequences for the production of a polypeptide, precursor, or RNA (e.g., rRNA, tRNA, sRNA, microRNA, lincRNA). The polypeptide can be encoded by a full-length coding sequence or by any portion of the coding sequence so long as the desired activity or functional properties (e.g., enzymatic activity, ligand binding, signal transduction, immunogenicity, etc.) of the full-length or fragment are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (e.g., mutated, added in multiple copies, linked to non-native regulatory sequences, etc.). Heterologous genes are distinguished from endogenous genes in that the heterologous gene sequences are typically joined to DNA sequences that are not found naturally associated with the gene sequences in the chromosome or are associated with portions of the chromosome not found in nature (e.g., genes expressed in loci where the gene is not normally expressed).

As used herein, the term "oligonucleotide," refers to a short length of single-stranded polynucleotide chain. Oligonucleotides are typically less than about 300 residues long (e.g., between 15 and 100), however, as used herein, the term is also intended to encompass longer polynucleotide chains. Oligonucleotides are often referred to by their length. For example, a 24-residue oligonucleotide is referred to as a "24-mer." Oligonucleotides can form secondary and tertiary structures by self-hybridizing or by hybridizing to other polynucleotides. Such structures can include, but are not limited to, duplexes, hairpins, cruciforms, bends, and triplexes.

The term "homology" and "homologous" refers to a degree of identity. There may be partial homology or complete homology. A partially homologous sequence is one that is less than 100% identical to another sequence.

As used herein, the terms "complementary" or "complementarity" are used in reference to polynucleotides (e.g., a sequence of nucleotides such as an oligonucleotide or a target nucleic acid) related by the base-pairing rules. For example, for the sequence "5'-A-G-T-3'" is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

In some contexts, the term "complementarity" and related terms (e.g., "complementary", "complement") refers to the nucleotides of a nucleic acid sequence that can bind to another nucleic acid sequence through hydrogen bonds, e.g., nucleotides that are capable of base pairing, e.g., by Watson-Crick base pairing or other base pairing. Nucleotides that can form base pairs, e.g., that are complementary to one another, are the pairs: cytosine and guanine, thymine and adenine, adenine and uracil, and guanine and uracil. The percentage complementarity need not be calculated over the entire length of a nucleic acid sequence. The percentage of complementarity may be limited to a specific region of which the nucleic acid sequences that are base-paired, e.g., starting from a first base-paired nucleotide and ending at a last base-paired nucleotide. The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

Thus, in some embodiments, "complementary" refers to a first nucleobase sequence that is at least 60%, 65%, 70%, 75%, 80%, 85%, 90%, 95%, 97%, 98%, or 99% identical to the complement of a second nucleobase sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases, or that the two sequences hybridize under stringent hybridization conditions. "Fully complementary" means each nucleobase of a first nucleic acid is capable of pairing with each nucleobase at a corresponding position in a second nucleic acid. For example, in certain embodiments, an oligonucleotide wherein each nucleobase has complementarity to a nucleic acid has a nucleobase sequence that is identical to the complement of the nucleic acid over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases.

As used herein, a "double-stranded nucleic acid" may be a portion of a nucleic acid, a region of a longer nucleic acid, or an entire nucleic acid. A "double-stranded nucleic acid" may be, e.g., without limitation, a double-stranded DNA, a double-stranded RNA, a double-stranded DNA/RNA hybrid, etc. A single-stranded nucleic acid having secondary structure (e.g., base-paired secondary structure) and/or higher order structure comprises a "double-stranded nucleic acid". For example, triplex structures are considered to be "double-stranded". In some embodiments, any base-paired nucleic acid is a "double-stranded nucleic acid"

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (e.g., a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (i.e., the oligonucleotide or polynucleotide may be single-stranded), but may contain both the sense and anti-sense strands (i.e., the oligonucleotide or polynucleotide may be double-stranded).

As used herein, the term "purified" or "to purify" refers to the removal of components (e.g., contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

Preferred methods and materials are described below, although methods and materials similar or equivalent to those described herein can be used in practice or testing of the present disclosure. The materials, methods, and examples disclosed herein are illustrative only and not intended to be limiting.

### 2. Gap Editors

CRISPR-based genome editing tools have found widespread application, relying on their easily programmable targeting and robust activity. Early use of these CRISPR-based tools has focused on the ability of Cas nucleases to cleave DNA. In the process of repairing the cleaved DNA, a genomic edit is introduced. DNA cleavage is, however, among the most toxic events a cell can endure. DNA cleavage sets off cellular alarm systems which lead to mutations, DNA rearrangements, or loss of cellular viability. Subsequent CRISPR-Cas genome editing tools have sought to minimize these toxic effects by instead introducing single-stranded nicks or directly modifying DNA via an enzyme. Still, these newer methods exhibit a limited range of edits that can be introduced and can suffer from undesired insertions, deletions, and mutations.

Embodiments of the present disclosure demonstrate that efficient non-toxic genome modification can be performed through the introduction and repair of single-stranded DNA gaps. Previous work has demonstrated that site-specific introduction of abasic sites into DNA drives homology-dependent gap recombination. By introducing an ectopic DNA repair template, genome modification can be achieved at DNA sequences adjacent to the introduced abasic site. However, in some cases, this approach can be dependent on the stabilization of the abasic sites. Therefore, embodiments of the present disclosure include the development of a system to induce homology-dependent gap repair with the addition of stable chemical groups onto DNA. This modified DNA is not recognized or repaired by cellular glycosylases, which increases lesion stability, and drives homology-dependent gap repair. Site specific DNA targeting is achieved by fusion of the modification enzyme to a Cas effector, and in some cases, the rate of genome modification can be increased using a Cas effector to nick the target DNA strand. As described further herein, the combination of nicking and DNA modification can have synergistic effects on genome modification because they mutually abrogate sister chromatid repair.

As would be recognized by one of ordinary skill in the art, the original and most widely used CRISPR-Cas genome editing technology relies on Cas nucleases introducing a double strand break which is then repaired through homologous recombination via an editing template, similar to gap editors. While broadly applied, the toxicity of double-stranded breaks and their tendency to drive mutations or chromosomal rearrangements is a consistent challenge for therapeutic applications. These DNA breaks are highly toxic (particularly in bacteria) and often lead to error prone repair via non-homologous end joining pathways. Cleave and repair is potentially the best known way to insert large segments of DNA, which is important for many scientific and industrial applications.

Additionally, base editors can be used in an effort to avoid toxicity by enzymatically converting nucleotides from one to another. For example, cytosine can be converted to thymine and adenine can be converted to guanine. However, these base editors can only change one or a few nucleotides at a time, and they have to be carefully targeted to avoid undesired editing. Furthermore, base editors are mutagenic, meaning that untargeted nucleotides are more likely to be incorrectly replicated while the base editors are being used. Base editors are also constrained by the availability of target sequences. Compared to other techniques, base editors are relatively efficient and only rely on nicking a single strand of DNA, as opposed to cutting both strands.

Prime editors have only recently been described. Based on recent publications, it seems that prime editors are relatively efficient, and they have a major advantage in that they use a very small repair template which is encoded on the backbone of the Cas9 single guide RNA. While touted as a double-strand break-free technique, efficient prime editing still involves nicking both strands of DNA in relatively close (<200 bp) proximity. This dual nicking is only moderately less toxic than the cleave-and-repair approach. Error-prone insertions and deletions still occur in mammalian cells as a result of dual nicking. It is unclear to what degree prime editors will function in prokaryotes. It also is unclear whether any mutagenic side effects might occur in their application, though their CRISPR-dependent off-target activity is muted.

As compared to other techniques, gap editors have the least amount of data pertaining to their use. Regardless, gap editors seem to have minimal toxic effects, as described further herein; and some experiments show no detectable toxicity. The lack of toxicity may be especially advantageous for therapeutic applications, as low toxicity typically indicates a low rate of undesired mutations, DNA insertions, or DNA rearrangements. Also, multiplex engineering is commonly hampered by toxicity (particularly in bacteria). For *in vivo* therapeutics, gap editors would likely suffer from the same DNA and protein delivery issues as all of the other CRISPR-Cas methods, although there are newer delivery platforms that allow co-delivery of RNPs with repair templates.

Embodiments of the present disclosure include compositions, systems, kits, and methods for targeted modification of a nucleic acid in a genome. In accordance with these embodiments, the present disclosure provides gap editors and gap editor complexes that generally include a DNA-recognition domain and a DNA-modifying domain. As described further in the Examples provided herein, gap editors and gap editor complexes facilitate programmable DNA targeting with a DNA-recognition domain that is functionally coupled to a DNA-modifying domain to drive genome modification via homology-directed gap repair. In some embodiments, the DNA-recognition domain binds a DNA target sequence in the genome, and the DNA-modifying domain induces formation of a replication blocking moiety on at least one nucleotide in the genome. Targeting of gap editors in a specific orientation generates persistent DNA gaps, thereby improving gap editor efficiency.

In some embodiments, the DNA-recognition domain and the DNA-modifying domain are functionally coupled. Functionally coupled includes any means for integrating the DNA-recognition domain and the DNA-modifying domain at a specific target site for the purposes of functioning as genome editors. In some embodiments, "functionally coupled," includes but is not limited to polypeptide fusions, peptide tags, peptide linkers, RNA tags, and any combinations thereof. For example, a gap editor or gap editor complex can include a DNA-recognition domain that is fused to a DNA-modifying domain (e.g., a fusion polypeptide). The DNA-recognition domain of the gap editor fusion protein recognizes a specific site (e.g., nucleic acid sequence in a genome) in a target nucleic acid, and the DNA-modifying domain is then capable of modifying one or more nucleic acids in or around the target site to facilitate genome modification.

As would be recognized by one of ordinary skill in the art based on the present disclosure, the gap editor complexes described herein can be used to modify any part of a genome of a cell. For example, the gap editor complexes of the present disclosure can be used to target a specific site in a genome to generate a desired site-specific modification, and/or the gap editor complexes of the present disclosure can be used to target one or more specific sites in a genome to generate a modification that results in the addition, exchange, and/or removal of a portion of the genome. Additionally, the gap editor complexes of the present disclosure can be used to target any region of a gene, including but not limited to, an open reading frame, an intron, an exon, an intron-exon boundary, a functional non-coding region, and any upstream and/or downstream DNA/gene regulatory sequences. The terms "DNA/gene regulatory sequences," "control elements," and "regulatory elements," used interchangeably herein, refer to transcriptional and translational control sequences, such as promoters, enhancers, polyadenylation signals, terminators, protein degradation signals, and the like, that provide for and/or regulate transcription of a non-coding sequence or a coding sequence and/or regulate translation of an encoded polypeptide. Thus, the gap editor complexes of the present disclosure can be used to generate modifications in the genome that result in altered gene expression patterns and/or activity (e.g., upregulation or downregulation).

In some embodiments, the DNA-recognition domain and the DNA-modifying domain do not comprise a fusion polypeptide (e.g., do not form a single fusion polypeptide or protein). In some embodiments, the DNA-modifying domain is recruited to the gap editor or gap editor complex by the DNA-recognition domain. For example, the DNA-recognition domain of the gap editor can recruit the DNA-modifying domain via a protein-protein interaction. In some embodiments, this recruitment is facilitated by a tag or linker that serves to recruit and functionally couple the DNA-modifying domain to the DNA-recognition domain at a specific site of a target nucleic acid. Other means for recruiting and functionally coupling the DNA-modifying domain to the DNA-recognition domain based on protein-protein interactions can also be used, including but not limited to, antigen-antibody interactions (e.g., the DNA-modifying domain fused to an antigen binding domain and the DNA-recognition domain fused to the corresponding antigen), protein tags (e.g., a streptavidin-biotin interaction), a peptide and single chain variable antibody fragment, a split-protein system, or any ligand-receptor interaction. In other embodiments, the DNA-modification domain can be integrated into the DNA-recognition domain, such as, for example, by replacing the HNH domain of Cas9 with the DNA-modification domain, or inserting the DNA-modification domain into the PAM-interacting domain.

In other embodiments, the DNA-modifying domain is recruited to the gap editor or gap editor complex by an interaction with a nucleic acid. For example, a guide RNA molecule that interacts with the DNA-recognition domain to bind a site in a target nucleic acid can include a sequence and/or structure that binds the DNA-modifying domain (e.g., a scaffold domain). In some embodiments, the sequence and/or structure on the guide RNA includes domains that are recognized by RNA binding proteins. In some embodiments, the DNA-modifying domain is fused to an RNA-binding protein that is recruited to the gap editor or gap editor complex via binding to the domain on the guide RNA. Other means for recruiting and functionally coupling the DNA-modifying domain to the DNA-recognition domain based on RNA-binding interactions can also be used. In some embodiments, the guide RNA is extended to encode an RNA aptamer that recognizes different proteins or protein domains, such as the MS2 coat protein, Tat, or Rev. The recognized protein or protein domain is then fused to the DNA-modifying domain. The guide RNA can encode multiple copies of the same protein-binding domain or different protein-binding domains. These protein-binding domains can be incorporated into different parts of the gRNA, such as through the loop of the gRNA or sgRNA or at the 3' end of the sgRNA.

As described further herein, the gap editor complexes of the present disclosure can be used to generate various modifications in the genome of an organism or cell, such as through the mechanism of homology directed repair. In some embodiments, genome modifications using the gap editors of the present disclosure can generate specific nucleotide modifications ranging from a single nucleotide change to large insertions or deletions. In some embodiments, the gap editor complexes of the present disclosure can be used to add or remove large sequences of DNA through the use of more than one guide RNA sequence to target distinct sites in the genome (e.g., generate large genomic deletions by removing the sequence between two gRNA target sites and/or inserting an exogenous DNA sequence). In some embodiments, multiple gRNAs can be used to target multiple sites in a genome to generate any number of desired modifications in a genome (e.g., multiplexing). As would be recognized by one of ordinary skill in the art based on the present disclosure, any type of genetic modification can be achieved using the gap editor complexes of the present disclosure in any cell type and/or organism, regardless of how the gap editor complexes are delivered to the cell (e.g., transformation), including in vitro, ex vivo, or in vivo methods of delivery. A general discussion of these methods can be found in Ausubel, et al., Short Protocols in Molecular Biology, 3rd ed., Wiley & Sons, 1995.

*DNA-Recognition Domains.* In accordance with these embodiments, the DNA-recognition domains of the gap editors or gap editor complexes of the present disclosure include use of a sequence-specific nucleic acid binding component (e.g., molecule, biomolecule, or complex of one or more molecules and/or biomolecules) to target a specific nucleic acid target site). In some embodiments, the DNA-recognition domain includes at least one Cas protein or fragment thereof lacking nuclease or deoxyribonuclease activity. In some embodiments, the DNA-recognition domain comprises a complex of Cas proteins lacking nuclease or deoxyribonuclease activity. In some embodiments, the DNA-recognition domain includes at least one Cas protein or a complex of Cas proteins that exhibit nickase activity, including but not limited to, a Cas9 or a Cas12a with nickase activity.

In some embodiments, the Cas protein or Cas protein complex comprises a Type I Cascade, a Type II Cas9, a Type IV effector module, a Type V Cas12, a Cas9-related IscB, a Cas9-related TnpB, and combinations thereof. Cascade is a set of Cas proteins that form a stable complex in different proportions with the guide RNA. The gRNA is normally encoded within a CRISPR array, where the Cas6 protein of the complex cleaves a hairpin in the transcribed repeat. The other proteins then form around the freed RNA. The fully-formed complex binds target DNA flanked by a protospacer-adjacent motif (PAM) encoded on the 5' end of the non-target strand. Upon target recognition, the complex then recruits the Type I endonuclease Cas3 to nick and processively degrade the non-target strand in the 3'-to-5' direction, although the complex will stably bind target DNA in the absence of Cas3. The specific number and stoichiometry of the proteins in Cascade varies between CRISPR-Cas subtypes, such as Cas8c(1):Cas5c(1):Cas7(7) for the I-C sub-type and Cse1(1):Cse2(2):Cas5e(1):Cas7(6):Cas6e(1) for the I-E sub-type. Furthermore, these proteins can be fused to recapitulate the complex with fewer expressed polypeptides, and the Cas6 protein is dispensable if the guide RNA is expressed as a processed CRISPR RNA. Varying the length of the guide sequence within the gRNA can further alter the protein stoichiometry of Cascade and can change the length of the R-loop and displaced DNA strand. Cas9 is a single-effector nuclease that binds target DNA with a PAM encoded on the 3' end of the non-target strand. Bound DNA is then nicked on opposite strands through the HNH and RuvC domains of Cas9, resulting in a double-stranded break. The gRNA utilized by Cas9 is normally encoded with a CRISPR array, where a trans-activating crRNA (tracrRNA) pairs with the transcribed repeat, and the RNA duplex is cleaved by the endoribonuclease RNase III. The resulting processed crRNA:tracrRNA duplex is bound by Cas9 and directs DNA targeting. The crRNA:tracrRNA duplex can be fused to form a single guide RNA (sgRNA). Cas12 represents a diverse family of Cas nucleases designated by their sub-type (e.g. Cas12a, Cas12e) and have been given alternative names such as Cpf1, C2c1, CasX, or Cas14a. Cas12 nucleases target DNA with a PAM encoded on the 5' end of the non-target strand, with the nuclease's RuvC domain nicking the both the target and non-target stranded to create a staggered double-stranded break with a 5' overhang. The gRNA is encoded within a CRISPR array and can be processed from the transcribed CRISPR array through one of two mechanisms depending on the nuclease: cleavage of a hairpin within the repeat by a riboendonucleolytic domain with the Cas12 nuclease (e.g. Cas12a), or pairing of the transcribed repeat with a tracrRNA that is subsequently cleaved by RNase III. As a result, the gRNA can be readily expressed in its processed form when the nuclease alone is responsible for crRNA processing, the gRNA can be expressed as an sgRNA when a tracrRNA is involved in crRNA processing.

In some embodiments, the DNA-recognition domain comprises a deoxyribonuclease-inactivated Cas9 ("dCas9"), which can be generated by introducing deactivating mutations within the HNH domain and the RuvC domain of the protein. In some embodiments, the DNA-recognition domain comprises a deoxyribonuclease-inactivated Cas12a ("dCas12a"), which can be generated by introducing deactivating mutations within at least one of the RuvC domains, such as RuvC-I. Alternatively, a guide RNA that is truncated on the PAM-distal end or contains mismatches with the target can allow DNA binding but not DNA nicking or cleavage by an otherwise catalytically active Cas nuclease.

In some embodiments, various other DNA-recognition domains can also be used in the gap editor complexes of the present disclosure. For example, certain embodiments of the compositions and methods described herein do not require guide RNAs to effectuate efficient genome editing and modification. As described above, these gap editor complexes include, but are not limited to, meganucleases, zinc-fingers (ZFs), and transcription activator-like effectors (TALEs). In some embodiments, the DNA-recognition domains of the present disclosure can include a meganuclease. Meganucleases can be used to replace, eliminate or modify sequences in a targeted manner and their recognition target sequence can be altered through protein engineering. Meganucleases can be used to modify all genome types, whether bacterial, plant or animal, and they are amendable to in vivo delivery due to their relatively small sizes. The high degree of target specificity of meganucleases allows for a concomitantly high degree of precision and much lower cell toxicity. However, targeting novel sequences is challenging due to the limited number of the meganuclease available.

In some embodiments, the DNA-recognition domains of the present disclosure can include zinc-fingers (ZFs). ZFs are fusions of the nonspecific DNA cleavage domain from the restriction endonuclease with zinc-finger proteins. ZFNs can target specific DNA sequences and this allows the ZFN to address and accurately change unique sequences inside a target organisms. A single zinc-finger is made up of around 30 amino acids in a conserved ββα figure. Some amino acids on the surface of the α-helix usually select three base pairs within the DNA smooth groove. Zinc-finger proteins have become an important framework for the design of custom DNA-binding proteins, as the development of unnatural arrays with more than three domains have become available, along with the development of a highly-conserved linker sequence that allows synthetic zinc-finger proteins, which recognize DNA sequences 9 to 18 bps in length.

In some embodiments, the DNA-recognition domains of the present disclosure can include transcription activator-like effectors (TALEs). TALES are very versatile and can be combined with numerous effector domains to affect genomic structure and function, including nucleases, transcriptional activators and repressors, recombinases, transposases, DNA and histone methyltransferases, and histone acetyltransferases. TALENs are transcription activator-like effector nucleases which are fusions of the Fok1 cleavage domain and DNA-binding domains. TALEs are naturally occurring proteins from bacteria with genus *Xanthomonas* and contain DNA-binding domains made up of a series of 33-35 amino acid repeat domains that each recognize a single base pair. TALE specificity is determined by two hypervariable amino acids that are known as repeat-variable di-residues (RVDs). Numerous effector domains have been made available to fuse to TALE repeats for targeted genetic modifications, including nucleases, transcriptional activators, and site-specific recombinases. While the single base recognition of TALE-DNA binding repeats affords greater design flexibility than triplet-confined zinc-fingers, the cloning of repeat TALE arrays presents an elevated technical challenge due to extensive identical repeat sequences.

***DNA-Modifying Domains.*** In some embodiments, the DNA-modifying domain catalyzes the formation or addition of at least one replication blocking moiety to at least one nucleotide in the DNA target sequence. In some embodiments, the DNA-modifying domain blocks DNA replication by adding the replication blocking moiety to at least one nucleotide in the DNA strand complementary to the DNA target sequence. In some embodiments, the DNA-modifying domain blocks DNA replication by adding the replication blocking moiety to at least one nucleotide in the DNA strand containing the DNA target sequence. In some embodiments, the DNA-modifying domain blocks DNA replication by adding the replication blocking moiety to both a nucleotide in the DNA strand complementary to the DNA target sequence and a nucleotide in the DNA strand containing the DNA target sequence.

In some embodiments, the DNA-recognition domain induces a single-stranded break in the DNA target strand (via nickase activity), and the DNA-modifying domain adds the replication blocking moiety to at least one nucleotide in the DNA strand complementary to the DNA target sequence. In some embodiments, the DNA-modifying domain catalyzes addition of ADP ribose to a thymine or guanine nucleotide. In some embodiments, the DNA-modifying domain comprises a DarT enzyme or a functional fragment, derivative, or variant thereof. In some embodiments, the DarT enzyme has been engineered to have reduced DNA binding, increased specificity to single-stranded DNA, and/or decreased enzymatic activity. DarT homologs (and any fragments, derivatives, or variants thereof) that can be used in the various embodiments disclosed herein include, but are not limited to, those provided in Table 1 below. In some embodiments, the DNA-modifying domain comprises a Scabin enzyme or a functional fragment, derivative, or variant thereof. In some embodiments, the Scabin enzyme has been engineered to have reduced DNA binding, increased specificity to single-stranded DNA, and/or decreased enzymatic activity. Scabin homologs (and any fragments, derivatives, or variants thereof) that can be used in the various embodiments disclosed herein include, but are not limited to, those provided in Table 1 below. In some embodiments, the Mom enzyme has been engineered to have reduced DNA binding, increased specificity to single-stranded DNA, and/or decreased enzymatic activity. Mom homologs (and any fragments, derivatives, or variants thereof) that can be used in the various embodiments disclosed herein include, but are not limited to, those provided in Table 1 below.

**Table 1: DarT homologs and their corresponding UniProt reference numbers.**

| **DarT Homologs UniProt Ref. No.** | **Scabin Homologs UniProt Ref. No.** | **Mom Homologs UniProt Ref. No.** |
|---|---|---|
| A0A3Y1AXM4 | P06018 | A0A7G7C6V3 |
| A0A0M9E739 | P08794 | A0A6G3TAN8 |
| A0A6H3DQB7 | A0A0A6ZQD1 | A0A4Q4DBR5 |
| A0A2D5FEV0 | A0A747H2I6 | A0A7K2MJA2 |
| A0A009QG24 | F3WIW6 | A0A1I5DGQ6 |
| A0A1Y1QH60 | A0A5Y2Q823 | A0A0N1NCQ4 |
| A0A1H2WEE3 | A0A5T7EP05 | A0A117EGR9 |
| A0A365SDE9 | A0A5X5CI68 | A0A7K3F6T9 |
| A0A2T2YIK3 | A0A736I828 | A0A7K3QWB6 |
| U7P928 | Q32F84 | A0A4Z1DI83 |
| A0A0B7IUM8 | Q53980 | A0A3N6FY95 |
| A0A1C4E3X9 | A0A0A6ZUU6 | A0A7K2GZ37 |
| UPI0009FFBBAF | A0A090NAC5 | A0A1X1N6K7 |
| UPI0011835755 | A0A734N076 | A0A286EGA2 |
| UPI000A066936 | A0A5Z9VNA9 | A0A1H1REA6 |
| G7TGB0 | A0A0E1SZ91 | L8PML2 |
| A0A109CYV8 | A0A718VE50 | A0A401MBD2 |
| A0A1J1EN49 | A0A3V2P1F8 | A0A505DEP0 |
| A0A6N8HLA1 | F4ST91 | A0A5C4V5D6 |
| A0A0F9A3N8 | A0A0L1BX31 | A0A6G2X7S2 |
| A0A0F9ID55 | A0A6N8K5P2 | A0A231PCB5 |
| UPI00146D40AF | A0A2X2IFR7 | A0A117RXM5 |
| UP10015EC5998 | Q32I99 | A0A854W491 |
| X0U0F3 | A0A398TE36 | A0A7K2M2S6 |
| A0A1F2WQI4 | A0A366YZA8 | A0A845VQ73 |
| A0A4Q9B657 | A0A2X3K063 | A0A444QU29 |
| A0A1A6KRV4 | A0A6C9HIT1 | A0A126Y4C7 |
| A0A2W0FJ31 | F3WLY8 | A0A3Q9KV10 |
| UP100131E585C | A0A4D9HQK3 | A0A8B0F419 |
| A0A521GSZ3 | A0A7B2BKV1 | A0A1B1MHN6 |
| A0A3C0UL77 | A0A659GZW5 | A0A0M8WMD9 |
| A0A128EDT6 | A0A376P4X4 | A0A3S9MED3 |
| A0A0S4KU33 | A0A829JC85 | A0A7G1P3D5 |
| A0A0K8QWE7 | A0A8A5HYQ3 | L7FDM7 |
| A0A1I2BV64 | A0A2Y0KN27 | A0A7H0IBA3 |
| A0A074JDH1 | A0A6C8GMD6 | A0A1V4ECW4 |
| S6GJD4 | A0A855SJL4 | A0A7K2GG48 |
| UPI0003A70E4B | A0A1X3JSV2 | A0A6B3CTN6 |
| A0A1G7QJ47 | F3WRA7 | A0A5J6EZ40 |
| A0A1G7XXY4 | A0A0L1BYZ7 | A0A3N6F8E7 |
| A0A077F777 | A0A2X9WZ16 | A0A2C8XEE2 |
| A1WMK8 | A0A5T6ITA7 | A0A0M4DAA4 |
| M5AN74 | A0A5Z9MRI6 | A0A7M3P2N8 |
| A0A0X1T5G3 | A0A774N8E0 | A0A6B3QVN7 |
| A0A2A9FUD7 | A0A653FTS2 | A0A6GAV177 |
| UPI000BE34E2B | A0A7D7IKR8 | A0A7D8B5M0 |
| A0A021VVM8 | A0A793PNZ0 | A0A7Y6CBB1 |
| UPI0009EEB1C1 | A0A3Y6RE47 | A0A542HUQ5 |
| A0A212J8X1 | A0A7U8TEQ3 | A0A1Q5GYR2 |
| A0A143XZK3 | A0A7T2JHL6 | A0A7K2JG06 |
| A0A2D8CA1 | A0A2X2K6P7 | A0A0N1FX41 |
| A0A2M6ZMD7 | A0A828BG22 | A0A1Q5KVP4 |
| D4ZX17 | A0A243UWN1 | A0A421LHY3 |
| A0A1V2YE96 | A0A7D3UWA8 | A0A1C4SR45 |
| UPI0004795285 | A0A7D3QJ09 | A0A7H8P376 |
| A0A2I1RLA3 | A0A6I4LGA3 | A0A4V2U6X2 |
| A0A069DSZ4 | A0A833L0X9 | A0A2A3GZG2 |
| A0A1B1TKQ4 | A0A844VV27 | D6K1C1 |
| A0A1M5YS26 | A0A2X3A730 | A0A7H0HXY6 |
| UPI001081FF81 | A0A7D3UWP6 | A0A7K2VU35 |
| UPI00058ECA86 | A0A7D3QJ52 | A0A6I6RSN3 |
| A0A439F9A2 | A0A789M987 | A0A6H1NCH2 |
| A0A0K6IM62 | A0A479J9Y1 | A0A2N3K2V7 |
| A0A3M1TMP6 | A0A1X3J0Y0 | A0A7K2ULE5 |
| A0A4Z0LYH6 | A0A6L7FCA8 | V4I776 |
| UPI000CEA333A | A0A398QB61 | A0A5J6IH58 |
| A0A0E9M297 | E7STE3 | A0A2Z5K877 |
| A0A4R4QZG6 | A0A4Z0T8W4 | A0A3N4ZXP2 |
| A0A5C4P404 | A0A7G6K9Y2 | A0A2P8A6J8 |
| A0A2E5CCR5 | A0A2Y4XYF1 | A0A3R9UHD1 |
| A0A0F9FER9 | F3WJW5 | A0A6B3DTW3 |
| A0A6L6K3W2 | F5NRV4 | A0A7K3E8Z7 |
| A0A2N0GBR2 | A0A2S8JPX1 | A0A5P8KCS9 |
| A0A3D0ST31 | B3X6Z6 | A0A6G3W7K4 |
| A0A086DYY8 | A0A826W5G8 | A0A7S7X9R1 |
| UP100138FF367 | A0A656BX08 | A0A5Q4TE11 |
| UPI0009E9D184 | A0A2T3SJ22 | A0A2G7F715 |
| A0A0Q4H114 | A0A5E8GB30 | A0A2P8PUY9 |
| A0A1C6SGK0 | F3WQG1 | A0A7H8H741 |
| A0A2W5HPA9 | A0A376FNN0 | A0A6I5D8I2 |
| A0A2P8KB33 | A0A3U8JEK9 | A0A1I6W4M7 |
| UPI0009C0D9CF | I6CWT9 | A0A6A0BTB8 |
| A0A4S5BBM9 | A0A3P6KJV4 | A0A1V9KFP9 |
| A0A2G6E1H5 | A0A3U5WED1 | A0A4Q7Z2V3 |
| A0A2V4F7G0 | B3X4P5 | A0A0T1UEA6 |
| UPI000C6F263C | E7SSY4 | A0A5N6A8S8 |
| UPI0004B149FA | E0J798 | A0A6G3ABW5 |
| UPI000BF71297 | A0A1X0YFM5 | A0A0B5DFX2 |
| A0A0S8HVY0 | A0A854VRL6 | A0A540PEE8 |
| A0A081BFQ8 | A0A379ZXH3 | A0A2M9I3D9 |
| A0A2T3K4E8 | A0A6D0FK22 | A0A086GVM1 |
| UPI00140B28F9 | A0A193LSI7 | A0A250VCC4 |
| A0A450ZNU6 | A0A746IF37 | A0A7K2WAZ7 |
| A0A434FTJ1 | A0A6X7AJ78 | A0A7K2WPB2 |
| UPI001575F606 | A0A826N5K3 | A0A6G9GX41 |
| UPI00131CDEC9 | A0A6D0FPQ2 | A0A5R9FQN8 |
| UPI000E34E22D | | A0A380MTQ1 |
| UPI001575232E | | A0A2A3J625 |
| A0A2V5QXN0 | | A0A1D8SUV6 |
| A0A1H3GAX0 | | A0A1S2P573 |
| A0A1G6MG07 | | |
| A0A2A5E1Y0 | | |
| A0A662P7C8 | | |
| A0A6L7A0Y8 | | |
| A0A1I2KC92 | | |
| A0A5Q4HAE6 | | |
| A0A0G3UZG3 | | |
| A0A1V3SKR4 | | |
| A0A0D5M555 | | |
| UPI0003F90624 | | |
| X0QNL7 | | |
| UPI0009DA5757 | | |
| UPI0002EF3C8F | | |
| A0A399YQF2 | | |
| A0A2D3M0N6 | | |
| A0A087MEL2 | | |
| A0A1J5TVU6 | | |
| UPI00143CD06E | | |
| A0A3G6X2L4 | | |
| A0A369I9T2 | | |
| UPI0015935B35 | | |
| A0A699RGA3 | | |
| A0A0Q8DZI6 | | |
| A0A1T4V1K5 | | |
| UPI00081C8979 | | |
| A0A0F9B5C2 | | |
| A0A6I7PSY2 | | |
| UPI000C7E3428 | | |
| UPI00066E6B23 | | |
| A0A0K8QWM3 | | |
| A0A1F7S2E1 | | |
| UPI00106D6FED | | |
| A0A0N7A0X9 | | |
| A0A3B0TNW4 | | |
| A0A1B3LKQ8 | | |
| A0A1V0QE61 | | |
| UPI000A33B150 | | |
| UPI00145C4C23 | | |
| A0A654U036 | | |
| UPI000BB413AC | | |
| A0A2J6NE32 | | |
| A0A4P5X2M7 | | |
| J1H157 | | |
| A0A562Y4W9 | | |
| A0A222SFK8 | | |
| A0A3L7NYM4 | | |
| A0A3B8NG16 | | |
| UPI0014451E71 | | |
| A0A398DRP6 | | |
| A0A1H3ZRX1 | | |
| U6H3Z0 | | |
| A0A2E0XMC9 | | |
| A0A3Q2ZTE2 | | |
| A0AIQ5T734 | | |
| J1Y9X6 | | |
| A0A1X9SM09 | | |
| A0A4U0XTT2 | | |
| A0A151NT80 | | |
| A0A2E6Y7V9 | | |
| A0A0F9A8D5 | | |
| A0A562XL28 | | |
| UPI000A32FC88 | | |
| UPI001295C460 | | |
| A0A059ZR15 | | |
| A0A2K1Z809 | | |
| A0A4R4IBZ9 | | |
| A0A193FXT9 | | |
| A0A328V872 | | |
| F9FTA7 | | |
| A0A2A4PLD2 | | |
| A0A6B1F5X5 | | |
| A0A0N1D5X2 | | |
| UPI00114F1E30 | | |
| A0A6A4SK98 | | |
| A0A416G6Z1 | | |
| A0A2D8R8I3 | | |
| A0A0F9S1T0 | | |
| A0A2H3U3T0 | | |
| A0A0J6SV50 | | |
| A0A3M1HEV7 | | |
| A0A1Q4RC56 | | |
| A0A1H9ZTD0 | | |
| M5XRC1 | | |
| A0A4P8RI99 | | |
| A0A287ISE0 | | |
| A0A3M1HHN8 | | |
| A0A1I8FRJ7 | | |
| A0A1Q9P5U5 | | |
| U2QX64 | | |
| UPI000B773353 | | |
| UP10004140561 | | |
| A0A0K2R4T0 | | |
| A0A1Z4JP41 | | |
| A0A2W6XRC8 | | |
| A0A1B7W4E5 | | |
| A0A367V7P0 | | |
| A0A1U8LNE6 | | |
| A0A165DJ89 | | |
| A0A0U1M3L7 | | |
| A0A109CYU7 | | |
| A0A3C1G1M6 | | |
| A0A6A6P153 | | |
| A0A078K042 | | |
| A0A0F9E1N9 | | |
| A0A6L2M8A9 | | |
| A0A384DPW3 | | |
| UPI0006B07CD7 | | |
| UP10012B63E61 | | |
| A0A679F6I9 | | |
| M4EQE8 | | |
| A0A2N2MUF5 | | |
| A0A1I8J2P8 | | |
| A0A699GHG3 | | |
| A0A061RT73 | | |
| A0A4Q5Z9M4 | | |
| A0A0C3CY40 | | |
| A0A562LHY2 | | |
| A0A1H2WEE3 | | |
| A0A1F9LMB0 | | |
| A0A6B0VHE9 | | |
| A0A1W9IKF6 | | |
| A0A1J4WMX2 | | |
| A0A4Q6DQE0 | | |
| UPI00131D0A3D | | |
| A0A5Q0PIV9 | | |
| UPI0014767B89 | | |
| A0A0D9YA74 | | |
| UPI0003C8CEDA | | |
| A0A4P7QDQ0 | | |
| A0A1I3L2R8 | | |
| A0A060SSG3 | | |
| UPI0011DDD910 | | |
| A0A2V9JXV7 | | |
| A0A0D0ARU6 | | |
| T1EWK1 | | |
| A0A1G8HQU1 | | |
| A0A1C6SGK0 | | |
| A0A238YN77 | | |
| A0A0C4ETD4 | | |
| UPI0015A92654 | | |
| A0A218WZU7 | | |
| L9L887 | | |
| A0A0T9QHP2 | | |
| A0A1H4B661 | | |
| A0A4D9EGJ1 | | |
| UPI00145515B0 | | |
| A0A1V2LC08 | | |
| A0A6F9DHT9 | | |
| A0A1E3NPN8 | | |
| A0A1X6MJD8 | | |

As would be recognized by one of ordinary skill in the art based on the present disclosure, other DNA-modifying domains/enzymes can be used in the gap editors and gap editor complexes of the present disclosure to induce formation of a replication blocking moiety at a given target site. For example, in some embodiments, the DNA-modifying domain/enzyme can include, but is not limited to, any of the following enzymes (or functional fragments, derivatives, or variants thereof): Pierisin, Scabin, Cell cycle and apoptosis regulator 1 (CARP-1), SCO5461 protein (ScARP), adenine modification enzyme, acetyltransferase, amino acid transferase, nucleotidyl transferase, uridyltransferase, acyltransferase, ADP-ribosyltransferase, methylthiotransferase, N-acetyl transferase 10, tRNA(Met) cytidine acetyltransferase (TmcA), tRNA cytidine acetyltransferase, GCN5-related N-acetyltransferase, lysidine synthase, m⁷G methyltransferase, N6C carbamoylmethyltransferase (Mom), N6-adenosine threonylcarbamoyltransferase, threonyl carbomyl transferase or threonyl carbomyl transferase complex, TsaB-TsaE-TsaD (TsaBDE) complex, tRNA N6-adenosine threonylcarbamoyltransferase (Qri7, Tcs4), methyltransferase, ATrm5a, tRNA:m¹G/imG2 methyltransferase, tRNA (adenosine(37)-N6)-dimethylallyltransferase, tRNA dimethylallyltransferase (MiaA), and isopentenyltransferase.

In some embodiments, the DNA-modifying domain used in the gap editor complexes of the present disclosure includes a catalytic domain (or a functional fragment, derivative, or variant thereof) that induces formation of a replication blocking moiety on at least one nucleotide in a genome. In some embodiments, the catalytic domain includes a portion of a DarT enzyme that is sufficient to carry out ADP-ribosylation of a target nucleic acid, as described further herein. In some embodiments, the catalytic domain includes a portion of a Scabin enzyme that is sufficient to carry out ADP-ribosylation of a target nucleic acid, as described further herein.

For example, the catalytic domain of the DNA-modifying domain that can be used in the gap editor complexes of the present disclosure includes, but is not limited to, any sequence having at least 70% amino acid identity with any of SEQ ID NOs: 18-21. In some embodiments, the DNA-modifying domain includes a catalytic domain having at least 75% amino acid sequence identity with SEQ ID NO: 18. In some embodiments, the DNA-modifying domain includes a catalytic domain having at least 80% amino acid sequence identity with SEQ ID NO: 18. In some embodiments, the DNA-modifying domain includes a catalytic domain having at least 85% amino acid sequence identity with SEQ ID NO: 18. In some embodiments, the DNA-modifying domain includes a catalytic domain having at least 90% amino acid sequence identity with SEQ ID NO: 18. In some embodiments, the DNA-modifying domain includes a catalytic domain having at least 91% amino acid sequence identity with SEQ ID NO: 18. In some embodiments, the DNA-modifying domain includes a catalytic domain having at least 92% amino acid sequence identity with SEQ ID NO: 18. In some embodiments, the DNA-modifying domain includes a catalytic domain having at least 93% amino acid sequence identity with SEQ ID NO: 18. In some embodiments, the DNA-modifying domain includes a catalytic domain having at least 94% amino acid sequence identity with SEQ ID NO: 18. In some embodiments, the DNA-modifying domain includes a catalytic domain having at least 95% amino acid sequence identity with SEQ ID NO: 18. In some embodiments, the DNA-modifying domain includes a catalytic domain having at least 96% amino acid sequence identity with SEQ ID NO: 18. In some embodiments, the DNA-modifying domain includes a catalytic domain having at least 97% amino acid sequence identity with SEQ ID NO: 18. In some embodiments, the DNA-modifying domain includes a catalytic domain having at least 98% amino acid sequence identity with SEQ ID NO: 18. In some embodiments, the DNA-modifying domain includes a catalytic domain having at least 99% amino acid sequence identity with SEQ ID NO: 18.

In some embodiments, the DNA-modifying domain includes a catalytic domain having at least 75% amino acid sequence identity with SEQ ID NO: 19. In some embodiments, the DNA-modifying domain includes a catalytic domain having at least 80% amino acid sequence identity with SEQ ID NO: 19. In some embodiments, the DNA-modifying domain includes a catalytic domain having at least 85% amino acid sequence identity with SEQ ID NO: 19. In some embodiments, the DNA-modifying domain includes a catalytic domain having at least 90% amino acid sequence identity with SEQ ID NO: 19. In some embodiments, the DNA-modifying domain includes a catalytic domain having at least 91% amino acid sequence identity with SEQ ID NO: 19. In some embodiments, the DNA-modifying domain includes a catalytic domain having at least 92% amino acid sequence identity with SEQ ID NO: 19. In some embodiments, the DNA-modifying domain includes a catalytic domain having at least 93% amino acid sequence identity with SEQ ID NO: 19. In some embodiments, the DNA-modifying domain includes a catalytic domain having at least 94% amino acid sequence identity with SEQ ID NO: 19. In some embodiments, the DNA-modifying domain includes a catalytic domain having at least 95% amino acid sequence identity with SEQ ID NO: 19. In some embodiments, the DNA-modifying domain includes a catalytic domain having at least 96% amino acid sequence identity with SEQ ID NO: 19. In some embodiments, the DNA-modifying domain includes a catalytic domain having at least 97% amino acid sequence identity with SEQ ID NO: 19. In some embodiments, the DNA-modifying domain includes a catalytic domain having at least 98% amino acid sequence identity with SEQ ID NO: 19. In some embodiments, the DNA-modifying domain includes a catalytic domain having at least 99% amino acid sequence identity with SEQ ID NO: 19.

In some embodiments, the DNA-modifying domain includes a catalytic domain having at least 75% amino acid sequence identity with SEQ ID NO: 20. In some embodiments, the DNA-modifying domain includes a catalytic domain having at least 80% amino acid sequence identity with SEQ ID NO: 20. In some embodiments, the DNA-modifying domain includes a catalytic domain having at least 85% amino acid sequence identity with SEQ ID NO: 20. In some embodiments, the DNA-modifying domain includes a catalytic domain having at least 90% amino acid sequence identity with SEQ ID NO: 20. In some embodiments, the DNA-modifying domain includes a catalytic domain having at least 91% amino acid sequence identity with SEQ ID NO: 20. In some embodiments, the DNA-modifying domain includes a catalytic domain having at least 92% amino acid sequence identity with SEQ ID NO: 20. In some embodiments, the DNA-modifying domain includes a catalytic domain having at least 93% amino acid sequence identity with SEQ ID NO: 20. In some embodiments, the DNA-modifying domain includes a catalytic domain having at least 94% amino acid sequence identity with SEQ ID NO: 20. In some embodiments, the DNA-modifying domain includes a catalytic domain having at least 95% amino acid sequence identity with SEQ ID NO: 20. In some embodiments, the DNA-modifying domain includes a catalytic domain having at least 96% amino acid sequence identity with SEQ ID NO: 20. In some embodiments, the DNA-modifying domain includes a catalytic domain having at least 97% amino acid sequence identity with SEQ ID NO: 20. In some embodiments, the DNA-modifying domain includes a catalytic domain having at least 98% amino acid sequence identity with SEQ ID NO: 20. In some embodiments, the DNA-modifying domain includes a catalytic domain having at least 99% amino acid sequence identity with SEQ ID NO: 20.

In some embodiments, the DNA-modifying domain includes a catalytic domain having at least 75% amino acid sequence identity with SEQ ID NO: 21. In some embodiments, the DNA-modifying domain includes a catalytic domain having at least 80% amino acid sequence identity with SEQ ID NO: 21. In some embodiments, the DNA-modifying domain includes a catalytic domain having at least 85% amino acid sequence identity with SEQ ID NO: 21. In some embodiments, the DNA-modifying domain includes a catalytic domain having at least 90% amino acid sequence identity with SEQ ID NO: 21. In some embodiments, the DNA-modifying domain includes a catalytic domain having at least 91% amino acid sequence identity with SEQ ID NO: 21. In some embodiments, the DNA-modifying domain includes a catalytic domain having at least 92% amino acid sequence identity with SEQ ID NO: 21. In some embodiments, the DNA-modifying domain includes a catalytic domain having at least 93% amino acid sequence identity with SEQ ID NO: 21. In some embodiments, the DNA-modifying domain includes a catalytic domain having at least 94% amino acid sequence identity with SEQ ID NO: 21. In some embodiments, the DNA-modifying domain includes a catalytic domain having at least 95% amino acid sequence identity with SEQ ID NO: 21. In some embodiments, the DNA-modifying domain includes a catalytic domain having at least 96% amino acid sequence identity with SEQ ID NO: 21. In some embodiments, the DNA-modifying domain includes a catalytic domain having at least 97% amino acid sequence identity with SEQ ID NO: 21. In some embodiments, the DNA-modifying domain includes a catalytic domain having at least 98% amino acid sequence identity with SEQ ID NO: 21. In some embodiments, the DNA-modifying domain includes a catalytic domain having at least 99% amino acid sequence identity with SEQ ID NO: 21.

In some embodiments, the catalytic domain of the DNA-modifying domain that can be used in the gap editor complexes of the present disclosure includes, but is not limited to, any sequence having at least 70% amino acid identity with any of SEQ ID NOs: 22-24. In some embodiments, the DNA-modifying domain includes a catalytic domain having at least 75% amino acid sequence identity with SEQ ID NO: 22. In some embodiments, the DNA-modifying domain includes a catalytic domain having at least 80% amino acid sequence identity with SEQ ID NO: 22. In some embodiments, the DNA-modifying domain includes a catalytic domain having at least 85% amino acid sequence identity with SEQ ID NO: 22. In some embodiments, the DNA-modifying domain includes a catalytic domain having at least 90% amino acid sequence identity with SEQ ID NO: 22. In some embodiments, the DNA-modifying domain includes a catalytic domain having at least 91% amino acid sequence identity with SEQ ID NO: 22. In some embodiments, the DNA-modifying domain includes a catalytic domain having at least 92% amino acid sequence identity with SEQ ID NO: 22. In some embodiments, the DNA-modifying domain includes a catalytic domain having at least 93% amino acid sequence identity with SEQ ID NO: 22. In some embodiments, the DNA-modifying domain includes a catalytic domain having at least 94% amino acid sequence identity with SEQ ID NO: 22. In some embodiments, the DNA-modifying domain includes a catalytic domain having at least 95% amino acid sequence identity with SEQ ID NO: 22. In some embodiments, the DNA-modifying domain includes a catalytic domain having at least 96% amino acid sequence identity with SEQ ID NO: 22. In some embodiments, the DNA-modifying domain includes a catalytic domain having at least 97% amino acid sequence identity with SEQ ID NO: 22. In some embodiments, the DNA-modifying domain includes a catalytic domain having at least 98% amino acid sequence identity with SEQ ID NO: 22. In some embodiments, the DNA-modifying domain includes a catalytic domain having at least 99% amino acid sequence identity with SEQ ID NO: 22.

In some embodiments, the DNA-modifying domain includes a catalytic domain having at least 75% amino acid sequence identity with SEQ ID NO: 23. In some embodiments, the DNA-modifying domain includes a catalytic domain having at least 80% amino acid sequence identity with SEQ ID NO: 23. In some embodiments, the DNA-modifying domain includes a catalytic domain having at least 85% amino acid sequence identity with SEQ ID NO: 23. In some embodiments, the DNA-modifying domain includes a catalytic domain having at least 90% amino acid sequence identity with SEQ ID NO: 23. In some embodiments, the DNA-modifying domain includes a catalytic domain having at least 91% amino acid sequence identity with SEQ ID NO: 23. In some embodiments, the DNA-modifying domain includes a catalytic domain having at least 92% amino acid sequence identity with SEQ ID NO: 23. In some embodiments, the DNA-modifying domain includes a catalytic domain having at least 93% amino acid sequence identity with SEQ ID NO: 23. In some embodiments, the DNA-modifying domain includes a catalytic domain having at least 94% amino acid sequence identity with SEQ ID NO: 23. In some embodiments, the DNA-modifying domain includes a catalytic domain having at least 95% amino acid sequence identity with SEQ ID NO: 23. In some embodiments, the DNA-modifying domain includes a catalytic domain having at least 96% amino acid sequence identity with SEQ ID NO: 23. In some embodiments, the DNA-modifying domain includes a catalytic domain having at least 97% amino acid sequence identity with SEQ ID NO: 23. In some embodiments, the DNA-modifying domain includes a catalytic domain having at least 98% amino acid sequence identity with SEQ ID NO: 23. In some embodiments, the DNA-modifying domain includes a catalytic domain having at least 99% amino acid sequence identity with SEQ ID NO: 23.

In some embodiments, the DNA-modifying domain includes a catalytic domain having at least 75% amino acid sequence identity with SEQ ID NO: 24. In some embodiments, the DNA-modifying domain includes a catalytic domain having at least 80% amino acid sequence identity with SEQ ID NO: 24. In some embodiments, the DNA-modifying domain includes a catalytic domain having at least 85% amino acid sequence identity with SEQ ID NO: 24. In some embodiments, the DNA-modifying domain includes a catalytic domain having at least 90% amino acid sequence identity with SEQ ID NO: 24. In some embodiments, the DNA-modifying domain includes a catalytic domain having at least 91% amino acid sequence identity with SEQ ID NO: 24. In some embodiments, the DNA-modifying domain includes a catalytic domain having at least 92% amino acid sequence identity with SEQ ID NO: 24. In some embodiments, the DNA-modifying domain includes a catalytic domain having at least 93% amino acid sequence identity with SEQ ID NO: 24. In some embodiments, the DNA-modifying domain includes a catalytic domain having at least 94% amino acid sequence identity with SEQ ID NO: 24. In some embodiments, the DNA-modifying domain includes a catalytic domain having at least 95% amino acid sequence identity with SEQ ID NO: 24. In some embodiments, the DNA-modifying domain includes a catalytic domain having at least 96% amino acid sequence identity with SEQ ID NO: 24. In some embodiments, the DNA-modifying domain includes a catalytic domain having at least 97% amino acid sequence identity with SEQ ID NO: 24. In some embodiments, the DNA-modifying domain includes a catalytic domain having at least 98% amino acid sequence identity with SEQ ID NO: 24. In some embodiments, the DNA-modifying domain includes a catalytic domain having at least 99% amino acid sequence identity with SEQ ID NO: 24.

In some embodiments, the DNA-modifying domain used in the gap editor complexes of the present disclosure includes a catalytic domain (or a functional fragment, derivative, or variant thereof) of a Mom (also referred to as methylcarbamoyltransferase, methylcarbamoylase, or acetyltransferase). The catalytic domain can include the portion of a methylcarbamoylase enzyme that is sufficient to carry out methylcarbamoylation of adenine using acetyl CoA as a donor substrate transferred to a target nucleic acid, as described further herein. For example, the catalytic domain of a Mom that can be used as the DNA-modifying domain in the gap editor complexes of the present disclosure includes, but is not limited to, any sequence that has at least 70% amino acid identity with any of SEQ ID NOs: 25-27. In some embodiments, the DNA-modifying domain includes a catalytic domain having at least 75% amino acid sequence identity with SEQ ID NO: 25. In some embodiments, the DNA-modifying domain includes a catalytic domain having at least 80% amino acid sequence identity with SEQ ID NO: 25. In some embodiments, the DNA-modifying domain includes a catalytic domain having at least 85% amino acid sequence identity with SEQ ID NO: 25. In some embodiments, the DNA-modifying domain includes a catalytic domain having at least 90% amino acid sequence identity with SEQ ID NO: 25. In some embodiments, the DNA-modifying domain includes a catalytic domain having at least 91% amino acid sequence identity with SEQ ID NO: 25. In some embodiments, the DNA-modifying domain includes a catalytic domain having at least 92% amino acid sequence identity with SEQ ID NO: 25. In some embodiments, the DNA-modifying domain includes a catalytic domain having at least 93% amino acid sequence identity with SEQ ID NO: 25. In some embodiments, the DNA-modifying domain includes a catalytic domain having at least 94% amino acid sequence identity with SEQ ID NO: 25. In some embodiments, the DNA-modifying domain includes a catalytic domain having at least 95% amino acid sequence identity with SEQ ID NO: 25. In some embodiments, the DNA-modifying domain includes a catalytic domain having at least 96% amino acid sequence identity with SEQ ID NO: 25. In some embodiments, the DNA-modifying domain includes a catalytic domain having at least 97% amino acid sequence identity with SEQ ID NO: 25. In some embodiments, the DNA-modifying domain includes a catalytic domain having at least 98% amino acid sequence identity with SEQ ID NO: 25. In some embodiments, the DNA-modifying domain includes a catalytic domain having at least 99% amino acid sequence identity with SEQ ID NO: 25.

In some embodiments, the DNA-modifying domain includes a catalytic domain having at least 75% amino acid sequence identity with SEQ ID NO: 26. In some embodiments, the DNA-modifying domain includes a catalytic domain having at least 80% amino acid sequence identity with SEQ ID NO: 26. In some embodiments, the DNA-modifying domain includes a catalytic domain having at least 85% amino acid sequence identity with SEQ ID NO: 26. In some embodiments, the DNA-modifying domain includes a catalytic domain having at least 90% amino acid sequence identity with SEQ ID NO: 26. In some embodiments, the DNA-modifying domain includes a catalytic domain having at least 91% amino acid sequence identity with SEQ ID NO: 26. In some embodiments, the DNA-modifying domain includes a catalytic domain having at least 92% amino acid sequence identity with SEQ ID NO: 26. In some embodiments, the DNA-modifying domain includes a catalytic domain having at least 93% amino acid sequence identity with SEQ ID NO: 26. In some embodiments, the DNA-modifying domain includes a catalytic domain having at least 94% amino acid sequence identity with SEQ ID NO: 26. In some embodiments, the DNA-modifying domain includes a catalytic domain having at least 95% amino acid sequence identity with SEQ ID NO: 26. In some embodiments, the DNA-modifying domain includes a catalytic domain having at least 96% amino acid sequence identity with SEQ ID NO: 26. In some embodiments, the DNA-modifying domain includes a catalytic domain having at least 97% amino acid sequence identity with SEQ ID NO: 26. In some embodiments, the DNA-modifying domain includes a catalytic domain having at least 98% amino acid sequence identity with SEQ ID NO: 26. In some embodiments, the DNA-modifying domain includes a catalytic domain having at least 99% amino acid sequence identity with SEQ ID NO: 26.

In some embodiments, the DNA-modifying domain includes a catalytic domain having at least 75% amino acid sequence identity with SEQ ID NO: 27. In some embodiments, the DNA-modifying domain includes a catalytic domain having at least 80% amino acid sequence identity with SEQ ID NO: 27. In some embodiments, the DNA-modifying domain includes a catalytic domain having at least 85% amino acid sequence identity with SEQ ID NO: 27. In some embodiments, the DNA-modifying domain includes a catalytic domain having at least 90% amino acid sequence identity with SEQ ID NO: 27. In some embodiments, the DNA-modifying domain includes a catalytic domain having at least 91% amino acid sequence identity with SEQ ID NO: 27. In some embodiments, the DNA-modifying domain includes a catalytic domain having at least 92% amino acid sequence identity with SEQ ID NO: 27. In some embodiments, the DNA-modifying domain includes a catalytic domain having at least 93% amino acid sequence identity with SEQ ID NO: 27. In some embodiments, the DNA-modifying domain includes a catalytic domain having at least 94% amino acid sequence identity with SEQ ID NO: 27. In some embodiments, the DNA-modifying domain includes a catalytic domain having at least 95% amino acid sequence identity with SEQ ID NO: 27. In some embodiments, the DNA-modifying domain includes a catalytic domain having at least 96% amino acid sequence identity with SEQ ID NO: 27. In some embodiments, the DNA-modifying domain includes a catalytic domain having at least 97% amino acid sequence identity with SEQ ID NO: 27. In some embodiments, the DNA-modifying domain includes a catalytic domain having at least 98% amino acid sequence identity with SEQ ID NO: 27. In some embodiments, the DNA-modifying domain includes a catalytic domain having at least 99% amino acid sequence identity with SEQ ID NO: 27.

***Replication Blocking Moieties**.* One of ordinary skill in the art would recognize, based on the present disclosure, that a replication blocking moiety can include, but is not limited to, glucose, threonyl carbamoyl adenosine, acetate, glyceryl, L-ascorbic acid, uridine, adenosine mono-phosphate, adenosine di-phosphate ribose, methylcarbamoyl, a lipid, an amino acid, agmatine, L-threonylcarbamoyladenylate, L-threonylcarbamoyl, methylthiolate, sulfur, a methyl group, S-adenosyl-L-methione or a subgroup of S-adenosyl-L-methione, and dimethylallyl diphosphate or a subgroup thereof. These and other replication blocking moieties have the general feature of being able to functionalize a nucleotide in a target sequence such that DNA replication is blocked and homology-directed gap repair is induced. This can occur by enzymatic means or by enzyme-independent means.

***Guide RNA**.* Embodiments of the present disclosure also include gap editors and gap editor complexes that can include at least one guide RNA molecule. In accordance with these embodiments, the guide RNA molecule comprises a handle sequence and a targeting sequence. The targeting sequence interacts with a sequence in the target nucleic acid, and the handle sequence facilitates binding of the gap editor or gap editor complex. As would be recognized by one of ordinary skill in the art based on the present disclosure, a single chimeric guide RNA (sgRNA) can mimic the structure of an annealed crRNA/tracrRNA; this type of guide RNA has become more widely used than crRNA/tracrRNA because the gRNA approach provides a simplified system with only two components (e.g., the Cas9 and the sgRNA). Thus, sequence-specific binding to a nucleic acid target can be guided by a natural dual-RNA complex (e.g., comprising a crRNA, a tracrRNA, and Cas9) or a chimeric single-guide RNA (e.g., a sgRNA and Cas9). (see, e.g., Jinek et al. (2012) "A Programmable Dual-RNA-Guided DNA Endonuclease in Adaptive Bacterial Immunity" Science 337:816-821). Multiple gRNAs can be further expressed using CRISPR arrays that naturally encode the crRNA utilized by the nucleases. The gRNAs can also be expressed separately by being operably linked to a promoter and terminator. The gRNAs can also be fused in a single transcript by including intervening RNA cleavages sites, such as ribozymes or sites recognized by RNA-cleaving enzymes such as RNase P, RNase Z, RNase III, or Csy4. The gRNAs or sgRNAs may include RNA templates for reverse transcription into cDNA repair templates. The sgRNAs may include aptamer sequences, for example, RNA-binding protein recognition sites so as to recruit accessory genome editing factors to the gap editor complex or gap editor target site.

As described further herein, genome modifications using the gap editors of the present disclosure can generate specific nucleotide modifications ranging from a single nucleotide change to large insertions or deletions. In some embodiments, the gap editor complexes of the present disclosure can be used to add, exchange, and/or remove large sequences of DNA through the use of more than one guide RNA sequence to target distinct sites in the genome. For example, large genomic deletions can be generated by removing the sequence between two gRNA target sites and/or inserting an exogenous DNA sequence (e.g., by virtue of the endogenous repair/recombination mechanisms in a cell or organism). In some embodiments, multiple gRNAs can be used to target multiple sites in a genome to generate any number of desired modifications in a genome (e.g., multiplexing).

In some embodiments, guide RNA molecules are not required in the gap editor complexes of the present disclosure. For example, certain embodiments of the compositions and methods described herein do not require guide RNAs to effectuate efficient genome editing and modification. As described above, these gap editor complexes include, but are not limited to, meganucleases, zinc-fingers (ZFs), and transcription activator-like effectors (TALEs).

***Donor Template.*** In some embodiments, the presence of a donor nucleic acid template facilitates homology-directed gap recombination and/or repair, which includes the donor nucleic acid template or a fragment thereof being recombined into the double-stranded target DNA molecule. In some embodiments, the donor DNA template can serve as a replication template, resulting in the sequence encoded by the exogenous DNA or RNA being copied into the genome, but the exogenous DNA or RNA polynucleotide molecule itself is not directly transferred into the genome. The donor nucleic acid template can be single-stranded or double-stranded. In some embodiments, the donor template is a cDNA that has reversed transcribed from an endogenous, expressed, synthetic, or delivered RNA. The donor nucleic acid may be delivered into a cell as plasmid or linear DNA. A donor nucleic acid may also be generated in vivo from a template ribonucleic acid by a reverse transcriptase. In other embodiments, the donor nucleic acid may itself be a ribonucleic acid. The donor nucleic acid can also contain chemical modifications. The donor nucleic acid may include chemical modifications or sequences specifically recruited to the gap editor complex, or gap editor target site.

In some embodiments, the donor nucleic acid template comprises a polynucleotide from an endogenous homologous sequence corresponding to the DNA target sequence. In some embodiments, the donor nucleic acid template comprises a polynucleotide from an endogenous allele (e.g., to facilitate loss of heterozygosity). In some embodiments, the donor nucleic acid template comprise an exogenous single-stranded DNA (ssDNA) molecule or double-stranded DNA (dsDNA) molecule. In some embodiments, the presence of the donor nucleic acid template facilitates homology-directed gap repair and/or recombination, wherein the donor nucleic acid template or a fragment thereof is recombined into the genome of the DNA target sequence. In accordance with these embodiments, the gap editors of the present disclosure can be particularly advantageous for inserting large donor DNA sequences, replacing large segments of DNA, and/or removing large DNA sequences in a genome. In some embodiments, the gap editor complexes of the present disclosure can be used to add, exchange, and/or remove large sequences of DNA through the use of more than one guide RNA sequence to target distinct sites in the genome. For example, large genomic deletions can be generated by removing the sequence between two gRNA target sites and/or inserting an exogenous DNA sequence (e.g., by virtue of the endogenous repair/recombination mechanisms in a cell or organism). In some embodiments, multiple gRNAs can be used to target multiple sites in a genome to generate any number of desired modifications in a genome (e.g., multiplexing).

***Accessory Factors.*** In some embodiments, the compositions and systems of the present disclosure further comprise a one gap editor accessory factor. In some embodiments, the composition further comprises at least one gap editor accessory factor. In some embodiments, the at least one gap editor accessory factor comprises a protein that augments at least one step in a genome modification process. In some embodiments, the at least one gap editor accessory factor is recruited to the gap editor complex via interaction with the DNA-modifying domain, the DNA-recognition domain, and/or the at least one guide RNA. In some embodiments, the recruitment of the at least one gap editor accessory factor to the gap editor complex comprises a peptide tag, a peptide linker, an RNA tag, and any combinations thereof. In some embodiments, the at least one gap editor accessory factor comprises Rap, DarG, Orf, ExoI, Exonuclease III, PrimPol, RecJ, RecQ1, Rad51, Rad52, CtIP, Rad18, and any combinations thereof. In some embodiments, and as described further herein, the present disclosure can include gap editor complexes in which the DNA-modifying domain comprises DarT. In accordance with these embodiments, DarG, TARG1, or another glycohydolase domain can be included as a gap editor accessory factor by modulating off-target editing (e.g., attenuating DarT activity) or removing the added ADPr after HDGR occurs.

As would be recognized by one of ordinary skill in the art based on the present disclosure, methods for delivering gap editors and gap editor complexes into a cell include any currently known methods and systems for delivering polynucleotides and/or polypeptides/proteins. For example, gap editors and gap editor complexes can be delivered using plasmid DNA, ssDNA, RNA, or other means for delivering polynucleotide molecules, including but not limited to, lipid-based delivery systems (e.g., using cationic lipids), conjugation from a donor cell, viral/bacteriophage-based delivery systems, and chemical-based systems (e.g., calcium phosphate precipitation, DEAE-dextran, polybrene). In some embodiments, the delivery system can include mechanical and/or electrical devices and methods for delivering the gap editors and gap editor complexes of the present disclosure as polynucleotides and/or as polypeptides/proteins (or any combinations thereof). In some embodiments, gap editors and gap editor complexes are delivered using a gene gun (e.g., bombardment and Agrobacterium transformation as used for plant cells), and electroporation-based methods, as well as any other physical methods (e.g., mechanical, electrical, thermal, optical, chemical stimulation, and the like) that use membrane disruption as a means for delivering polynucleotides and polypeptides/proteins (see, e.g., Sun et al., Recent advances in micro/nanoscale intracellular delivery, Nanotechnology and Precision Engineering 3, 18 (2020)).

### 3. Kits, Systems, and Methods

Embodiments of the present disclosure also include kits and systems for targeted modification of a nucleic acid. In accordance with these embodiments, the kit includes a gap editor complex comprising a DNA-recognition domain and a DNA-modifying domain. In some embodiments, the kit also includes at least one guide RNA molecule. In some embodiments, the DNA-recognition domain binds a DNA target sequence in the genome, and the DNA-modifying domain induces formation of a replication blocking moiety on at least one nucleotide in the genome. As would be recognized by one of ordinary skill based on the present disclosure, the kits and systems can also include one or more of the other components of the gene modification compositions described herein (e.g., gap editor accessory factors). In some embodiments of the kit, the composition further comprises a donor nucleic acid template. In some embodiments of the kit, the presence of the donor nucleic acid template facilitates homology-directed gap repair and/or recombination.

In some embodiments of the kit, the DNA-recognition domain comprises at least one Cas protein or fragment thereof lacking deoxyribonuclease activity. In some embodiments of the kit, the DNA-recognition domain comprises at least one Cas protein or fragment thereof having nickase activity. In some embodiments, the Cas protein or Cas protein complex comprises a Type I Cascade, a Type II Cas9, a Type IV effector module, a Type V Cas12, a Cas9-related IscB, a Cas9-related TnpB, and combinations thereof.

In some embodiments of the kit, the DNA-recognition domain and the DNA-modifying domain are functionally coupled. In some embodiments of the kit, the DNA-recognition domain induces a single-stranded break in the DNA target strand, and the DNA-modifying domain adds the replication blocking moiety to at least one nucleotide in the DNA strand complementary to the DNA target sequence. In some embodiments of the kit, the DNA-modifying domain catalyzes addition of ADP ribose to a thymine or guanine nucleotide. In some embodiments, the DNA-modifying domain comprises a DarT enzyme or a functional fragment, derivative, or variant thereof. In some embodiments of the kit, the DarT enzyme has been engineered to have reduced DNA binding, increased specificity to single-stranded DNA, and/or decreased enzymatic activity.

In some embodiments of the kit, the DNA-modifying domain catalyzes addition of a replication blocking moiety selected from the group consisting of: glucose, threonyl carbamoyl adenosine, acetate, glyceryl, L-ascorbic acid, uridine, adenosine mono-phosphate, a lipid, an amino acid, agmatine, L-threonylcarbamoyladenylate, L-threonylcarbamoyl, methylthiolate, sulfur, a methyl group, S-adenosyl-L-methione or a subgroup of S-adenosyl-L-methione, and dimethylallyl diphosphate or a subgroup thereof. In some embodiments of the kit, the DNA-modifying enzyme domain comprises an enzyme or functional fragment, derivative, or variant thereof, selected from the group consisting of: Pierisin, Scabin, Cell cycle and apoptosis regulator 1 (CARP-1), SCO5461 protein (ScARP), adenine modification enzyme, acetyltransferase, amino acid transferase, nucleotidyl transferase, uridyltransferase, acyltransferase, ADP-ribosyltransferase, methylthiotransferase, N-acetyl transferase 10, tRNA(Met) cytidine acetyltransferase (TmcA), tRNA cytidine acetyltransferase, GCN5-related N-acetyltransferase, lysidine synthase, m⁷G methyltransferase, N6 carbamoylmethyltransferase (Mom), N6-adenosine threonylcarbamoyltransferase, threonyl carbomyl transferase or threonyl carbomyl transferase complex, TsaB-TsaE-TsaD (TsaBDE) complex, tRNA N6-adenosine threonylcarbamoyltransferase (Qri7, Tcs4), methyltransferase, ATrm5a, tRNA:m¹G/imG2 methyltransferase, tRNA (adenosine(37)-N6)-dimethylallyltransferase, tRNA dimethylallyltransferase (MiaA), and isopentenyltransferase.

In some embodiments of the kit, the at least one guide RNA comprises gRNA, sgRNA, crRNA, or any combinations thereof. In some embodiments of the kit, the at least one guide RNA comprises a handle sequence and a targeting sequence. In some embodiments of the kit, the targeting sequence in the at least one guide RNA is complementary to the DNA target sequence. In some embodiments, the gap editor complexes of the present disclosure can be used to add, exchange, and/or remove large sequences of DNA through the use of more than one guide RNA sequence to target distinct sites in the genome. For example, large genomic deletions can be generated by removing the sequence between two gRNA target sites and/or inserting an exogenous DNA sequence (e.g., by virtue of the endogenous repair/recombination mechanisms in a cell or organism). In some embodiments, multiple gRNAs can be used to target multiple sites in a genome to generate any number of desired modifications in a genome (e.g., multiplexing).

Embodiments of the present disclosure also include methods for targeted modification of a nucleic acid. In accordance with these embodiments, the methods include introducing any of the components of the genome modification compositions described herein, and assessing the cell for presence of a desired genetic alteration using techniques known in the art. In some embodiments of the method, the components include gap editors and gap editor complexes comprising a DNA-recognition domain and a DNA-modifying domain, at least one guide RNA molecule, and a donor nucleic acid template. In some embodiments, one or more gap editor accessory factors can also be included. One or more of these factors can be introduced into a cell as a polypeptide(s), mRNA(s), and/or DNA expression construct(s), or any combination thereof, by means known in the art.

In some embodiments of the method, at least one of these components are introduced into the cell as part of a gene drive system. In a gene drive system, all or some of genome modification components such as the DNA-recognition domain, DNA-modifying domain, gRNA, and accessory factors are encoded within the donor nucleic acid sequence present in one copy of a chromosome. The gRNA directs the DNA-modifying domain to the sister chromosome in the region where the donor nucleic acid sequence would reside. Upon targeting by the gap editor proteins or complexes, the donor nucleic acid (which also encodes the gap editor system) is copied over to a new chromosome. Thus, the gap editor system becomes self-propagating, efficiently forming homozygously edited organisms. Example organisms in which gene drives can be implemented include fungi, flatworms, mosquitos, and mice.

In some embodiments, the compositions, systems, and methods of the present disclosure include one or more components that enhance or improve one or more aspects of gene modification. In some embodiments, improving or enhancing one or more aspects of genome modification includes the use of a gap editor accessory factor(s), as described above. In some embodiments, methods that enhance or improve one or more aspects of genome modification include reducing or attenuating nuclease activity in a cell in which genome modification is desired. Reducing nuclease activity in a cell can lead to enhanced or improved modification frequency and/or efficiency. In some embodiments, reducing nuclease activity in a cell includes reducing activity of an endogenous AP endonuclease (e.g., encoded by *xthA*) by any means known in the art. In some embodiments, nuclease activity in a cell can be reduced via genetic means and/or by pharmacological means (e.g., treatment with endonuclease inhibitors including but not limited to AJAY-4, CRT0044876, aurintricarboxylic acid, 6-hydroxy-DL-DOPA, Reactive Blue 2, myricetin, mitoxantrone, methyl-3,4-dephostatin, thiolactomycin, and (2E)-3-[5-(2,3-dimethoxy-6-methyl-1,4-benzoquinoyl)]-2-nonyl-2-propenoic acid (E3330)).

Embodiments of the compositions, systems, and methods provided herein can be used to edit the genome of a cell. The cell can be a prokaryotic cell, a eukaryotic cell, or a plant cell. In some embodiments, the cell is a mammalian cell. The present disclosure also provides an isolated cell comprising any of the components or systems described herein. Exemplary cells can include those that can be easily and reliably grown, have reasonably fast growth rates, have well characterized expression systems, and can be transformed or transfected easily and efficiently. Examples of suitable prokaryotic cells include, but are not limited to, cells from the genera *Bacillus* (such as *Bacillus subtilis* and *Bacillus brevis*), Clostridia (such as *Clostridium difficile* or *Clostridium autoethanogenum*)*, Escherichia* (such as *E. coli*), *Lactobacilli, Klebsiella,* Myxobacteria, *Pseudomonas, Streptomyces, Salmonella, Vibrio* (such as *Vibrio cholerae* or *Vibrio nutrifaciens*) and *Envinia.* Suitable eukaryotic cells are known in the art and include, for example, yeast cells, insect cells, and mammalian cells. Examples of suitable yeast cells include those from the genera *Kluyveromyces, Pichia, Rhino-sporidium, Saccharomyces,* and *Schizosaccharomyces.* Exemplary insect cells include Sf-9 and HIS (Invitrogen, Carlsbad, Calif.) and are described in, for example, Kitts et al., Biotechniques, 14: 810-817 (1993); Lucklow, Curr. Opin. Biotechnol., 4: 564-572 (1993); and Lucklow et al., J. Virol., 67: 4566-4579 (1993).

In some embodiments, the compositions and methods of the present disclosure can be employed to induce DNA modification, and/or transcriptional modulation in mitotic or post-mitotic cells in vivo and/or ex vivo and/or in vitro (e.g., to produce genetically modified cells that can be reintroduced into an individual). Because the gap editors of the present disclosure include site-specific DNA-targeting, a mitotic and/or post-mitotic cell-of-interest can include a cell from any organism (e.g. a bacterial cell, an archaeal cell, a cell of a single-cell eukaryotic organism, a plant cell, an algal cell, e.g., *Botryococcus braunii, Chlamydomonas reinhardtii, Nannochloropsis gaditana, Chlorella pyrenoidosa, Sargassum patens* C. Agardh, and the like, a fungal cell (e.g., a yeast cell), an animal cell, a cell from an invertebrate animal (e.g. fruit fly, cnidarian, echinoderm, nematode, etc.), a cell from a vertebrate animal (e.g., fish, amphibian, reptile, bird, mammal), a cell from a mammal, a cell from a rodent, a cell from a human, etc.). Any type of cell may be of interest (e.g. a stem cell, e.g. an embryonic stem (ES) cell, an induced pluripotent stem (iPS) cell, a non-human germ cell; a somatic cell, e.g. a fibroblast, a hematopoietic cell, a neuron, a muscle cell, a bone cell, a hepatocyte, a pancreatic cell) Cells may be from established cell lines or they may be primary cells, where "primary cells", "primary cell lines", and "primary cultures" are used interchangeably herein to refer to cells and cells cultures that have been derived from a subject and allowed to grow in vitro for a limited number of passages of the culture. Target cells can include any unicellular organisms or any cells grown in culture.

In some embodiments, the cell can also be a cell that is used for therapeutic purposes. The cell can be a mammalian cell, and in some embodiments, the cell is a human cell. A number of suitable mammalian and human cells are known in the art, and many are available from the American Type Culture Collection (ATCC, Manassas, Va.). Examples of suitable mammalian cells include, but are not limited to, Chinese hamster ovary cells (CHO) (ATCC No. CCL61), CHO DHFR-cells (Urlaub et al., Proc. Natl. Acad. Sci. USA, 97: 4216-4220 (1980)), human embryonic kidney (HEK) 293 or 293T cells (ATCC No. CRL1573), and 3T3 cells (ATCC No. CCL92). Other suitable mammalian cell lines are the monkey COS-1 (ATCC No. CRL1650) and COS-7 cell lines (ATCC No. CRL1651), as well as the CV-1 cell line (ATCC No. CCL70). Further exemplary mammalian cells include primate, rodent, and human cell lines, including transformed cell lines. Normal diploid cells, cell strains derived from *in vitro* culture of primary tissue, as well as primary explants, are also suitable. Other suitable mammalian cell lines include, but are not limited to, mouse neuroblastoma N2A cells, HeLa, HEK, A549, HepG2, mouse L-929 cells, and BHK or HaK hamster cell lines. Methods for selecting suitable cells and methods for transformation, culture, amplification, screening, and purification of cells are known in the art. Examples of suitable plant cell lines are derived from plants such as *Arabidopsis* (such as the Landsberg erecta cell line), sugarcane, tomato, pea, rice, wheat, tobacco (such as the BY-2 cell line).

In accordance with the methods described above embodiments, the compositions and systems of the present disclosure can be used to edit a genome of a cell in a manner that reduces the degree of indel formation, chromosomal rearrangements, or DNA duplications. In some embodiments, the compositions, systems, and methods described herein reduce cell toxicity as compared to currently available methods, at least in part due to the lack double-stranded breaks in the target nucleic acid.

### 4. Materials and Methods

Measurement of gap editing in *E. coli* by a colorimetric assay was performed by co-transforming the DNA modifying domain fused to a DNA binding domain such as Cas9 (e.g. DarT-ScdCas9) and an sgRNA and nucleic acid donor into *E. coli* by electroporation and plated on LB agar plus the appropriate antibiotic(s). The resulting colonies were picked and inoculated into 750 mL of liquid LB media in a deep well plate shaking at 900 rpm and 37°C for 12 to 16 hours overnight. Gap editor expression was induced by diluting overnight culture 1:500 into 750 mL of liquid LB media with antibiotics, 1 mM IPTG and 33 mM arabinose, shaking at 900 rpm for 8 hours. After 8 hours, samples were removed for spot plating on LB agar with antibiotics, IPTG, and X-gal. The next day, white and blue colonies were counted to determine frequency of *lacZ* recombination and repair. Repair was confirmed by sanger sequencing.

Measurement of gap editing in *E. coli* by antibiotic resistance assays was performed by co-transforming a DNA modifying domain fused to a DNA binding domain such as Cas9 or Cas12a, and an sgRNA with nucleic acid donor by electroporation. The transformation mixture was plated on LB agar plus the appropriate antibiotics. The resulting colonies were picked and inoculated into 750 mL of liquid LB media in a deep well plate shaking at 900 rpm and 30°C for 12 to 16 hours overnight. Gap editor cultures were first back-diluted 1:100 into liquid LB with antibiotics shaking at 37°C for 1 hour. Gap editor expression was then induced by further diluting this culture 1:100 into 750 mL of liquid LB media with antibiotics and 33 mM arabinose, shaking at 900 rpm for 5 hours. After 5 hours of induction, samples were removed for spot plating on two separate LB agar plates. One plate contained antibiotics to selected only for the gap editor, sgRNA, and repair template (typically chloramphenicol and ampicillin) and the other plate also included either rifampicin or kanamycin to select for edited cells. The next day colonies were counted. Genome editing efficiency was tabulated as being the number of colonies on the plates with rifampicin or kanamycin divided by the number of colonies on plates without rifampicin or kanamycin.

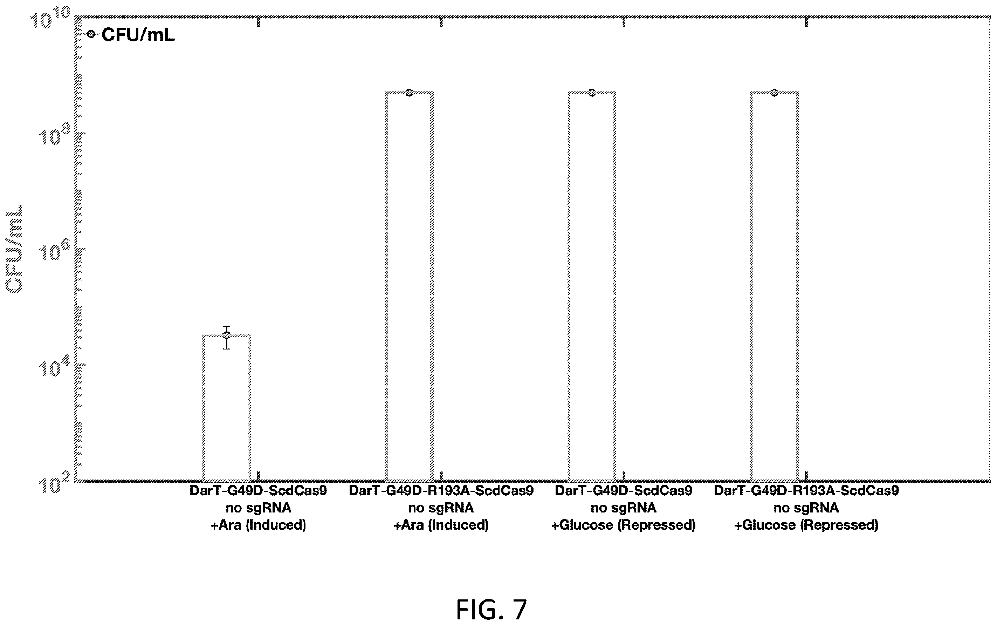

The measurement of gap editor toxicity in FIG. 7 was performed by co-transforming DarT-ScdCas9 gap editors into an *E. coli* strain lacking recA, a key factor in homologous recombination. These bacterial lack the capability for lesion bypass by homologous recombination, and are thus highly sensitive to replication blocking lesions on the DNA. Thus, DNA modification domains are expected to be especially toxic in these strains, unless their latent DNA binding activity is contained. In this fashion, we can more easily assess gap editor complexes for undesirable off-target DNA modification. After transforming and plating, single colonies were selected and inoculated into 750 mL of LB Chloramphenicol in a deep well plate shaking at 37°C overnight. The next day, cultures were back-diluted 1:500 into LB Chloramphenicol with glucose to maintain gap editor repression, or arabinose to induce expression of the gap editor. Cultures were incubated shaking at 900 rpm in a deep well plate at 37°C for 5 hours. Cultures were then spot plated on LB Chloramphenicol. The next day, colonies were counted to assess the final cell density, and therefore the rate of off-target DNA modification.

Measurement of ssDNA-templated gap editing in *E. coli* by rifampicin resistance was performed by first co-transforming the strand annealing beta recombinase plasmid and a DNA modifying domain fused to a DNA binding domain such as Cas9. The resulting clones were inoculated into LB, antibiotics, and anhydrotetracycline for induction of beta recombinase expression. These cultures were prepared for electroporation and transformed with the sgRNA plasmid, and cultured for 3 hours in a rich media at 37°C and shaking at 250 RPM prior to spot plating on two separate LB agar plates. One plate contained antibiotics to selected only for the gap editor, sgRNA, and recombinase. The other plate additionally included rifampicin to select for edited cells. The next day colonies were counted. Genome editing efficiency was tabulated as being the number of colonies on the plates with rifampicin divided by the number of colonies on plates without rifampicin.

**Table 2: Strain information corresponding to gap editors and gap editor complexes used in the present disclosure.**

| **DNA or Strain Name** | **Composition** | **Function** | **Appears in:** |
|---|---|---|---|
| SPC1879 Or dTd-ScdC9 | darT G49D-ScdCas9 pBAD araC CmR p15a | Site specific replication block onto thymine, induction of HDGR | FIG. 1 |
| SPC1881 Or GE2 | darT G49D_K56A-ScdCas9 pBAD araC CmR p15a | Site specific replication block onto thymine, induction of HDGR, with reduced DarT DNA binding | FIGS. 1-3 |
| SPC1883 or dTd-ScnC9 | darT G49D-ScnCas9 pBAD araC CmR p15a | Site specific replication block onto thymine, induction of HDGR | FIG. 9 |
| SPC1884 Or GE2n | darT G49D_K56A-ScnCas9 pBAD araC CmR p15a | Site specific replication block onto thymine, induction of HDGR, with reduced DarT DNA binding, with target strand nicking | FIG. 16 |
| SPC1466 | lacZ_sg705-araF_pCON ΔaraBAD | *E. coli* with defective lacZ gene | FIGS. 1-3 |
| SPC1911 | ScdCas9 pBAD araC CmR p15a | DNA binding only | FIG. 1 |
| SPC1912 | ScnCas9 pBAD araC CmR p15a | Nicking of target strand | FIG. 2 |
| SPC1901 | darT_G49D_K56 A-ScdCas9-darG pBAD araC CmR p15a | Site specific replication block onto thymine, induction of HDGR, with reduced DarT DNA binding, with full length DarT inhibitor, DarG | FIG. 3 |
| SPC1902 | darT_G49D_K56 A-ScdCas9-darG_Cterminal pBAD araC CmR p15a | Site specific replication block onto thymine, induction of HDGR, with reduced DarT DNA binding with C terminal domain of DarT inhibitor, DarG | FIG. 3 |
| SPC1903 | darT_G49D_K56 A-ScdCas9-darG_Nterminal pBAD araC CmR p15a | Site specific replication block onto thymine, induction of HDGR, with reduced DarT DNA binding, with N terminal domain of DarT inhibitor, DarG | FIG. 3 |
| SPC1904 | darT_G49D_K56 A-ScnCas9-darG pBAD araC CmR p15a | Site specific replication block onto thymine, induction of HDGR, with reduced DarT DNA binding, with target strand nicking, with full length DarT inhibitor, DarG | FIG. 3 |
| SPC1905 | darT_G49D_K56 A-ScnCas9-darG_Cterminal pBAD araC CmR p15a | Site specific replication block onto thymine, induction of HDGR, with reduced DarT DNA binding, with target strand nicking, with C terminal domain of DarT inhibitor, DarG | FIG. 3 |
| SPC1906 | darT_G49D_K56 A-ScnCas9-darG_Nterminal pBAD araC CmR p15a | Site specific replication block onto thymine, induction of HDGR, with reduced DarT DNA binding, with target strand nicking, with N terminal domain of DarT inhibitor, DarG | FIG. 3 |
| SPC2503 | Scabin-K130A-ScdCas9) | Site specific replication block (adenosine di-phosphate ribose) transfer onto guanine, induction of HDGR, nuclease-inactive Cas9 | FIG. 4 |
| SPC2548 | Scabin-K130A-E160A-ScdCas9 | Catalytically inactive scabin fused to nuclease inactive Cas9 to serve as a negative control | FIG. 4 |
| SPC2488 | Non-targeting sgRNA SS2 KanR HRT L2/RE AmpR ColE1 | Negative control, non-targeting guide RNA. Includes repair template for kanamycin resistance gene repair, but lacks a guide RNA directing the gap editor to the correct genomic location. | FIGS. 4, 5, 6, 8, 9 |
| SPC2480 | Scabin stop sgRNA SS2 KanR HRT L2/RE AmpR ColE1 | Guide RNA directing the gap editor complex to the target site for scabin gap editor-directed kanamycin gene repair. Includes repair template for kanamycin gene restoration. For use with strain SPC2496. | FIG. 4 |
| SPC2496 | KanR_mut Scabin stop lead_first::SS2 araF_pCON ΔaraBAD ΔlacZ_519 | A mutated kanamycin resistance gene inserted into the *E. coli* genome with a site for targeting by a scabin gap editor. Targeting this site will trigger HDGR and confer resistance to kanamycin. | FIG. 4 |
| SPC2642 | MOM-D149A-ScdCas9 | Site specific replication block (carbamoyl group) transfer onto adenine, induction of HDGR, nuclease-inactive Cas9 | FIG. 5 |
| SPC2490 | Mom sgRNA SS2 KanR HRT L2/RE AmpR ColE1 | Guide RNA directing the gap editor complex to the target site for mom gap editor-directed kanamycin gene repair. Includes repair template for kanamycin gene restoration. For use with strain SPC2514. | FIG. 5 |
| SPC2514 | KanR_mut mom stop lead_first::SS2 araF_pCON ΔaraBAD ΔlacZ_519 | A mutated kanamycin resistance gene inserted into the E. coli genome with a site for targeting by a mom gap editor. Targeting this site will trigger HDGR and confer resistance to kanamycin. | FIG. 5 |
| SPC2495 | KanR_mut DarT stop lead_first::SS2 araF_pCON ΔaraBAD ΔlacZ_519 | A mutated kanamycin resistance gene inserted into the E. coli genome with a site for targeting by a DarT gap editor. Targeting this site will trigger HDGR and confer resistance to kanamycin. | FIGS. 6, 8, 9 |
| SPC1134 | MG1655 ΔrecA | An *E. coli* strain defective for the homologous recombination factor recA. Sensitizes *E. coli* to off-target DNA modifications. Allows for easier measurement of off-target DNA modifications. | FIG. 7 |
| SPC2716 | DarT-G49D-R193A-ScdCas9 | Site specific replication block onto thymine, induction of HDGR, with reduced DarT DNA binding, nuclease-inactive Cas9. | FIG. 7, 8, 9 |
| SPC2690 | DarT-G49D-M86L-R92A-R193A-ScdCas9 | Site specific replication block onto thymine, induction of HDGR, with further reduced DarT DNA binding, nuclease-inactive Cas9. | FIG. 8 |
| SPC2189 | DarT_G49D_R19 3A-ScnCas9 pBAD araC CmR p15a | Site specific replication block onto thymine, induction of HDGR, with reduced DarT DNA binding, nicking Cas9. | FIG. 9 |
| SPC2530 | DarT_G49D_R19 3A-ScnCas9 huOpt pGAL Leu CEN AmpR | Site specific replication block onto thymine, induction of HDGR, with reduced DarT DNA binding, nicking Cas9. Yeast expression. | FIG. 10 |
| SPC2525 | ScnCas9 D10A huOpt pGAL Leu CEN AmpR | Cas9 nickase, yeast expression. | FIG. 10 |
| SPC2435 | FCY1 KO HRT sgRNA 5 pSNR52 sgRNA TRP1 2micron LS/R1 AmpR | Guide RNA directing the DarT gap editor complex to a genomic site in the *fcy1* gene. Includes a repair template encoding stop codons to edit and disrupt the translation of *fcy1,* resulting in 5-FC resistance and colony growth. | FIG. 10 |
| SPC2467 | FCY1 KO HRT Non-Targeting sgRNA TRP1 2micron LS/R1 | Negative control, non-targeting guide RNA. Includes a repair template for disruption of the *fcy1* gene, but lacks the guide RNA directing the gap editor to the correct genomic site. | FIG. 10 |
| SPC2629 | FCY1 US1 KO HRT sgRNA 5 pSNR52 sgRNA TRP1 2micron LS/R1 | Guide RNA directing the DarT gap editor complex to a genomic site in the fcy1 gene. Includes a repair template encoding stop codons to edit and disrupt the translation of fcy1, resulting in 5-FC resistance and colony growth. | FIG. 10 |
| SPC2631 | FCY1 DS1 KO HRT sgRNA 5 pSNR52 sgRNA TRP1 2micron LS/R1 | Guide RNA directing the DarT gap editor complex to a genomic site in the fcy1 gene. Includes a repair template encoding stop codons to edit and disrupt the translation of fcy1, resulting in 5-FC resistance and colony growth. | FIGS. 10, 11 |
| SPC2635 | FCY1 US2 KO HRT Non-Targeting sgRNA TRP1 2micron LS/R1 | Guide RNA directing the DarT gap editor complex to a genomic site in the fcy1 gene. Includes a repair template encoding stop codons to edit and disrupt the translation of fcy1, resulting in 5-FC resistance and colony growth. | FIG. 10 |
| SPC2637 | FCY1 DS2 KO HRT Non-Targeting sgRNA TRP1 2micron LS/R1 | Guide RNA directing the DarT gap editor complex to a genomic site in the fcy1 gene. Includes a repair template encoding stop codons to edit and disrupt the translation of fcy1, resulting in 5-FC resistance and colony growth. | FIG. 10 |
| SPC2722 | DarT_G49D_R19 3A_M86L_R92A-ScnCas9 huOpt pGAL Leu CEN AmpR | Site specific replication block onto thymine, induction of HDGR, with further reduced DarT DNA binding, nicking Cas9. Yeast expression. | FIG. 11 |
| SPC2777 | DarT_G49D_R19 3A-dLbCas12a pBAD CmR p15a | Site specific replication block onto thymine, induction of HDGR, with reduced DarT DNA binding, nuclease-inactive Cas12a fusion. | FIG. 13 |
| SPC2795 | LbCas12a Non-targeting crRNA mut short lacZ HRT AmpR ColE1 | Negative control, non-targeting gRNA with lacZ repair template encoding a stop codon. | FIG. 13 |
| SPC2796 | LbCas12a crRNA 1 mut short lacZ HRT AmpR ColE1 | gRNA directing LbCas12a gap editor complex to lacZ gene and repair template encoding a stop codon as a genome editing template. | FIG. 13 |
| SPC2797 | LbCas12a crRNA 2 mut short lacZ HRT AmpR ColE1 | gRNA directing LbCas12a gap editor complex to lacZ gene and repair template encoding a stop codon as a genome editing template. | FIG. 13 |
| SPC2798 | LbCas12a crRNA 3 mut short lacZ HRT AmpR ColE1 | gRNA directing LbCas12a gap editor complex to lacZ gene and repair template encoding a stop codon as a genome editing template. | FIG. 13 |
| SPC2799 | LbCas12a crRNA 4 mut short lacZ HRT AmpR ColE1 | gRNA directing LbCas12a gap editor complex to lacZ gene and repair template encoding a stop codon as a genome editing template. | FIG. 13 |
| SPC2800 | LbCas12a crRNA 5 mut short lacZ HRT AmpR ColE1 | gRNA directing LbCas12a gap editor complex to lacZ gene and repair template encoding a stop codon as a genome editing template. | FIG. 13 |
| SPC2801 | LbCas12a crRNA 6 mut short lacZ HRT AmpR ColE1 | gRNA directing LbCas12a gap editor complex to lacZ gene and repair template encoding a stop codon as a genome editing template. | FIG. 13 |
| SPC2802 | LbCas12a crRNA 7 mut short lacZ HRT AmpR ColE1 | gRNA directing LbCas12a gap editor complex to lacZ gene and repair template encoding a stop codon as a genome editing template. | FIG. 13 |
| SPC1895 | DarT_G49D-ScnCas9 Ec86 RT pBAD araC CmR p15a | Site specific replication block onto thymine, induction of HDGR, fusion with nicking Cas9. Co-expression of Ec86 reverse transcriptase for use of RNA repair templates. | FIG. 15 |
| SPC2132 | rpoB GE2n retron FWD 1d1 D516 sgRNA AmpR ColE1 | Guide RNA targeting the DarT gap editor complex to the *rpoB* gene at residue D516 for genome editing and rifampicin resistance. Includes the an RNA repair template with flanking sequences for reverse transcription by Ec86 reverse transcriptase. | FIG. 15 |
| SPC2133 | Non-Targeting DarT D516 rpoB retron FWD sgRNA AmpR ColE1 | Negative control for D516 *rpoB* editing with RNA repair template. Includes RNA repair template expression, but lacks a guide RNA targeting the DarT gap editor complex to the *rpoB* gene. | FIG. 16 |
| SPC2095 | rpoB ld1 sgRNA AmpR ColE1 | Guide RNA targeting *rpoB* gene at residue D516 for genome editing and rifampicin resistance | FIG. 16 |
| SPC2026 | lambda beta pTet 4.6k TIR tetR kanR sc101 | Beta recombinase under an anhydrotetracycline inducible promoter. Used for gap editing using ssDNA and RNA templates. | FIGS. 15, 16 |

### 5. Examples

It will be readily apparent to those skilled in the art that other suitable modifications and adaptations of the methods of the present disclosure described herein are readily applicable and appreciable, and may be made using suitable equivalents. Having now described the present disclosure in detail, the same will be more clearly understood by reference to the following examples, which are merely intended only to illustrate some aspects and embodiments of the disclosure.

The present disclosure has multiple aspects, illustrated by the following non-limiting examples.

### Example 1

Experiments were conducted to assess the efficiency and toxicity of the gap editor complexes of the present disclosure. In one set of experiments, the DarT enzyme from *E. coli* EPEC with the attenuating mutation G49D was fused to the N-terminus of the fully or partially catalytically-dead version of ScCas9 (ScdCas9, or ScCas9 D10A also known as ScnCas9) with a long flexible linker. It was hypothesized that if chemical modification would occur, they would be made to the non-target strand exposed by ScdCas9 binding to its DNA target. Previous work indicated that DarT modifies thymine within a sequence motif possibly as wide as TYTN. Accordingly, genome editing in *E. coli* was assessed using these gap editor complexes.

The DarT-ScdCas9 fusion protein (gap editor complex) was targeted to four sites containing an NGG or NAG PAM and a TTTC motif on the non-target strand. The four sites surrounded a premature stop codon in the *lacZ* gene, which was the desired site of genome modification. The targets were chosen such that if a replication blocking lesion was introduced, a DNA gap would form that overlapped the premature stop codon. The four sites included two lagging strand targets and two leading strand targets. A plasmid encoding an arabinose inducible DarT-ScdCas9 was co-transformed with a plasmid containing a 1.5 kb repair template encoding mutations to block ScdCas9 re-targeting while repairing the *lacZ* stop codon. After culturing these colonies overnight, the cells were back-diluted into inducing medium, cultured for 8 hours, and then plated onto selective media with the β-galactosidase (*lacZ* gene product) indicator dye X-gal with the inducer IPTG.

When targeting only one site, the *lacZ* gene was efficiently repaired, as demonstrated by the results of in FIG. 1. However, targeting this site included a 10-fold drop in CFUs compared to the non-targeting condition, and a 50-fold drop in CFUs compared to the ScdCas9 control. This observed cytotoxicity could be due to ScdCas9-independent binding of DarT to ssDNA, which introduced widespread DNA replication blocks. By attenuating DNA binding within DarT, it was hypothesized that DarT could be more dependent on ScdCas9 for DNA binding. Computational prediction tools were used to identify potential DNA binding sites. To improve prediction accuracy, a set of DarT homologs were identified with some sequence divergences and predicted DNA binding sites for all of these homologs. By aligning the proteins and the DNA predictions, some DNA binding site predictions were found to be conserved across these DarT homologs. Based on this, alanine mutations were installed at these predicted sites. In one example, a K56A mutation substantially reduced the cytotoxic effects of DarT-ScdCas9, while maintaining efficient genome modification activity (FIG. 1). This new DarT-ScdCas9 fusion protein was referred to as gap editor 2 (GE2).

### Example 2

Because a single replication block was being introduced into the DNA, it was expected that the dominant repair template would be the sister chromatid and not an ectopic repair template. Previous work has demonstrated that targeting two sites on either side of a DNA sequence-of-interest can boost genome modification, possibly by creating overlapping DNA gaps and interfering with sister chromatid repair. Therefore, it was hypothesized that the combination of DNA nicking and DNA modification/gap formation might similarly prevent sister chromatid repair, leaving the plasmid repair template as the preferred template for repair.

Cas9 nicking can drive low rates of genome editing in prokaryotes and eukaryotes. These nicks form single-ended double-strand breaks (seDSB) when encountered by the replisome. This typically involves replisome dissociation. These single-ended breaks are repaired by homologous recombination, most frequently with the sister chromatid. Importantly, in eukaryotic cells, Cas9 nicking can generate precise edits while minimizing indels presumably caused by non-homologous end-joining (NHEJ) machinery. There is no natural end joining partner at seDSBs, so NHEJ is inhibited at these breaks.

In accordance with the embodiments of the present disclosure, it was hypothesized that an overlapping DNA gap and seDSB could mutually exclude sister chromatid repair (e.g., exert synergistic effects). Where the seDSB end would typically look for homology on the sister chromatid, there would instead be a ssDNA gap. Similarly, where the DNA gap would typically find a homologous DNA template, there would be a seDSB, possibly resected to ssDNA. Therefore, the H848A mutation in ScdCas9 was re-activated, creating the target-strand nickase ScnCas9.

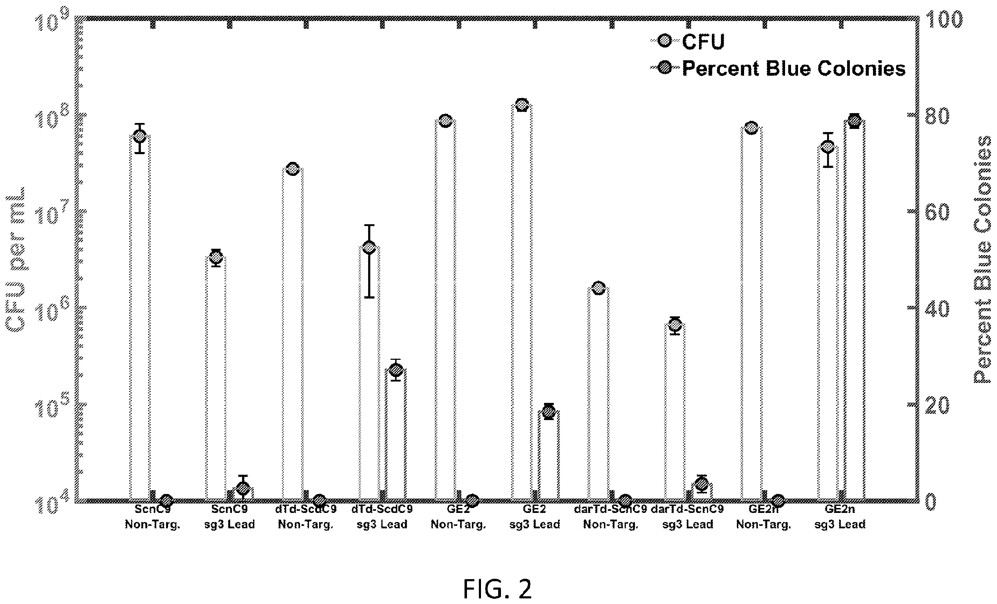

This nicking DarT-ScnCas9 fusion was tested in the *lacZ* repair assay described above using the most efficient target. As shown in FIG. 2, the nickase alone produced low levels of gene repair and a substantial drop in CFUs when expressed with the targeting sgRNA. DarT-ScdCas9 and the engineered DarT_K56A-ScdCas9 (GE2) produced modest levels of gene repair. After reactivating the nicking capacity, DarT-ScnCas9 proved to be cytotoxic, but DarT_K56A-ScnCas9 did not exhibit cytotoxicity and successfully edited nearly 80% of cells after 8 hours of induction. This nicking version of GE2 was referred to as GE2n.

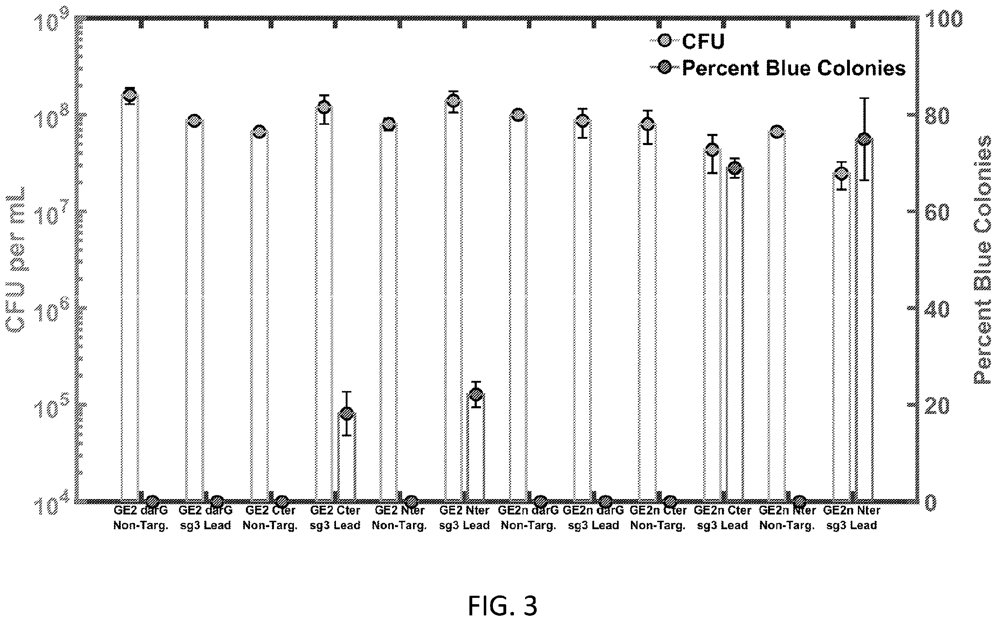

Experiments were also conducted to investigate the use of DarT's antitoxin partner, DarG, to determine whether it would eliminate the genome modificationcapacity of GE2. The N-terminal domain of DarG contains a glycohydrolase which can directly repair ADPr modified thymine. The C-terminal domain of DarG contains a DarT inhibitor. GE2 and GE2n were each co-expressed with full length DarG, the C-terminal domain of DarG, or the N-terminal domain of DarG in an operon in the *lacZ* gene repair assay (FIG. 3). As shown in FIG. 3, GE2 and GE2n genome modification capacity was attenuated when both the N-terminal and C-terminal domains of DarG were expressed. This provides a means to mitigate potential off-target modification effects and toxicity without compromising on-target modification.

Additionally, as would be recognized by one of ordinary skill in the art based on the present disclosure, either the N-terminal or C-terminal domains of DarG can be used to counteract DarT activity. The N-terminal domain can remove ADP ribose, reverting the nucleotide to its original state. The C-terminal domain can directly inhibit DarT activity. Thus, single domains of DarG can be expressed at a low level, and in some cases, randomly distributed through the cell, to help counteract off-target effects of the DarT-Cas protein. In some embodiments, a single DarT domain can be used to reduce off-target effects without affecting on-target genome modification activity.

### Example 3

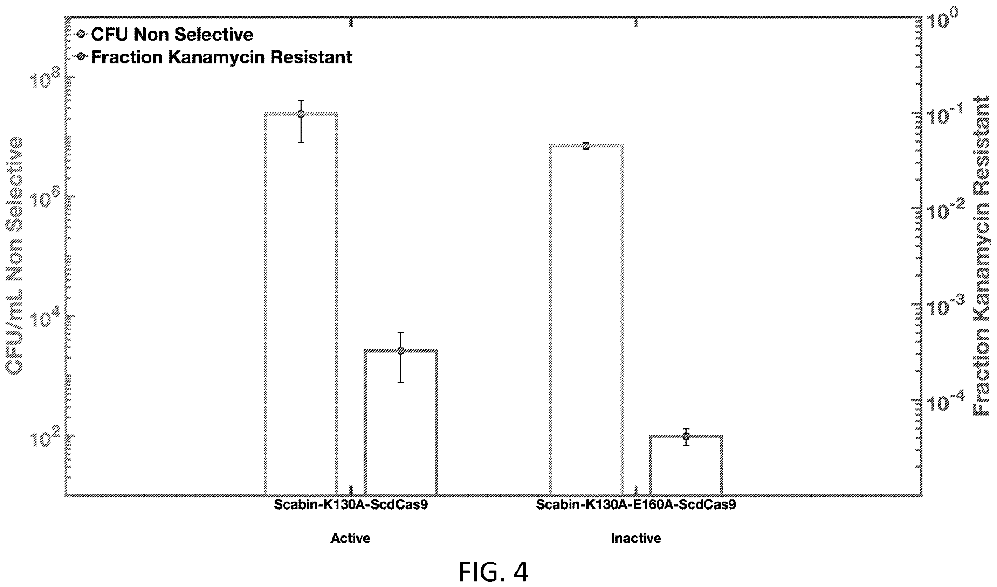

Experiments were conducted to test the ability of a gap editing complex comprising a Scabin DNA-modifying domain in combination with a Cas9 DNA-recognition domain (Scabin-K130A-ScdCas9) to induce successful genome modification, measured based on the frequency of kanamycin gene repair in *E. coli.* In this exemplary set of experiments, expression of a Scabin-dCas9 fusion protein increased the frequency of kanamycin gene repair dependent on Scabin's DNA modification catalytic activity. Scabin is known to modify guanine within single and double-stranded DNA with an adenosine diphosphate ribose group, but it is structurally and evolutionarily divergent from DarT outside of a single shared catalytic motif. Recombination between the plasmid repair template and the targeted defective kanamycin gene in the *E. coli* genome results in repair of the targeted gene, and consequently, kanamycin resistance. Therefore, the fraction of kanamycin resistance serves as a readout for the rate of genome modification. The K130A mutation in Scabin attenuated Scabin's activity, which is otherwise toxic to the cells. The E160A mutation catalytically inactivates Scabin, removing all DNA modification activity (negative control). As shown in FIG. 4, the Scabin-K130A-ScdCas9 gap editor complex resulted in successful genome modification through increased frequency of kanamycin gene repair.

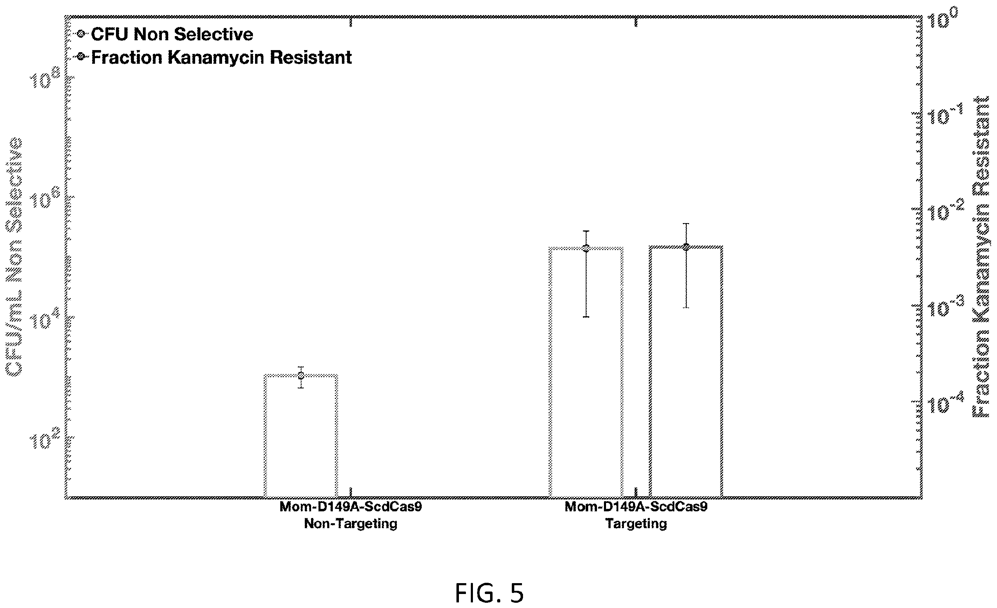

In another set of exemplary experiments, the ability of a gap editing complex comprising a Mom DNA-modifying domain in combination with a Cas9 DNA-recognition domain (Mom-D149A-ScdCas9) to induce successful genome modification, measured based on the frequency of kanamycin gene repair in *E. coli,* was also tested. Fusion of the Mom to dCas9 and targeting a defective kanamycin gene resulted in recombination, genome modification, and thereby kanamycin resistant cells. The Mom protein is known to modify adenine with a methylcarbamoyl group, which is known to block DNA replication, triggering gap repair recombination. The D149A mutation in Mom attenuated the catalytic activity, which is otherwise lethal to the cells. As shown in FIG. 5, the MOM-D149A-ScdCas9 gap editor complex resulted in successful genome modification through increased frequency of kanamycin gene repair.

### Example 4

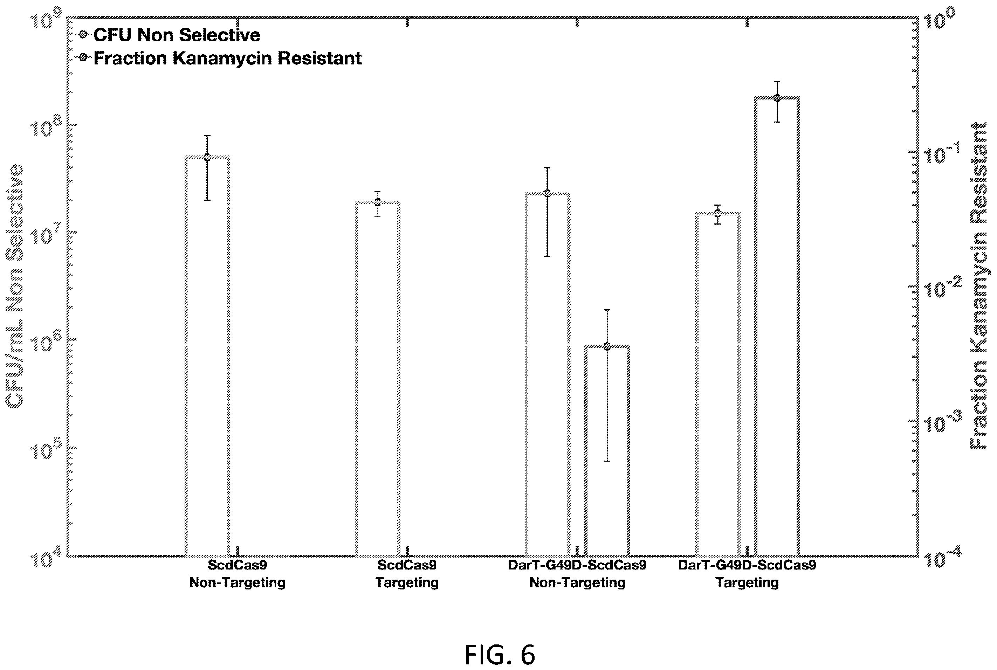

Experiments were also conducted to assess the DNA-modifying domain in the gap editing complexes of the present disclosure. Firstly, FIG. 6 includes representative results of experiments demonstrating that successful genome modification (e.g., though increased frequency of kanamycin gene repair) using gap editor complexes reliant on a DNA-modifying domain (DarT) in combination with a Cas9 DNA-recognition domain (DarT-G49D-ScdCas9). (ScdCas9 alone did not lead to kanamycin gene repair.) DarT was used as an exemplary DNA-modifying domain in these experiments.

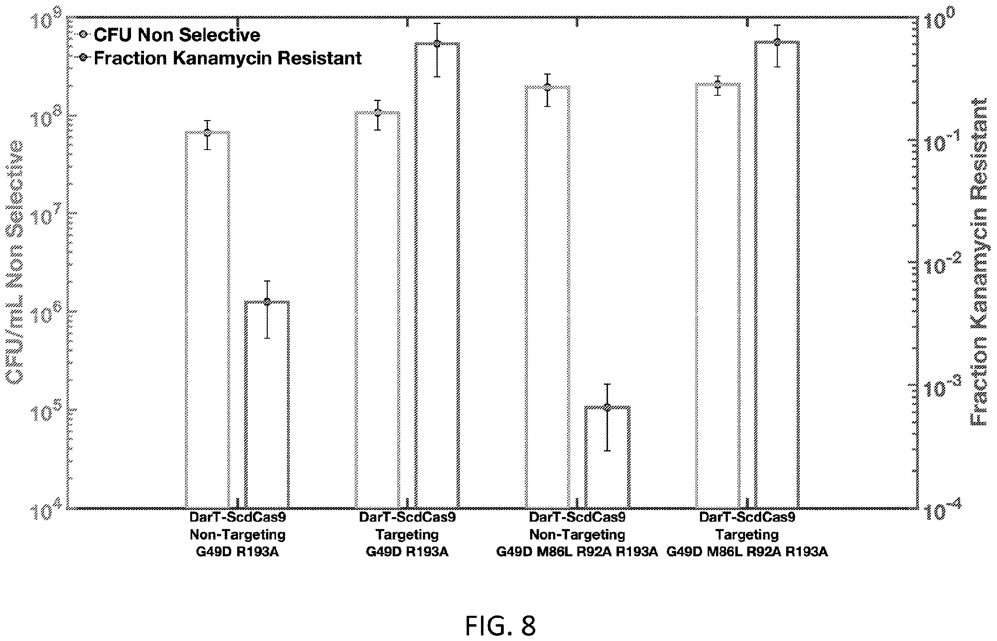

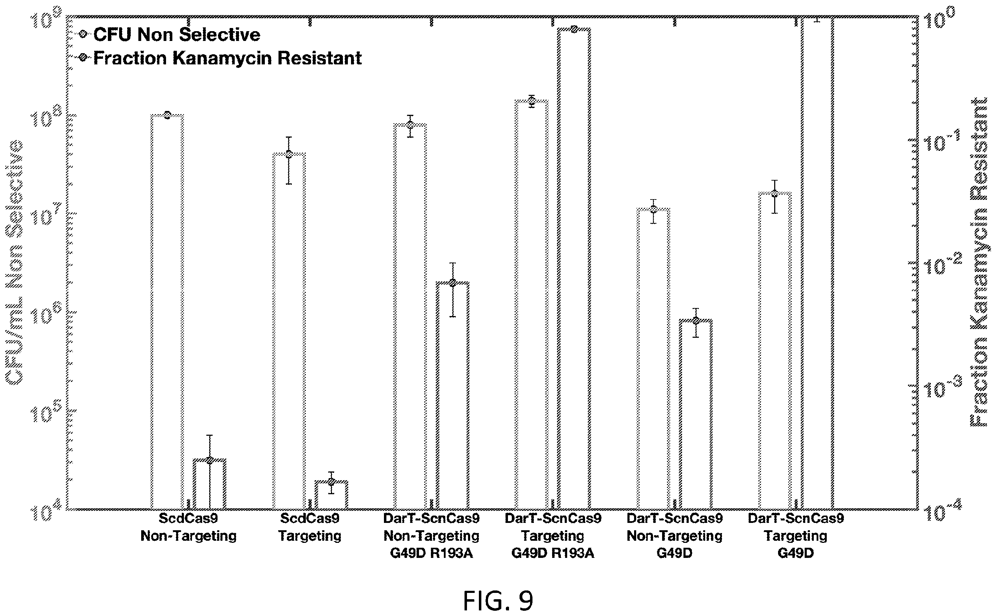

Additionally, experiments were conducted to investigate whether DarT could be improved by reducing its toxic effects on cells. As shown in FIG. 7, introduction of the R193A mutation into DarT (DarT-G49D-R193A-ScdCas9) significantly reduced the toxicity of DarT when expression was induced by the addition of arabinose to the culture media. As shown in FIG. 8, the M86L and R92A mutations further reduced the toxicity of DarT, and also reduced CRISPR independent off-target modification, over and above that of the R193A mutation (FIG. 7). Furthermore, FIG. 9 shows successful genome modification using gap editor complexes comprising a DarT DNA-modifying domain with mutations (G49D and/or R193A) that significantly reduced toxicity in combination with a Cas9 DNA-recognition domain having nickase activity (ScnCas9). Site-specific genome modification was nearly 100% effective.

Thus, these results demonstrate the novel CRISPR-based genome modification technology of the present disclosure, which facilitates efficient site-specific genome modification while minimizing the unintended modification and cellular toxicity associated with current genome editing approaches.

### Example 5

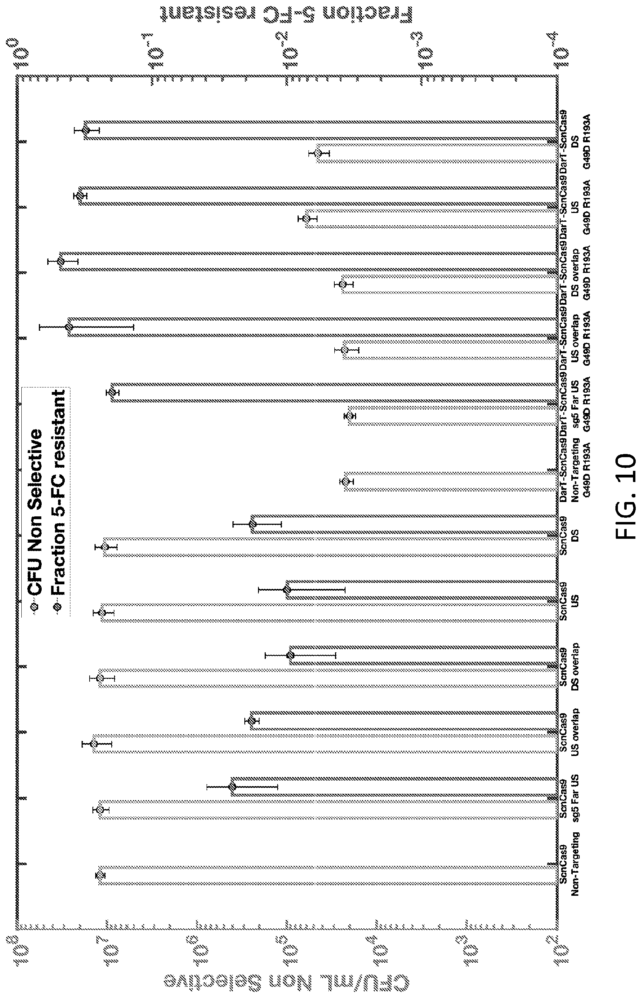

As shown in FIG. 10, experiments were conducted to assess the efficacy of genome modification in eukaryotic cells using the gap editor complexes of the present disclosure by assessing whether gene knockout of *fcy1* is able to confer resistance to 5-Fluorocytosine (5-FC). The *fcy1* gene was targeted in Saccharomyces Cerevisiae with a Cas9 nickase (ScnCas9) or the fusion of an engineered DarT gene to a Cas9 nickase and a repair template was provided. As shown, this resulted in successful genome modification at *fcy1.* The repair template encoded 6 mutations introducing two or three stop codons in *fcy1,* which resulted in a loss of *fcy1* function after genome modification, and resistance to 5-FC. Additionally, as shown, one single guide RNA is combined with 5 different repair templates. For all mutations, the fusion of DarT provided a >10 fold increase in the rate of genome modification, demonstrating the utility of the introduction of replication blocking moieties in a eukaryotic cell.

As shown in FIG. 11, experiments were conducted to assess the efficacy of genome modification using the gap editor complexes of the present disclosure by assessing whether gene knockout of *fcy1* is able to confer resistance to 5-Fluorocytosine (5-FC). The *fcy1* gene was targeted in Saccharomyces Cerevisiae with a Cas9 nickase (ScnCas9) or the fusion of an engineered DarT gene to a Cas9 nickase and a repair template was provided. As shown, this resulted in successful genome modification at *fcy1*. The repair template encoded 6 mutations introducing two or three stop codons in *fcy1,* which resulted in a loss of *fcy1* function after genome modification, and resistance to 5-FC. The use of an engineered DarT variant including the G49D, R193A, M86L and R92A mutations improved cell viability up to approximately 50 fold over DarT with the G49D and R193A mutations alone. This gap editor complex effectuates efficient and low toxicity genome modification using two separate single guide RNAs and repair templates targeting *fcy1* in yeast.

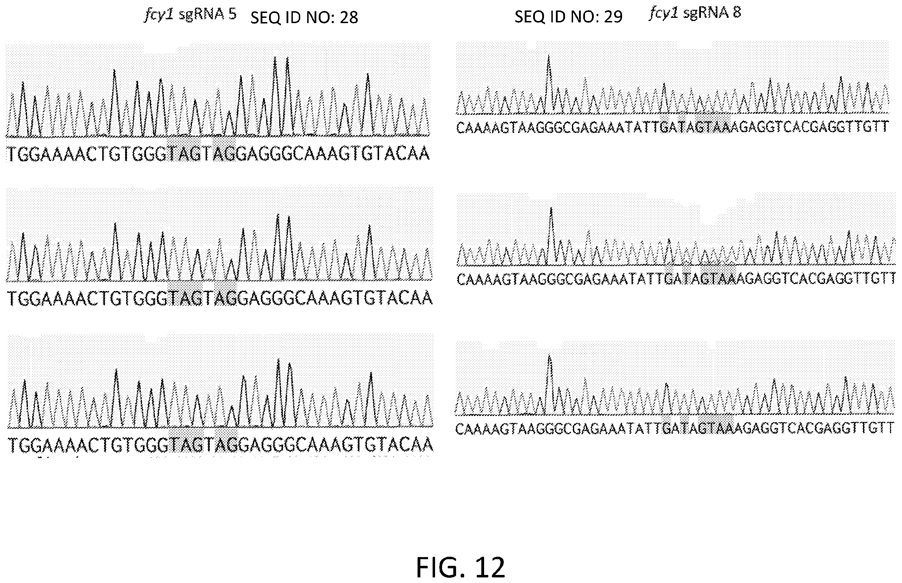

FIG. 12 includes representative chromatographs providing confirmation of *fcy1* genome modification and gene knockout by sanger sequencing. Two or three stop codons were introduced by targeting a gap editor complex to the *fcy1* gene and providing a DNA repair template. The edited nucleotides are highlighted in red. Genomic edits for two separate targets within *fcy1* are shown.

### Example 6

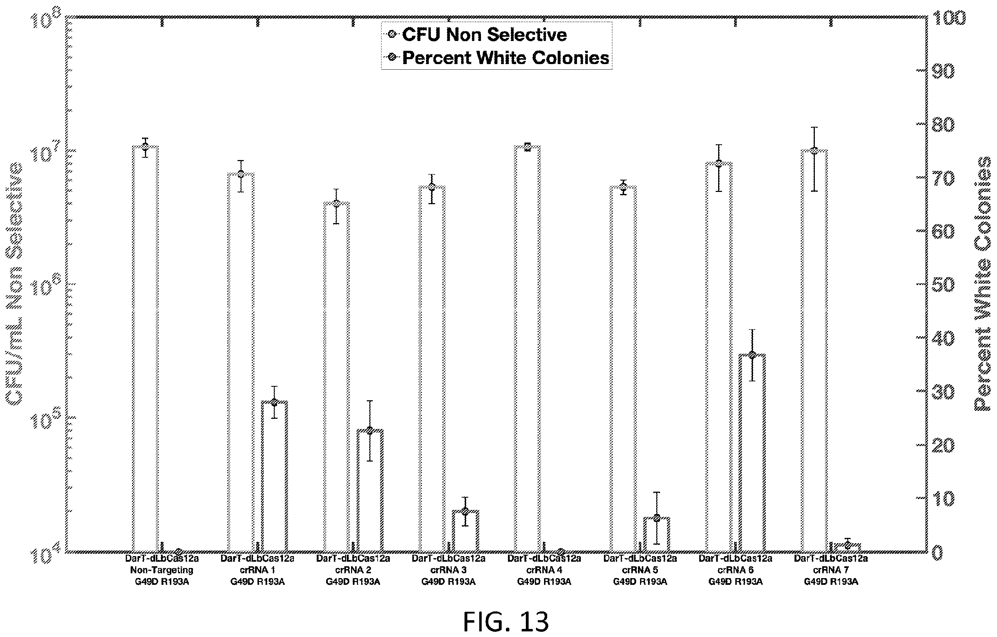

As shown in FIG. 13, experiments were conducted to assess the efficacy of genome modification using the gap editor complexes of the present disclosure by assessing whether gene knockout of *lacZ.* Gene knockout of *lacZ* results in a white colony color in the presence of the lactose analog IPTG and the colorimetric indicator X-gal. The *lacZ* gene was targeted in *E. coli* with a nuclease-inactive Cas12a protein (dLbCas12a) fused to an engineered DarT gene and a repair template was provided. As shown, this resulted in genome modification at *lacZ.* The repair template encoded *lacZ* DNA with a stop codon, which resulted in a loss of *lacZ* function after genome modification, and a white colony color. No genome modification was observed without targeting of the gap editor complex to the *lacZ* gene.

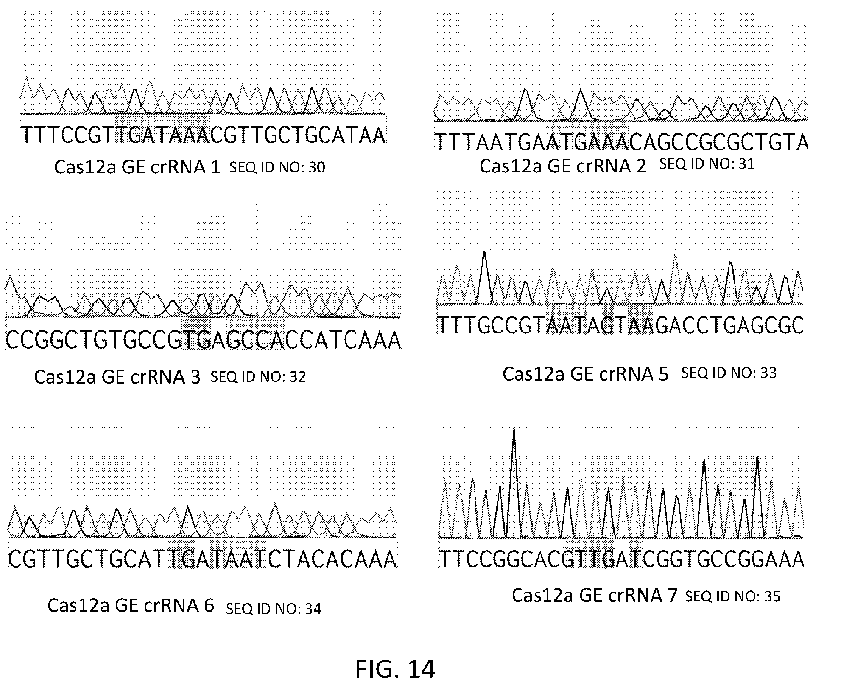

FIG. 14 includes representative chromatographs demonstrating successful introduction of one or more stop codons into the *lacZ* gene using DarT(G49D/R193A)-dLbCas12a associated with different crRNAs. The *lacZ* gene from white colored colonies was amplified and sent for sanger sequencing. Highlighted in red are mutations which introduce one or more stop codons into the *lacZ* gene, eliminating beta-galactosidase expression and thereby resulting in a white colored colony when plated in the presence of the inducer IPTG and the colorimetric indicator X-gal.

### Example 7

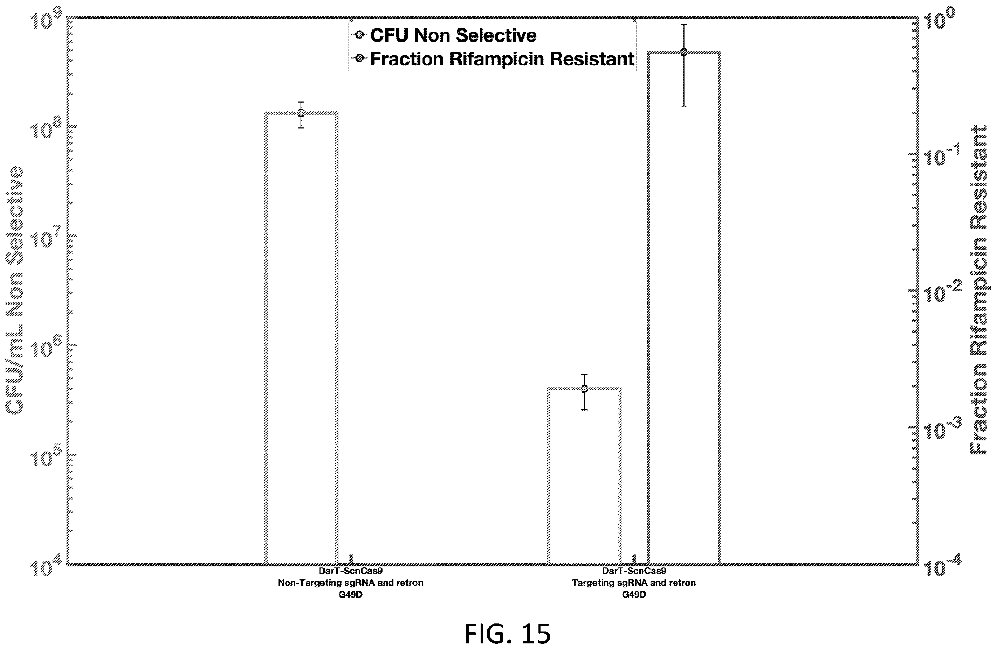

As shown in FIG. 15, experiments were conducted to assess the efficacy of genome modification using the gap editor complexes of the present disclosure by assessing whether the introduction of the D516G mutation into the *rpoB* gene is able to confer resistance to the antibiotic rifampicin. The *rpoB* gene was targeted in *E. coli* with an engineered DarT variant fused to a Cas9 nickase (ScnCas9), and an RNA repair template and a reverse transcriptase were co-expressed. This resulted in successful site-specific RNA templated genome modification. A recT type recombinase was co-expressed to accelerate strand annealing. The RNA repair template encoded the D516G mutation, and was successfully integrated into the genome after targeting by the gap editor complex.

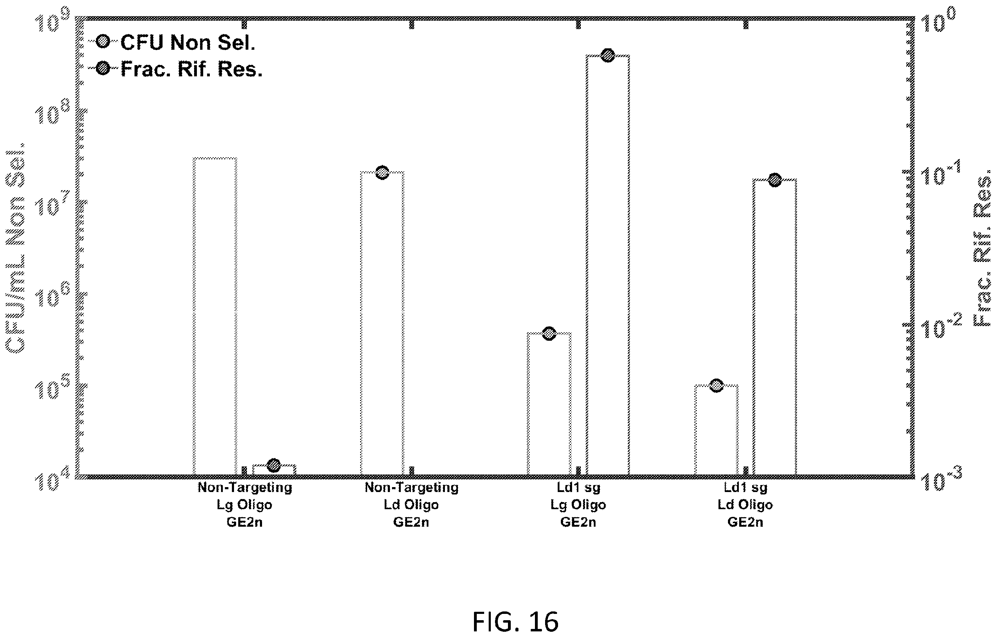

As shown in FIG. 16, experiments were conducted to assess the efficacy of genome modification using the gap editor complexes of the present disclosure by assessing whether the introduction of the D516G mutation into the *rpoB* gene is able to confer resistance to the antibiotic rifampicin. The *rpoB* gene was targeted in E. coli with an engineered DarT variant fused to a Cas9 nickase (ScnCas9) and a linear single-stranded DNA repair template was provided. As shown, this resulted in successful genome modification at *rpoB.* A recT type recombinase was co-expressed to accelerate annealing of the single-stranded DNA repair template. The repair template encoded the D516G mutation conferring rifampicin resistance. Two guides and repair templates were tested, targeting opposite DNA strands at the *rpoB* D516 genomic locus. Targeting of the gap editor complex to *rpoB* resulted in a 100 to 6,000 fold increase in genome modification rates, demonstrating the effect of the gap editors.

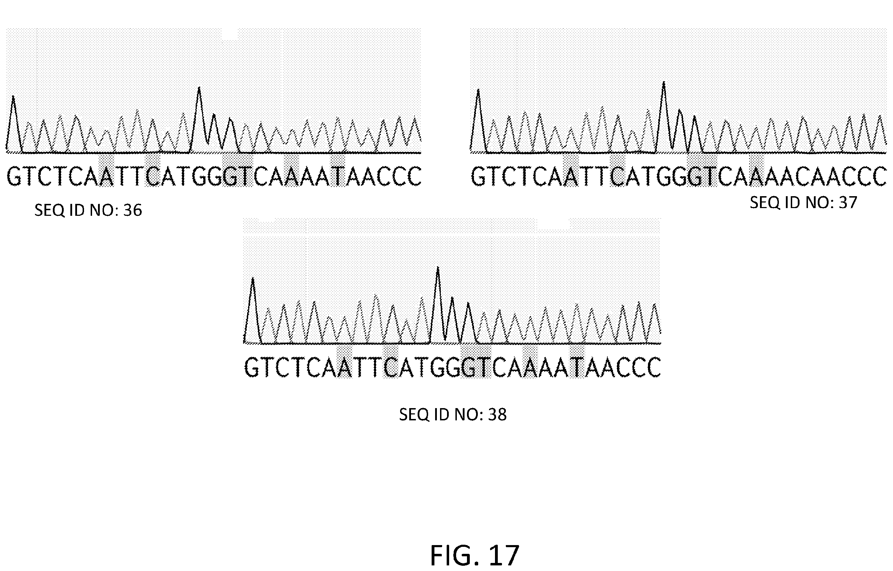

FIG. 17 includes representative chromatograms of the RNA-templated mutations in the *rpoB* gene introduced by the targeting of a gap editor complex to the *rpoB* gene, expression of the RNA repair template, and expression of the reverse transcriptase Ec86. Mutations include the AC>GT mutation required for D516G mediated rifampicin resistance.

***Sequences.*** Sequences of exemplary gap editors as described herein are provided below.
SPC1879 darT G49D-ScdCas9 pBAD araC CmR p15a: 
MAYDYSASLNPQKALIWRIVHRDNIPWILDNGLHCGNSLVQAENWINIDN
PELIGKRAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVRLQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
 
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
EQKKAIVDLLFKTNRKVTVKQLKEDYFKKIECFDSVEIIGVEDRFNASLGTYHDLLKII
KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
GDSLHEQIADLAGSPAIKKGILQTVKIVDELVKVMGHKPENIVIEMARENQTTTKGLQ
QSRERKKRIEEGIKELESQILKENPVENTQLQNEKLYLYYLQNGRDMYVDQELDINR
LSDYDVDAIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD* (SEQ ID NO: 1)
SPC1881 GE2 darT G49D-K56A-ScdCas9 pBAD araC CmR p15a: 
MAYDYSASLNPQKALIWRIVHRDNIPWILDNGLHCGNSLVQAENWINIDN
PELIGARAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVRLQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
 
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
EQKKAIVDLLFKTNRKVTVKQLKEDYFKKIECFDSVEIIGVEDRFNASLGTYHDLLKII
KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
GDSLHEQIADLAGSPAIKKGILQTVKIVDELVKVMGHKPENIVIEMARENQTTTKGLQ
QSRERKKRIEEGIKELESQILKENPVENTQLQNEKLYLYYLQNGRDMYVDQELDINR
LSDYDVDAIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD* (SEQ ID NO: 2)
SPC1883 darT G49D-ScnCas9 pBAD araC CmR p15a: 
MAYDYSASLNPQKALIWRIVHRDNIPWILDNGLHCGNSLVQAENWINIDN
PELIGKRAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVRLQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
 
EQKKAIVDLLFKTNRKVTVKQLKEDYFKKIECFDSVEIIGVEDRFNASLGTYHDLLKII
KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
GDSLHEQIADLAGSPAIKKGILQTVKIVDELVKVMGHKPENIVIEMARENQTTTKGLQ
QSRERKKRIEEGIKELESQILKENPVENTQLQNEKLYLYYLQNGRDMYVDQELDINR
LSDYDVDHIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD* (SEQ ID NO: 3)
SPC1884 GE2n darT G49D-K56A-ScnCas9 pBAD araC CmR p15a: 
MAYDYSASLNPQKALIWRIVHRDNIPWILDNGLHCGNSLVQAENWINIDN
PELIGARAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVRLQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
EQKKAIVDLLFKTNRKVTVKQLKEDYFKKIECFDSVEIIGVEDRFNASLGTYHDLLKII
KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
GDSLHEQIADLAGSPAIKKGILQTVKIVDELVKVMGHKPENIVIEMARENQTTKGLQ
 
QSRERKKRIEEGIKELESQILKENPVENTQLQNEKLYLYYLQNGRDMYVDQELDINR
LSDYDVDHIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD* (SEQ ID NO: 4)
DarG: 
MITYTQGNLLDAPVEALVNTVNTVGVMGKGIALMFKERFPENMKVYALA
CKQKQVITGKMFITETGELMGPRWIVNFPTKQHWRADSRMEWIEDGLQDLRRFLIEE
NVQSIAIPPLGAGNGGLNWPDVRAQIESALGDLQDVDILIYQPTEKYQNVAKSTGVK
KLTPARAAIAELVRRYWVLGMECSLLEIQKLAWLLQRAIEQHQQDDILKLRFEAHYY
GPYAPNLNHLLNALDGTYLKAEKRIPDSQPLDVIWFNDQKKEHVNAYLNNEAREWL
PALEQVSQLIDGFESPFGLELLATVDWLLSRGECQPTLDSVKEGLHQWPAGERWASR
KLRLFDNNNLQFAINRVMEFHC* (SEQ ID NO: 5)
DarG_C-terminal: 
MDVRAQIESALGDLQDVDILIYQPTEKYQNVAKSTGVKKLTPARAAIAELV
RRYWVLGMECSLLEIQKLAWLLQRAIEQHQQDDILKLRFEAHYYGPYAPNLNHLLN
ALDGTYLKAEKRIPDSQPLDVIWFNDQKKEHVNAYLNNEAREWLPALEQVSQLIDG
FESPFGLELLATVDWLLSRGECQPTLDSVKEGLHQWPAGERWASRKLRLFDNNNLQ
FAINRVMEFHC* (SEQ ID NO: 6)
DarG N-terminal: 
MITYTQGNLLDAPVEALVNTVNTVGVMGKGIALMFKERFPENMKVYALA
CKQKQVITGKMFITETGELMGPRWIVNFPTKQHWRADSRMEWIEDGLQDLRRFLIEE
NVQSIAIPPLGAGNGGLNWP* (SEQ ID NO: 7)
Mom: 
MPASIPRRNIVGKEKKSRILTKPCVIEYEGQIVGYGSKELRVETISCWLARTI
IQTKHYSRRFVNNSYLHLGVFSGRDLVGVLQWGYALNPNSGRRVVLETDNRGYME
LNRMWLHDDMPRNSESRAISYALKVIRLLYPSVEWVQSFADERCGRAGVVYQASNF
 
DFIGSHESTFYELDGEWYHEITMNAIKRGGQRGVYLRANKERAVVHKFNQYRYIRFL
NKRARKRLNTKLFKVQPYPK (SEQ ID NO: 8)
Mom_D149A: 
MPASIPRRNIVGKEKKSRILTKPCVIEYEGQIVGYGSKELRVETISCWLARTI
IQTKHYSRRFVNNSYLHLGVFSGRDLVGVLQWGYALNPNSGRRVVLETDNRGYME
LNRMWLHDDMPRNSESRAISYALKVIRLLYPSVEWVQSFAAERCGRAGVVYQASNF
DFIGSHESTFYELDGEWYHEITMNAIKRGGQRGVYLRANKERAVVHKFNQYRYIRFL
NKRARKRLNTKLFKVQPYPK (SEQ ID NO: 9)
Mom_D149A-ScdCas9: 
MPASIPRRNIVGKEKKSRILTKPCVIEYEGQIVGYGSKELRVETISCWLARTI
IQTKHYSRRFVNNSYLHLGVFSGRDLVGVLQWGYALNPNSGRRVVLETDNRGYME
LNRMWLHDDMPRNSESRAISYALKVIRLLYPSVEWVQSFAAERCGRAGVVYQASNF
DFIGSHESTFYELDGEWYHEITMNAIKRGGQRGVYLRANKERAVVHKFNQYRYIRFL
NKRARKRLNTKLFKVQPYPKSGGSSGGSSGSETPGTSESATPESSGGSSGGSEKKYSI
GLAIGTNSVGWAVITDDYKVPSKKFKVLGNTNRKSIKKNLMGALLFDSGETAEATR
LKRTARRRYTRRKNRIRYLQEIFANEMAKLDDSFFQRLEESFLVEEDKKNERHPIFGN
LADEVAYHRNYPTIYHLRKKLADSPEKADLRLIYLALAHIIKFRGHFLIEGKLNAENS
DVAKLFYQLIQTYNQLFEESPLDEIEVDAKGILSARLSKSKRLEKLIAVFPNEKKNGLF
GNIIALALGLTPNFKSNFDLTEDAKLQLSKDTYDDDLDELLGQIGDQYADLFSAAKN
LSDAILLSDILRSNSEVTKAPLSASMVKRYDEHHQDLALLKTLVRQQFPEKYAEIFKD
DTKNGYAGYVGIGIKHRKRTTKLATQEEFYKFIKPILEKMDGAEELLAKLNRDDLLR
KQRTFDNGSIPHQIHLKELHAILRRQEEFYPFLKENREKIEKILTFRIPYYVGPLARGNS
RFAWLTRKSEEAITPWNFEEVVDKGASAQSFIERMTNFDEQLPNKKVLPKHSLLYEY
FTVYNELTKVKYVTERMRKPEFLSGEQKKAIVDLLFKTNRKVTVKQLKEDYFKKIEC
FDSVEIIGVEDRFNASLGTYHDLLKIIKDKDFLDNEENEDILEDIVLTLTLFEDREMIEE
RLKTYAHLFDDKVMKQLKRRHYTGWGRLSRKMINGIRDKQSGKTILDFLKSDGFSN
RNFMQLIHDDSLTFKEEIEKAQVSGQGDSLHEQIADLAGSPAIKKGILQTVKIVDELV
KVMGHKPENIVIEMARENQTTTKGLQQSRERKKRIEEGIKELESQILKENPVENTQLQ
NEKLYLYYLQNGRDMYVDQELDINRLSDYDVDAIVPQSFIKDDSIDNKVLTRSVENR
GKSDNVPSEEVVKKMKNYWRQLLNAKLITQRKFDNLTKAERGGLSEADKAGFIKRQ
LVETRQITKHVARILDSRMNTKRDKNDKPIREVKVITLKSKLVSDFRKDFQLYKVRDI
NNYHHAHDAYLNAVVGTALIKKYPKLESEFVYGDYKVYDVRKMIAKSEQEIGKAT
AKRFFYSNIMNFFKTEVKLANGEIRKRPLIETNGETGEVVWNKEKDFATVRKVLAMP
QVNIVKKTEVQTGGFSKESILSKRESAKLIPRKKGWDTRKYGGFGSPTVAYSILVVAK
 
VEKGKAKKLKSVKVLVGITIMEKGSYEKDPIGFLEAKGYKDIKKELIFKLPKYSLFEL
ENGRRRMLASATELQKANELVLPQHLVRLLYYTQNISATTGSNNLGYIEQHREEFKE
IFEKIIDFSEKYILKNKVNSNLKSSFDEQFAVSDSILLSNSFVSLLKYTSFGASGGFTFL
DLDVKQGRLRYQTVTEVLDATLIYQSITGLYETRTDLSQLGGD (SEQ ID NO: 10)
Scabin: 
MRRRAAAVVLSLSAVLATSAATAPAQTPTATATSAKAAAPACPRFDDPVH
AAADPRVDVERITPDPVWRTTCGTLYRSDSRGPAVVFEQGFLPKDVIDGQYDIESYV
LVNQPSPYVSTTYDHDLYKTWYKSGYNYYIDAPGGVDVNKTIGDRHKWADQVEVA
FPGGIRTEFVIGVCPVDKKTRTEKMSECVGNPHYEPWH (SEQ ID NO: 11)
Scabin_K130A: 
MRRRAAAVVLSLSAVLATSAATAPAQTPTATATSAKAAAPACPRFDDPVH
AAADPRVDVERITPDPVWRTTCGTLYRSDSRGPAVVFEQGFLPKDVIDGQYDIESYV
LVNQPSPYVSTTYDHDLYKTWYASGYNYYIDAPGGVDVNKTIGDRHKWADQVEVA
FPGGIRTEFVIGVCPVDKKTRTEKMSECVGNPHYEPWH (SEQ ID NO: 12)
Scabin_K130A-ScdCas9: 
MRRRAAAVVLSLSAVLATSAATAPAQTPTATATSAKAAAPACPRFDDPVH
AAADPRVDVERITPDPVWRTTCGTLYRSDSRGPAVVFEQGFLPKDVIDGQYDIESYV
LVNQPSPYVSTTYDHDLYKTWYASGYNYYIDAPGGVDVNKTIGDRHKWADQVEVA
FPGGIRTEFVIGVCPVDKKTRTEKMSECVGNPHYEPWHSGGSSGGSSGSETPGTSESA
TPESSGGSSGGSEKKYSIGLAIGTNSVGWAVITDDYKVPSKKFKVLGNTNRKSIKKNL
MGALLFDSGETAEATRLKRTARRRYTRRKNRIRYLQEIFANEMAKLDDSFFQRLEES
FLVEEDKKNERHPIFGNLADEVAYHRNYPTIYHLRKKLADSPEKADLRLIYLALAHII
KFRGHFLIEGKLNAENSDVAKLFYQLIQTYNQLFEESPLDEIEVDAKGILSARLSKSKR
LEKLIAVFPNEKKNGLFGNIIALALGLTPNFKSNFDLTEDAKLQLSKDTYDDDLDELL
GQIGDQYADLFSAAKNLSDAILLSDILRSNSEVTKAPLSASMVKRYDEHHQDLALLK
TLVRQQFPEKYAEIFKDDTKNGYAGYVGIGIKHRKRTTKLATQEEFYKFIKPILEKMD
GAEELLAKLNRDDLLRKQRTFDNGSIPHQIHLKELHAILRRQEEFYPFLKENREKIEKI
LTFRIPYYVGPLARGNSRFAWLTRKSEEAITPWNFEEVVDKGASAQSFIERMTNFDE
QLPNKKVLPKHSLLYEYFTVYNELTKVKYVTERMRKPEFLSGEQKKAIVDLLFKTNR
KVTVKQLKEDYFKKIECFDSVEIIGVEDRFNASLGTYHDLLKIIKDKDFLDNEENEDIL
EDIVLTLTLFEDREMIEERLKTYAHLFDDKVMKQLKRRHYTGWGRLSRKMINGIRD
KQSGKTILDFLKSDGFSNRNFMQLIHDDSLTFKEEIEKAQVSGQGDSLHEQIADLAGS
PAIKKGILQTVKIVDELVKVMGHKPENIVIEMARENQTTTKGLQQSRERKKRIEEGIK
ELESQILKENPVENTQLQNEKLYLYYLQNGRDMYVDQELDINRLSDYDVDAIVPQSF
 
IKDDSIDNKVLTRSVENRGKSDNVPSEEVVKKMKNYWRQLLNAKLITQRKFDNLTK
AERGGLSEADKAGFIKRQLVETRQITKHVARILDSRMNTKRDKNDKPIREVKVITLKS
KLVSDFRKDFQLYKVRDINNYHHAHDAYLNAVVGTALIKKYPKLESEFVYGDYKV
YDVRKMIAKSEQEIGKATAKRFFYSNIMNFFKTEVKLANGEIRKRPLIETNGETGEVV
WNKEKDFATVRKVLAMPQVNIVKKTEVQTGGFSKESILSKRESAKLIPRKKGWDTR
KYGGFGSPTVAYSILVVAKVEKGKAKKLKSVKVLVGITIMEKGSYEKDPIGFLEAKG
YKDIKKELIFKLPKYSLFELENGRRRMLASATELQKANELVLPQHLVRLLYYTQNISA
TTGSNNLGYIEQHREEFKEIFEKIIDFSEKYILKNKVNSNLKSSFDEQFAVSDSILLSNS
FVSLLKYTSFGASGGFTFLDLDVKQGRLRYQTVTEVLDATLIYQSITGLYETRTDLSQ
LGGD (SEQ ID NO: 13)
DarT_G49D_R193A: 
MAYDYSASLNPQKALIWRIVHRDNIPWILDNGLHCGNSLVQAENWINIDN
PELIGKRAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVALQLEQWLFQRNLTMSVHTRSGWYFS (SEQ
ID NO: 14)
DarT_G49D_R193A-ScdCas9: 
MAYDYSASLNPQKALIWRIVHRDNIPWILDNGLHCGNSLVQAENWINIDN
PELIGKRAGHPVPVGTGGTLHDYVPFYFTPFSPMLMNIHSGRGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVALQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
EQKKAIVDLLFKTNRKVTVKQLKEDYFKKIECFDSVEIIGVEDRFNASLGTYHDLLKII
KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
 
GDSLHEQIADLAGSPAIKKGILQTVKIVDELVKVMGHKPENIVIEMARENQTTTKGLQ
QSRERKKRIEEGIKELESQILKENPVENTQLQNEKLYLYYLQNGRDMYVDQELDINR
LSDYDVDAIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD (SEQ ID NO: 15)
DarT_G49D_R193A_M86L_R92A: 
MAYDYSASLNPQKALIWRIVHRDNIPWILDNGLHCGNSLVQAENWINIDN
PELIGKRAGHPVPVGTGGTLHDYVPFYFTPFSPMLLNIHSGAGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVALQLEQWLFQRNLTMSVHTRSGWYFS (SEQ
ID NO: 16)
DarT_G49D_R193A_M86L_R92A-ScdCas9

MAYDYSASLNPQKALIWRIVHRDNIPWILDNGLHCGNSLVQAENWINIDN
PELIGKRAGHPVPVGTGGTLHDYVPFYFTPFSPMLLNIHSGAGGIKRRPNEEIVILVSN
LRNVAAHDVPFVFTDSHAYYNWTNYYTSLNSLDQIDWPILQARDFRRDPDDPAKFE
RYQAEALIWQHCPISLLDGIICYSEEVALQLEQWLFQRNLTMSVHTRSGWYFSSGGSS
GGSSGSETPGTSESATPESSGGSSGGSEKKYSIGLAIGTNSVGWAVITDDYKVPSKKF
KVLGNTNRKSIKKNLMGALLFDSGETAEATRLKRTARRRYTRRKNRIRYLQEIFANE
MAKLDDSFFQRLEESFLVEEDKKNERHPIFGNLADEVAYHRNYPTIYHLRKKLADSP
EKADLRLIYLALAHIIKFRGHFLIEGKLNAENSDVAKLFYQLIQTYNQLFEESPLDEIE
VDAKGILSARLSKSKRLEKLIAVFPNEKKNGLFGNIIALALGLTPNFKSNFDLTEDAKL
QLSKDTYDDDLDELLGQIGDQYADLFSAAKNLSDAILLSDILRSNSEVTKAPLSASMV
KRYDEHHQDLALLKTLVRQQFPEKYAEIFKDDTKNGYAGYVGIGIKHRKRTTKLAT
QEEFYKFIKPILEKMDGAEELLAKLNRDDLLRKQRTFDNGSIPHQIHLKELHAILRRQ
EEFYPFLKENREKIEKILTFRIPYYVGPLARGNSRFAWLTRKSEEAITPWNFEEVVDKG
ASAQSFIERMTNFDEQLPNKKVLPKHSLLYEYFTVYNELTKVKYVTERMRKPEFLSG
EQKKAIVDLLFKTNRKVTVKQLKEDYFKKIECFDSVEIIGVEDRFNASLGTYHDLLKII

KDKDFLDNEENEDILEDIVLTLTLFEDREMIEERLKTYAHLFDDKVMKQLKRRHYTG
WGRLSRKMINGIRDKQSGKTILDFLKSDGFSNRNFMQLIHDDSLTFKEEIEKAQVSGQ
GDSLHEQIADLAGSPAIKKGILQTVKIVDELVKVMGHKPENIVIEMARENQTTTKGLQ
QSRERKKRIEEGIKELESQILKENPVENTQLQNEKLYLYYLQNGRDMYVDQELDINR
LSDYDVDAIVPQSFIKDDSIDNKVLTRSVENRGKSDNVPSEEVVKKMKNYWRQLLN
AKLITQRKFDNLTKAERGGLSEADKAGFIKRQLVETRQITKHVARILDSRMNTKRDK
NDKPIREVKVITLKSKLVSDFRKDFQLYKVRDINNYHHAHDAYLNAVVGTALIKKYP
KLESEFVYGDYKVYDVRKMIAKSEQEIGKATAKRFFYSNIMNFFKTEVKLANGEIRK
RPLIETNGETGEVVWNKEKDFATVRKVLAMPQVNIVKKTEVQTGGFSKESILSKRES
AKLIPRKKGWDTRKYGGFGSPTVAYSILVVAKVEKGKAKKLKSVKVLVGITIMEKG
SYEKDPIGFLEAKGYKDIKKELIFKLPKYSLFELENGRRRMLASATELQKANELVLPQ
HLVRLLYYTQNISATTGSNNLGYIEQHREEFKEIFEKIIDFSEKYILKNKVNSNLKSSFD
EQFAVSDSILLSNSFVSLLKYTSFGASGGFTFLDLDVKQGRLRYQTVTEVLDATLIYQ
SITGLYETRTDLSQLGGD (SEQ ID NO: 17)

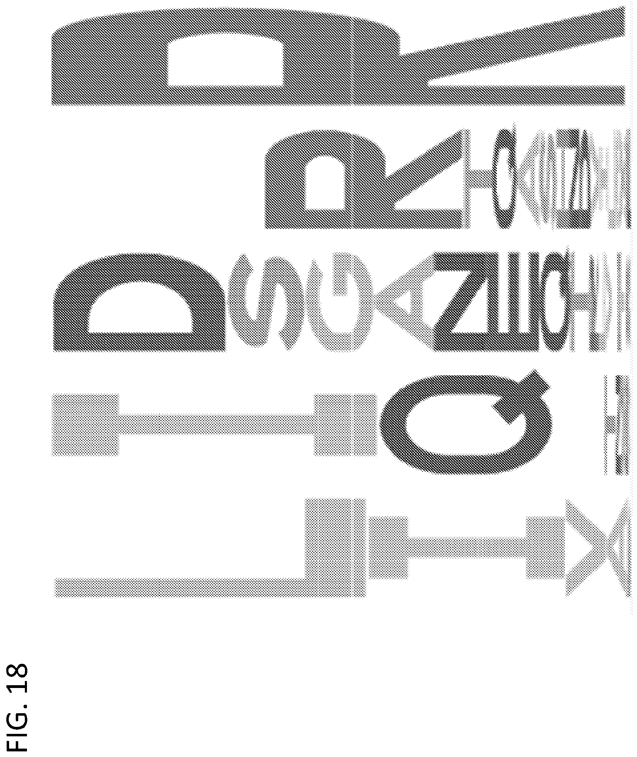

DarT catalytic domain motif: X₁X₂X₃X₃R (SEQ ID NO: 18), wherein X₁ is L, I, V, or A; X₂ is I, Q, K, T, or N; and X₃ is any amino acid (FIG. 18).

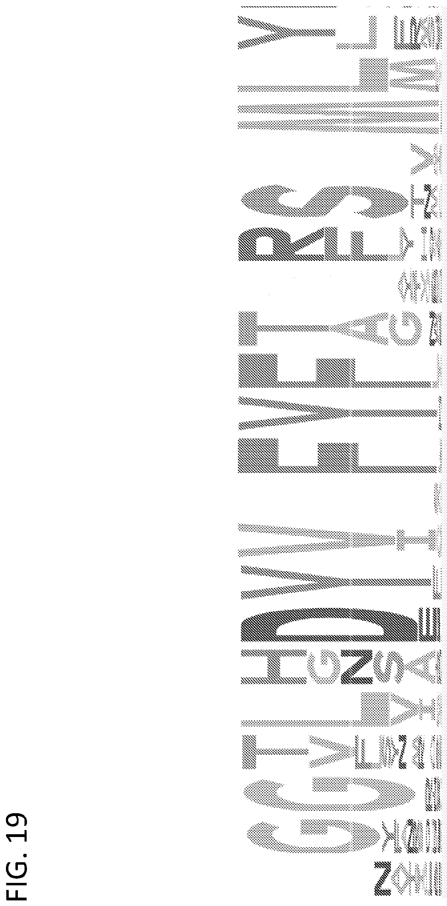

DarT catalytic domain motif: X₁X₁X₁X₁X₂X₃X₄X₅X₆PFYFX₇X₁X₁X₈X₉MX₁₀X1 (SEQ ID NO: 19), wherein X₁ is any amino acid; X₂ is L, V, or I; X₃ is H, G, N, S, or A; X₄ is D or E; X₅ is Y or F; X₆ is V, I, or A; X₇ is T, A, G, K, N, or W; X₈ is S, T, N, M, or K; and X₉ is P, V, M, I, A; X₁₀ is L, M or F (FIG. 19).

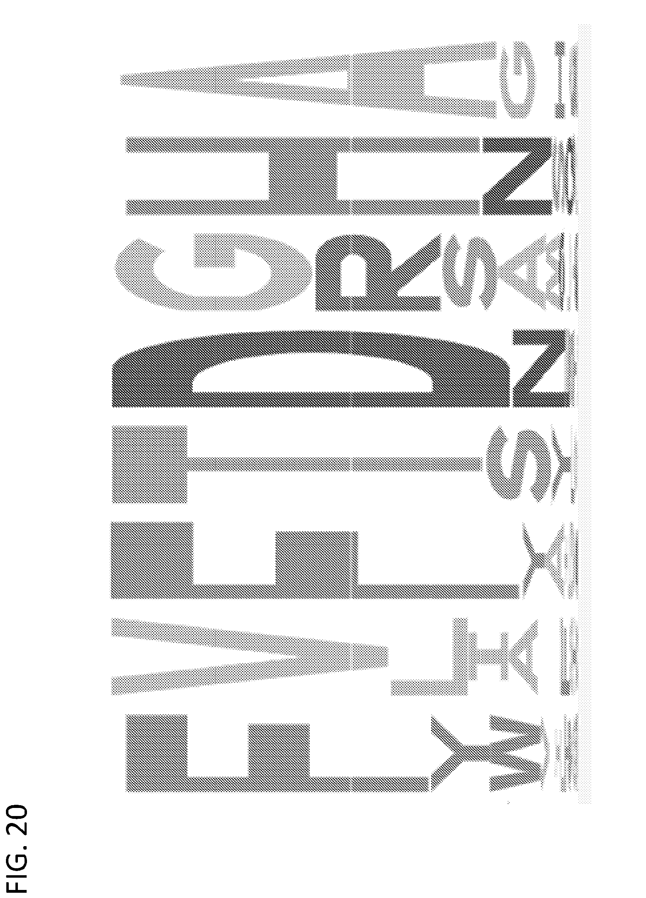

DarT catalytic domain motif: X₁X₂X₃X₄X₅X₆X₇X₈ (SEQ ID NO: 20), wherein X₁ is F, Y, W, V, or C; X₂ is V, L, I, A, C, or F; X₃ is F, Y, or A; X₄ is T, S, Y, or F; X₅ is D, N, or S; X₆ is G, R, S, A, M or Q; X₇ is H, N, S, or Q; and X₈ is A, G, C, H or K (FIG. 20).

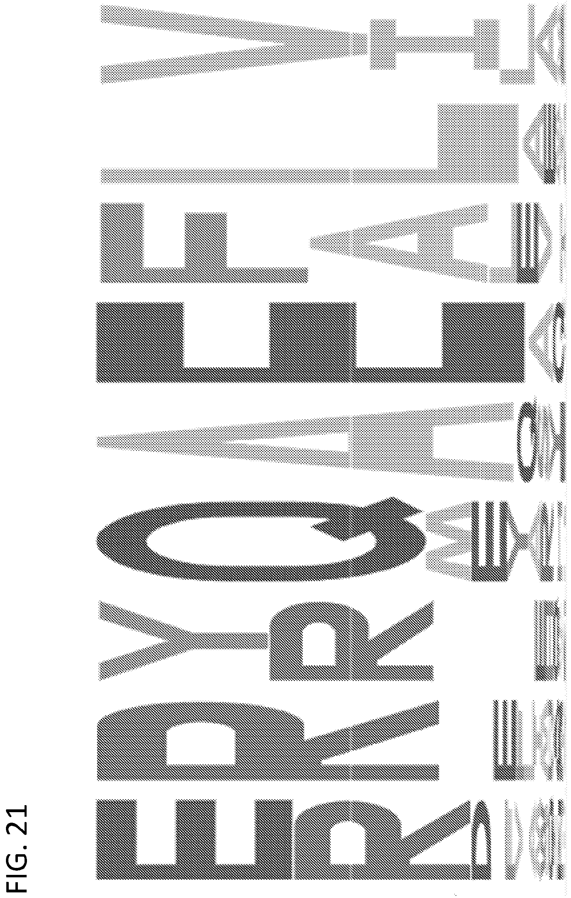

DarT catalytic domain motif: X₁X₂X₃X₄X₅X₆X₇X₈X₉ (SEQ ID NO: 21), wherein X₁ is and amino acid; X₂ is R, K, H, E, F, L, T, or M; X₃ is Y, R, K, D, E, or H; X₄ is Q, M, E, Y, A, R, or H; X₅ is A Q, S, or Y; X₆ is E, A, or Q; X₇ is F, A, L, E, V, or C; X₈ is L, A, E, or M; and X₉ is V, I, L, or A (FIG. 21).

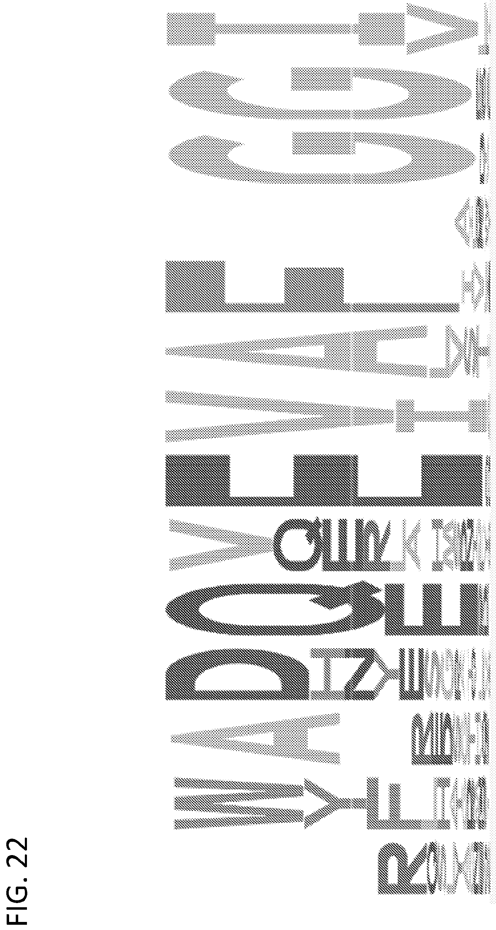

Scabin catalytic domain motif: X₁X₁X₁X₁X₂X₁EX₃X₄X₅X₆GGX₇ (SEQ ID NO: 22), wherein X₁ is and amino acid; X₂ is Q, E, or R; X₃ is V or I; X₄ is A, L, V, S, or T; X₅ is F, I, V, or L; X₆ is P, A, or I; and X₇ is I, V, or L (FIG. 22). DarT catalytic motif of SEQ ID NO: 21 and Scabin catalytic motif of SEQ ID NO: 22 are structural and functional analogs, with the conserved glutamate (E) being the catalytic residue.

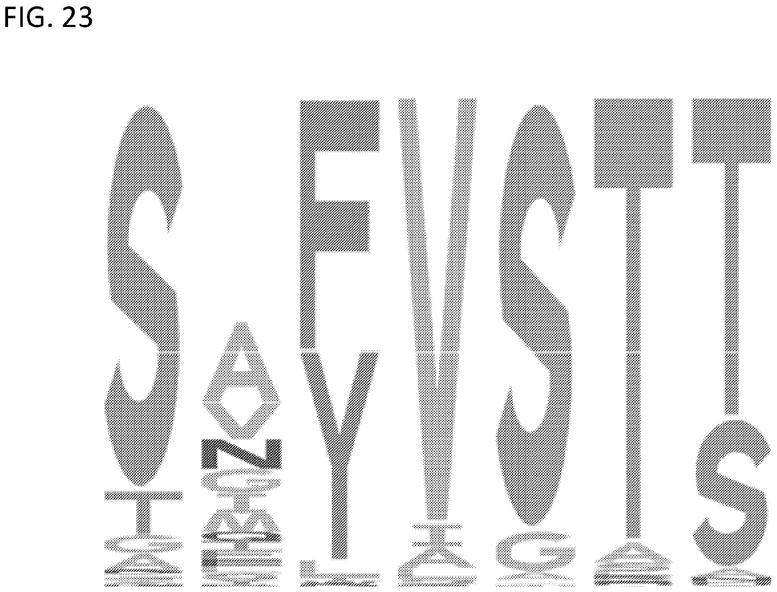

Scabin catalytic domain motif: X₁X₂X₃X₄X₅X₆X₇ (SEQ ID NO: 23), wherein X₁ is S, T, or G; X₂ is any amino acid; X₃ is F, Y, or L; X₄ is V, I, A, or L; X₅ is S, G, or A; X₆ is T or A; and X₇ is T, S, or A (FIG. 23).

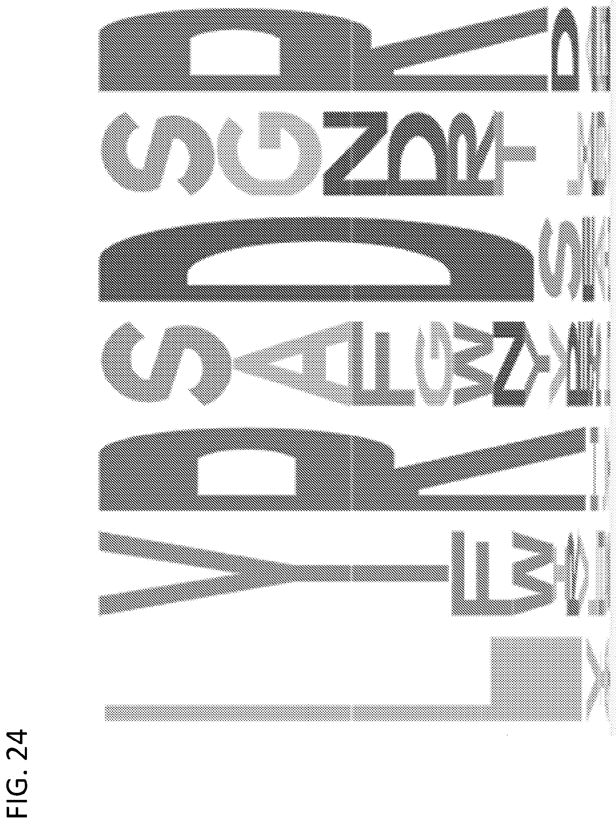

Scabin catalytic domain motif: X₁X₂X₃X₂X₄X₂X₅ (SEQ ID NO: 24), wherein X₁ is L or V; X₂ is any amino acid; X₃ is R, H, or K; X₄ is D, S, or A; and X₅ is R or D (FIG. 24).

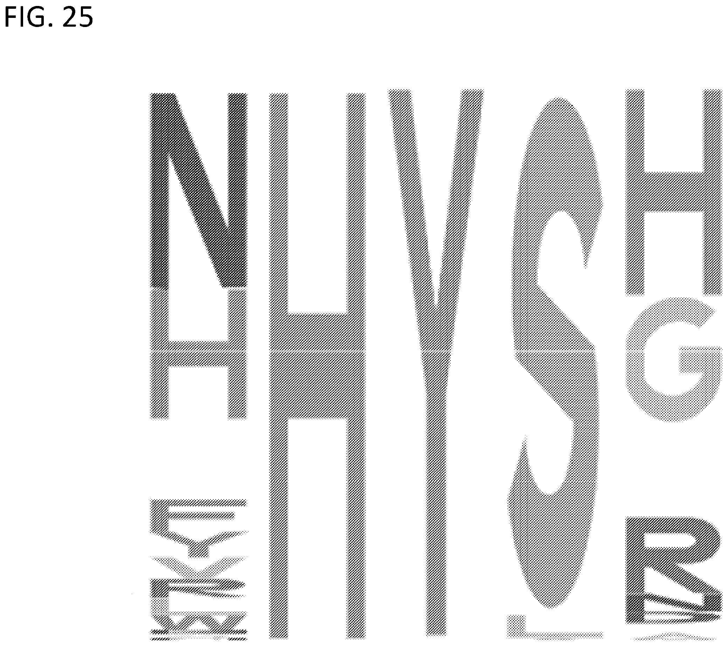

Mom catalytic domain motif: X₁HYX₂X₃ (SEQ ID NO: 25), wherein X₁ is any amino acid; X₂ is S or L; and X₃ is H, G, K, R, N, D, or A (FIG. 25).

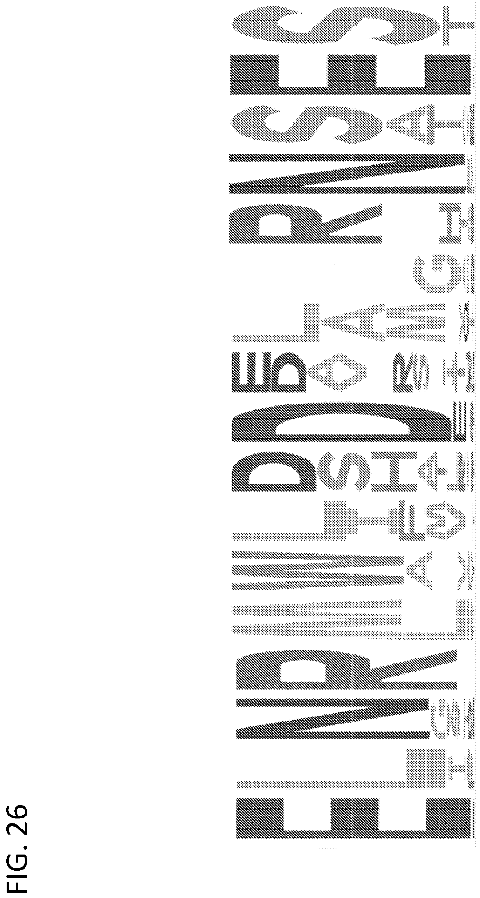

Mom catalytic domain motif: EX₁X₂X₃X₄X₅X₆X₇X₈X₇X₉X₁₀X₁₁X₁₂X₁₃EX₁₄ (SEQ ID NO: 26), wherein X₁ is L, I, or F; X₂ is N, G, S, or T; X₃ is R or K; X₄ is M, L, or A; X₅ is W, A, C, V, F, or Y; X₆ is L, I, F, M, V, C, or T; X₇ is any amino acid; X₈ is D or E; X₉ is L A M, C, V, Q, or T; X₁₀ is P, G, A, or L; X₁₁ is R, K, H, T, or M; X₁₂ is N or F; X₁₃ is S, A, T, or G; and X₁₄ is S or T (FIG. 26).

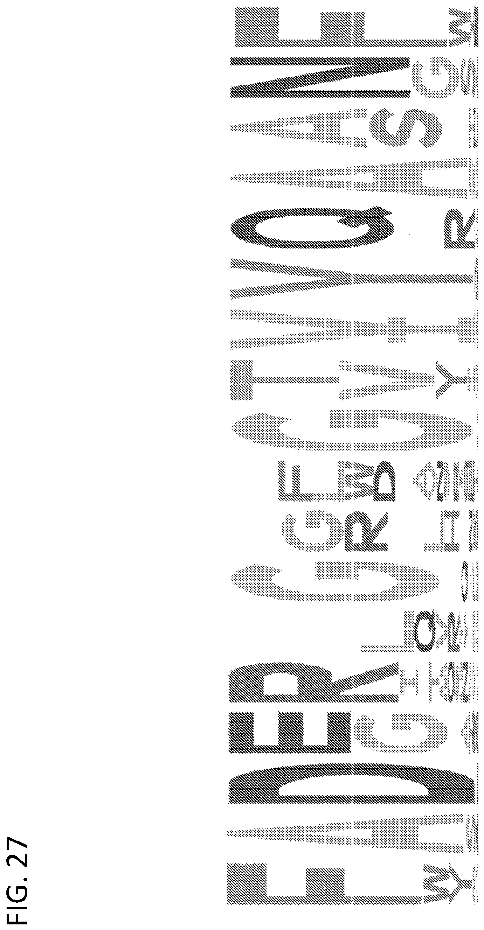

Mom catalytic domain motif: X₁X₂DX₃X₄X₄X₅X₄X₄GX₆X₇YX₈AX₉X₁₀X₁₁ (SEQ ID NO: 27), wherein X₁ is F, W, Y, or M; X₂ is A or S; X₃ is E, G, P, A, or T; X₄ is any amino acid; X₅ is G, C, or Q; X₆ is T, V, Y, or I; X₇ is V or I; X₅ is Q, K, or R; X₉ is A, S, C, T, or N; X₁₀ is N, G, or A; X₁₁ is F, W, or Y (FIG. 27).

## Claims

1. A composition or kit comprising said composition for targeted genome modification, the composition comprising a gap editor complex comprising a DNA-recognition domain and a DNA-modifying domain, wherein the DNA-recognition domain binds a DNA target sequence in the genome, and wherein said DNA modifying domain is an ADP-ribsoyltransferase.

2. The composition or kit of claim 1, wherein the composition further comprises a donor nucleic acid template, wherein preferably the donor nucleic acid template comprises a polynucleotide from an endogenous homologous sequence corresponding to the DNA target sequence, wherein further preferably the donor nucleic acid template comprises an exogenous single-stranded DNA (ssDNA) molecule, a double-stranded DNA (dsDNA) molecule, or an RNA molecule.

3. The composition or kit of claim 2, wherein the presence of the donor nucleic acid template facilitates homology-directed gap repair and/or recombination, wherein the donor nucleic acid template or a fragment thereof is recombined into the genome of the DNA target sequence.

4. The composition or kit of any of claims 1 to 3, wherein the composition comprises at least one guide RNA molecule, wherein said at least one guide RNA is preferably further defined by at least one of the following:
i) said at least one guide RNA comprises gRNA, sgRNA, crRNA, or any combinations thereof;
ii) the at least one guide RNA comprises a handle sequence and a targeting sequence, wherein preferably the targeting sequence in the at least one guide RNA is complementary to the DNA target sequence.

5. The composition or kit of any of claims 1 to 4, wherein the DNA-recognition domain is further defined by at least one of the following:
i) the DNA-recognition domain comprises at least one Cas protein or fragment thereof lacking deoxyribonuclease activity;
ii) the DNA-recognition domain comprises a complex of Cas proteins lacking deoxyribonuclease activity;
iii) the DNA-recognition domain comprises a Cas protein or fragment thereof having nickase activity.

6. The composition or kit of any of claims 1 to 5, wherein the Cas protein or Cas protein complex comprises a Type I Cascade, a Type II Cas9, a Type IV effector module, a Type V Cas12, a Cas9-related IscB, a Cas12a-related TnpB, and combinations thereof.

7. The composition or kit of any of claims 1 to 6, wherein the DNA-recognition domain and the DNA-modifying domain are functionally coupled, wherein preferably functionally coupled comprises polypeptide fusions, peptide tags, peptide linkers, RNA tags, and any combinations thereof.

8. The composition or kit of any of claims 1 to 7, wherein the DNA-recognition domain induces a single-stranded break in the DNA target strand, and wherein the DNA-modifying domain adds a replication blocking moiety to at least one nucleotide in the DNA strand complementary to the DNA target sequence.

9. The composition or kit of any of claims 1 to 8, wherein the DNA-modifying domain catalyzes addition of ADP ribose to a thymine or guanine nucleotide.

10. The composition or kit of any of claims 1 to 9, wherein the DNA-modifying domain comprises a DarT enzyme or a functional fragment, derivative, or variant thereof.

11. The composition or kit of any of claims 1 to 10, wherein the composition further comprises at least one gap editor accessory factor, wherein the at least one gap editor accessory factor preferably
i) comprises a protein that augments at least one step in a genome modification process;
ii) is recruited to the gap editor complex via interaction with the DNA-modifying domain, the DNA-recognition domain, and/or the at least one guide RNA, wherein further preferably the recruitment of the at least one gap editor accessory factor to the gap editor complex comprises a peptide tag, a peptide linker, an RNA tag, and any combinations thereof;
iii) comprises Rap, DarG, Orf, ExoI, Exonuclease III, PrimPol , RecJ, RecQ1, Rad51, Rad52, CtIP, Rad18, and any combinations thereof.

12. An *in vitro* method for targeted genome modification, the method comprising:
introducing any of the compositions of claims 1 to 11 into a cell; and
assessing the cell for presence of a desired genome alteration.

13. The method of claim 12, wherein the gap editor complex and/or the at least one guide RNA molecule are introduced into the cell as a polypeptide(s), mRNA(s), and/or DNA expression construct(s).

14. The method of claim 12, wherein the cell is a prokaryotic cell or a eukaryotic cell.

15. The method of any of claims 12 to 14, wherein the method leads to a reduced degree of indel formation, chromosomal rearrangements, DNA duplications, enhances cell viability, and/or reduces cell toxicity.

## Patentansprüche

1. Zusammensetzung oder Kit, das diese Zusammensetzung für eine gezielte Genom-Modifikation umfasst, wobei die Zusammensetzung einen Lücken-Editor-Komplex umfasst, der eine DNA-Erkennungsdomäne und eine DNA-Modifizierungsdomäne umfasst, wobei die DNA-Erkennungsdomäne an eine DNA-Zielsequenz im Genom bindet und wobei die DNA-Modifizierungsdomäne eine ADP-Ribosyltransferase ist.

2. Zusammensetzung oder Kit nach Anspruch 1, wobei die Zusammensetzung ferner eine Donor-Nukleinsäure-Matrize umfasst, wobei die Donor-Nukleinsäure-Matrize vorzugsweise ein Polynukleotid aus einer endogenen homologen Sequenz umfasst, die der DNA-Zielsequenz entspricht, wobei die Donor-Nukleinsäure-Matrize ferner vorzugsweise ein exogenes einzelsträngiges DNA-Molekül (ssDNA), ein doppelsträngiges DNA-Molekül (dsDNA) oder ein RNA-Molekül umfasst.

3. Zusammensetzung oder Kit nach Anspruch 2, wobei das Vorhandensein der Donor-Nukleinsäure-Matrize eine homologe-gerichtete Lückenreparatur und/oder Rekombination erleichtert, wobei die Donor-Nukleinsäure-Matrize oder ein Fragment davon in das Genom der DNA-Zielsequenz rekombiniert wird.

4. Zusammensetzung oder Kit nach einem der Ansprüche 1 bis 3, wobei die Zusammensetzung mindestens ein Leit-RNA-Molekül umfasst, wobei die mindestens eine Leit-RNA vorzugsweise ferner durch mindestens eines der folgenden Merkmale definiert ist:
i) die mindestens eine Leit-RNA umfasst gRNA, sgRNA, crRNA oder beliebige Kombinationen davon;
ii) die mindestens eine Leit-RNA umfasst eine Handle-Sequenz und eine Zielsequenz, wobei vorzugsweise die Zielsequenz in der mindestens einen Leit-RNA komplementär zur DNA-Zielsequenz ist.

5. Zusammensetzung oder Kit nach einem der Ansprüche 1 bis 4, wobei die DNA-Erkennungsdomäne ferner vorzugsweise durch mindestens eines der folgenden Merkmale definiert ist:
i) die DNA-Erkennungsdomäne umfasst mindestens ein Cas-Protein oder ein Fragment davon, dem die Desoxyribonuklease-Aktivität fehlt;
ii) die DNA-Erkennungsdomäne umfasst einen Komplex aus Cas-Proteinen, denen die Desoxyribonukleaseaktivität fehlt;
iii) die DNA-Erkennungsdomäne umfasst ein Cas-Protein oder ein Fragment davon mit Nickase-Aktivität.

6. Zusammensetzung oder Kit nach einem der Ansprüche 1 bis 5, wobei das Cas-Protein oder der Cas-Proteinkomplex eine Typ-I-Cascade, ein Typ-II-Cas9, ein Typ-IV-Effektormodul, ein Typ-V-Cas12, ein Cas9-verwandtes IscB, ein Cas12a-verwandtes TnpB und Kombinationen davon umfasst.

7. Zusammensetzung oder Kit nach einem der Ansprüche 1 bis 6, wobei die DNA-Erkennungsdomäne und die DNA-Modifizierungsdomäne funktionell gekoppelt sind, wobei funktionell gekoppelt vorzugsweise Polypeptidfusionen, Peptid-Tags, Peptid-Linker, RNA-Tags und beliebige Kombinationen davon umfasst.

8. Zusammensetzung oder Kit nach einem der Ansprüche 1 bis 7, wobei die DNA-Erkennungsdomäne einen einzelsträngigen Bruch im DNA-Zielstrang induziert und wobei die DNA-Modifizierungsdomäne eine replikationsblockierende Einheit an mindestens ein Nukleotid im DNA-Strang anfügt, der zur DNA-Zielsequenz komplementär ist.

9. Zusammensetzung oder Kit nach einem der Ansprüche 1 bis 8, wobei die DNA-Modifizierungsdomäne die Addition von ADP-Ribose an ein Thymin- oder Guanin-Nukleotid katalysiert.

10. Zusammensetzung oder Kit nach einem der Ansprüche 1 bis 9, wobei die DNA-Modifizierungsdomäne ein DarT-Enzym oder ein funktionelles Fragment, Derivat oder eine Variante davon umfasst.

11. Zusammensetzung oder Kit nach einem der Ansprüche 1 bis 10, wobei die Zusammensetzung ferner mindestens einen Lücken-Editor-Hilfsfaktor umfasst, wobei der mindestens eine Lücken-Editor-Hilfsfaktor vorzugsweise
i) ein Protein umfasst, das mindestens einen Schritt in einem Genom-Modifikationsprozess verstärkt;
ii) über eine Wechselwirkung mit der DNA-Modifizierungsdomäne, der DNA-Erkennungsdomäne und/oder der mindestens einen Leit-RNA an den Lücken-Editor-Komplex gebunden wird, wobei die Bindung des mindestens einen Lücken-Editor-Hilfsfaktors an den Lücken-Editor-Komplex vorzugsweise ein Peptid-Tag, einen Peptid-Linker, ein RNA-Tag und beliebige Kombinationen davon umfasst;
iii) Rap, DarG, Orf, ExoI, Exonuklease III, PrimPol, RecJ, RecQ1, Rad51, Rad52, CtIP, Rad18 und beliebige Kombinationen davon umfasst.

12. In-vitro-Verfahren zur gezielten Genom-Modifikation, wobei das Verfahren umfasst:
Einbringen einer beliebigen der Zusammensetzungen der Ansprüche 1 bis 11 in eine Zelle; und
Untersuchung der Zelle auf das Vorhandensein einer gewünschten Genomveränderung.

13. Verfahren nach Anspruch 12, wobei der Lücken-Editor-Komplex und/oder das mindestens eine Leit-RNA-Molekül als Polypeptid(e), mRNA(s) und/oder DNA-Expressionskonstrukt(e) in die Zelle eingebracht werden.

14. Verfahren nach Anspruch 12, wobei die Zelle eine prokaryotische Zelle oder eine eukaryotische Zelle ist.

15. Verfahren nach einem der Ansprüche 12 bis 14, wobei das Verfahren zu einem verringerten Ausmaß an Indel-Bildung, chromosomalen Umlagerungen und DNA-Duplikationen führt, die Zellviabilität verbessert und/oder die Zelltoxizität verringert.

## Revendications

1. Composition ou kit comprenant ladite composition pour une modification ciblée du génome, la composition comprenant un complexe éditeur de brèche comprenant un domaine de reconnaissance d'ADN et un domaine de modification d'ADN, dans lequel le domaine de reconnaissance d'ADN se liant à une séquence d'ADN cible dans le génome, et dans lequel ledit domaine de modification d'ADN est une ADP-ribosyltransférase.

2. Composition ou kit selon la revendication 1, la composition comprenant en outre une matrice d'acide nucléique donneur, de préférence, dans lequel la matrice d'acide nucléique donneur comprend un polynucléotide d'une séquence homologue endogène correspondant à la séquence d'ADN cible, dans lequel, plus préférentiellement, la matrice d'acide nucléique donneur comprend une molécule d'ADN simple brin (ADNsb), une molécule d'ADN double brin (ADNdb) ou une molécule d'ARN exogène.

3. Composition ou kit selon la revendication 2, dans lequel la présence de la matrice d'acide nucléique donneur facilite la réparation de brèche et/ou la recombinaison dirigées par homologie, dans lequel la matrice d'acide nucléique donneur ou un fragment de celle-ci est recombinée dans le génome de la séquence cible d'ADN.

4. Composition ou kit selon l'une quelconque des revendications 1 à 3, la composition comprenant au moins une molécule d'ARN guide, dans lequel ledit au moins un ARN guide est de préférence défini plus avant par au moins l'un des éléments suivants :
i) ledit ou lesdits ARN guides comprend un ARNg, un ARNsg, un ARNcr ou des combinaisons quelconques de ceux-ci ;
ii) le ou les ARN guides comprennent une séquence d'ancrage et une séquence de ciblage, de préférence, la séquence de ciblage dans le ou les ARN guides étant complémentaire de la séquence d'ADN cible.

5. Composition ou kit selon l'une quelconque des revendications 1 à 4, dans lequel le domaine de reconnaissance d'ADN est en outre défini par au moins l'un des éléments suivants :
i) le domaine de reconnaissance d'ADN comprend au moins une protéine Cas ou un fragment de celle-ci dépourvu d'activité désoxyribonucléase ;
ii) le domaine de reconnaissance d'ADN comprend un complexe de protéines Cas dépourvues d'activité désoxyribonucléase ;
iii) le domaine de reconnaissance d'ADN comprend une protéine Cas ou un fragment de celle-ci ayant une activité nickase.

6. Composition ou kit selon l'une quelconque des revendications 1 à 5, dans lequel la protéine Cas ou le complexe de protéine Cas comprend une cascade de type I, une Cas9 de type II, un module effecteur de type IV, une Cas12 de type V, un IscB associé à Cas9, un TnpB apparenté à Cas12a, et des combinaisons de ceux-ci.

7. Composition ou kit selon l'une quelconque des revendications 1 à 6, dans lequel le domaine de reconnaissance d'ADN et le domaine de modification d'ADN sont couplés fonctionnellement, dans lequel, de préférence, couplé fonctionnellement désigne des fusions de polypeptides, des étiquettes peptidiques, des lieurs peptidiques, des étiquettes d'ARN, et une combinaison quelconque de ceux-ci.

8. Composition ou kit selon l'une quelconque des revendications 1 à 7, dans lequel le domaine de reconnaissance d'ADN induit une rupture simple brin dans le brin d'ADN cible, et dans lequel le domaine de modification d'ADN ajoute un fragment bloquant la réplication à au moins un nucléotide dans le brin d'ADN complémentaire de la séquence d'ADN cible.

9. Composition ou kit selon l'une quelconque des revendications 1 à 8, dans lequel le domaine de modification d'ADN catalyse l'addition d'ADP ribose à un nucléotide thymine ou guanine.

10. Composition ou kit selon l'une quelconque des revendications 1 à 9, dans lequel le domaine de modification d'ADN comprend une enzyme DarT ou un fragment fonctionnel, un dérivé ou un variant de celle-ci.

11. Composition ou kit selon l'une quelconque des revendications 1 à 10, dans lequel la composition comprend en outre au moins un facteur accessoire d'éditeur de brèche, de préférence, dans lequel le ou les facteurs accessoires d'éditeur de brèche
i) comprennent une protéine qui augmente au moins une étape dans un processus de modification du génome ;
ii) sont recrutés pour le complexe éditeur de brèche par interaction avec le domaine de modification d'ADN, le domaine de reconnaissance d'ADN et/ou le ou les ARN guides, plus préférentiellement, le recrutement du ou des facteurs accessoires d'éditeur de brèche pour le complexe éditeur de brèche comprend une étiquette peptidique, un lieur peptidique, une étiquette d'ARN, et des combinaisons quelconques de ceux-ci ;
iii) comprend Rap, DarG, Orf, ExoI, l'exonucléase III, PrimPol, RecJ, RecQ1, Rad51, Rad52, CtIP, Rad18 et une combinaison quelconque de ceux-ci.

12. Procédé *in vitro* de modification du ciblée génome, le procédé comprenant :
l'introduction de l'une quelconque des compositions des revendications 1 à 11 dans une cellule ; et
l'évaluation de la présence d'une altération souhaitée du génome dans la cellule.

13. Procédé selon la revendication 12, dans lequel le complexe éditeur de brèche et/ou la ou les molécules d'ARN guide sont introduits dans la cellule sous la forme d'un ou plusieurs polypeptides, ARNm et/ou constructions d'expression d'ADN.

14. Procédé selon la revendication 12, dans lequel la cellule est une cellule procaryote ou une cellule eucaryote.

15. Procédé selon l'une quelconque des revendications 12 à 14, dans lequel le procédé conduit à un degré réduit de formation d'indel, de réarrangements chromosomiques, de duplications d'ADN, améliore la viabilité cellulaire et/ou réduit la toxicité cellulaire.
